(19) Europäisches Patentamt
European Patent Office
Office européen des brevets

(11) EP 3 520 775 A1

(12) EUROPEAN PATENT APPLICATION

(43) Date of publication:
**07.08.2019 Bulletin 2019/32**

(21) Application number: **19154186.1**

(22) Date of filing: **29.01.2019**

(51) Int Cl.:
*A61K 9/00* (2006.01) *A61K 9/127* (2006.01)
*A61K 31/282* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.01.2018 US 201862624538 P**

(71) Applicant: **Liplasome Pharma ApS**
**7100 Vejle (DK)**

(72) Inventors:
- **KNUDSEN, Steen**
  **Scottsdale, AZ Arizona 85258 (US)**
- **JENSEN, Peter**
  **3520 Farum (DK)**
- **BUHL, Ulla**
  **3520 Farum (DK)**
- **RASMUSSEN, Annie**
  **2900 Hellerup (DK)**
- **MADSEN, Mogens**
  **2830 Virum (DK)**

(74) Representative: **Hutter, Anton et al**
**Venner Shipley LLP**
**200 Aldersgate**
**London EC1A 4HD (GB)**

Remarks:
Claims filed after the date of receipt of the application (Rule 68(4) EPC).

# (54) METHODS FOR TREATING CANCER AND PREDICTING DRUG RESPONSIVENESS IN CANCER PATIENTS

(57) Featured are methods of treating a patient with cancer by administering, e.g., a secretory phospholipase $A_2$ ($sPLA_2$) hydrolysable, cisplatin-containing liposome composition (e.g., LiPlaCis). The patient may be assessed for their responsiveness to the liposomal therapy prior to treatment using the methods, devices, and kits also described herein for detecting a level of one or more biomarkers in a sample from the patient with cancer.

Figure 7

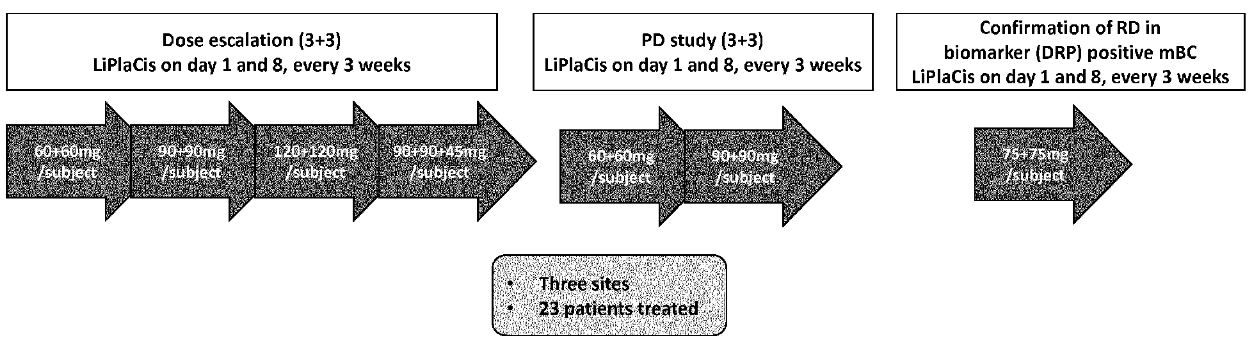


EP 3 520 775 A1

SEQUENCE LISTING

<110> LiPlasome Pharma ApS

<120> METHODS FOR TREATING CANCER AND PREDICTING DRUG RESPONSIVENESS IN CANCER PATIENTS

<130> 51181-006001

<160> 405

<170> PatentIn version 3.5

<210> 1
<211> 25
<212> DNA
<213> Homo sapiens

<400> 1
caggcctctc agatacaagg ggaac 25

<210> 2
<211> 25
<212> DNA
<213> Homo sapiens

<400> 2
atagctgcct taaagtcagt aactt 25

<210> 3
<211> 25
<212> DNA
<213> Homo sapiens

<400> 3
caccatgctg gctatccggg tgtta 25

<210> 4
<211> 25
<212> DNA
<213> Homo sapiens

<400> 4
ctccactctg actggcacag tcttt 25

<210> 5
<211> 25
<212> DNA
<213> Homo sapiens

<400> 5
gattcctgaa ctcaaggtac cagca 25

<210> 6
<211> 25
<212> DNA
<213> Homo sapiens

<400> 6
gatcatcgac gccatcaggc aggag 25

<210> 7
<211> 25
<212> DNA
<213> Homo sapiens

<400> 7
gtgtggttga acaggatgca atctt 25

<210> 8
<211> 25
<212> DNA
<213> Homo sapiens

<400> 8
gcttcccaag atgccatggc tggac 25

<210> 9
<211> 25
<212> DNA
<213> Homo sapiens

<400> 9
gcaggacttc ccttataaag caaaa 25

<210> 10
<211> 25
<212> DNA
<213> Homo sapiens

<400> 10
gattataacc gtgttgaact ctctg 25

<210> 11
<211> 25
<212> DNA
<213> Homo sapiens

<400> 11
gttttcggtc ctattttaat gctct 25

<210> 12
<211> 25
<212> DNA
<213> Homo sapiens

<400> 12
aaagaccaac aaatctcaag cccta 25

<210> 13
<211> 25
<212> DNA
<213> Homo sapiens

<400>  13
ggtctgttct tgtagataat gccct                                               25

<210>  14
<211>  25
<212>  DNA
<213>  Homo sapiens

<400>  14
tgctggccac ggattttgac gacga                                               25

<210>  15
<211>  25
<212>  DNA
<213>  Homo sapiens

<400>  15
ggtgttgtac agctcacatg tttac                                               25

<210>  16
<211>  25
<212>  DNA
<213>  Homo sapiens

<400>  16
ggtttgcctc attgtgctat ttgcc                                               25

<210>  17
<211>  25
<212>  DNA
<213>  Homo sapiens

<400>  17
ataagcattg attcctgcat ttctg                                               25

<210>  18
<211>  25
<212>  DNA
<213>  Homo sapiens

<400>  18
gcatttagtc caatgtcttt ttaag                                               25

<210>  19
<211>  25
<212>  DNA
<213>  Homo sapiens

<400>  19
atcatgctga ggcgccttgc aaatc                                               25

<210>  20
<211>  25
<212>  DNA
<213>  Homo sapiens

<400> 20
atgactggta tgatagctct tgaca 25

<210> 21
<211> 25
<212> DNA
<213> Homo sapiens

<400> 21
caatccaagc ataactcagt gacgc 25

<210> 22
<211> 25
<212> DNA
<213> Homo sapiens

<400> 22
gaatgtgtag ctcaaatgca aacca 25

<210> 23
<211> 25
<212> DNA
<213> Homo sapiens

<400> 23
ttccctcctt atagtcaagg accgt 25

<210> 24
<211> 25
<212> DNA
<213> Homo sapiens

<400> 24
tgacctcggt cacaaaagca gtttt 25

<210> 25
<211> 25
<212> DNA
<213> Homo sapiens

<400> 25
gaacagtttg tacagacgta tgctt 25

<210> 26
<211> 25
<212> DNA
<213> Homo sapiens

<400> 26
tacaacacta tacatacaca ccacc 25

<210> 27
<211> 25
<212> DNA
<213> Homo sapiens

<400> 27
ttatgccagc ttatattgtg agaac 25

<210> 28
<211> 25
<212> DNA
<213> Homo sapiens

<400> 28
gagttgcctg tttgtctctg gagat 25

<210> 29
<211> 25
<212> DNA
<213> Homo sapiens

<400> 29
gctgcagtgt agatggctct tgttt 25

<210> 30
<211> 25
<212> DNA
<213> Homo sapiens

<400> 30
acgttgtcac cggagcactg aagat 25

<210> 31
<211> 25
<212> DNA
<213> Homo sapiens

<400> 31
atagcagcac acattttcac gtttc 25

<210> 32
<211> 25
<212> DNA
<213> Homo sapiens

<400> 32
aagaccggca gatggtggtg ctgga 25

<210> 33
<211> 25
<212> DNA
<213> Homo sapiens

<400> 33
atttggctca agtccatttg gctgt 25

<210> 34
<211> 25
<212> DNA
<213> Homo sapiens

<400> 34
gcatgaagtt gcccttaacc actaa 25

<210> 35
<211> 25
<212> DNA
<213> Homo sapiens

<400> 35
taaccatgct tacacactaa actat 25

<210> 36
<211> 25
<212> DNA
<213> Homo sapiens

<400> 36
tgcccggtcg gagtaaacac agaat 25

<210> 37
<211> 25
<212> DNA
<213> Homo sapiens

<400> 37
gatgtcaact gggtcaatgg gggcc 25

<210> 38
<211> 25
<212> DNA
<213> Homo sapiens

<400> 38
gttggctgat attggagtgc tcatt 25

<210> 39
<211> 25
<212> DNA
<213> Homo sapiens

<400> 39
ccttttgtac ttcactcaga tacta 25

<210> 40
<211> 25
<212> DNA
<213> Homo sapiens

<400> 40
taagcatcct tagggttctg cctct 25

<210> 41
<211> 25
<212> DNA
<213> Homo sapiens

<400> 41
ctcatctccc tcaaggatgg ctacg 25

<210> 42
<211> 25
<212> DNA
<213> Homo sapiens

<400> 42
tgtgaatcat cctgcctttc aaatt 25

<210> 43
<211> 25
<212> DNA
<213> Homo sapiens

<400> 43
tacaaaccac attacttctg tcact 25

<210> 44
<211> 25
<212> DNA
<213> Homo sapiens

<400> 44
gtcccggatc caggacctgg tagca 25

<210> 45
<211> 25
<212> DNA
<213> Homo sapiens

<400> 45
agtgaagagg tcgtcctctc catct 25

<210> 46
<211> 25
<212> DNA
<213> Homo sapiens

<400> 46
gctgaaggca cctactcagt atctt 25

<210> 47
<211> 25
<212> DNA
<213> Homo sapiens

<400> 47
taagcattcc gtccatctaa gctca 25

<210> 48
<211> 25
<212> DNA
<213> Homo sapiens

<400> 48
tgatggagca taacgggtcc cagcc 25

<210> 49
<211> 25
<212> DNA
<213> Homo sapiens

<400> 49
atgatttctt agggtctgtg tactt 25

<210> 50
<211> 25
<212> DNA
<213> Homo sapiens

<400> 50
gttccatttc tctcattcac aagat 25

<210> 51
<211> 25
<212> DNA
<213> Homo sapiens

<400> 51
gaggccaaga aattccatgt tgttt 25

<210> 52
<211> 25
<212> DNA
<213> Homo sapiens

<400> 52
aaagctgtga atctgttccc tgctg 25

<210> 53
<211> 25
<212> DNA
<213> Homo sapiens

<400> 53
atgagctccc agattcgtca gaatt 25

<210> 54
<211> 25
<212> DNA
<213> Homo sapiens

<400> 54
tccccatcag gaagtcctcg cagaa 25

<210> 55
<211> 25
<212> DNA
<213> Homo sapiens

<400> 55
tgagtccctg gaacgccaga tgcgt 25

<210> 56
<211> 25
<212> DNA
<213> Homo sapiens

<400> 56
ttagagccag tcttcacact cggaa 25

<210> 57
<211> 25
<212> DNA
<213> Homo sapiens

<400> 57
aaaacttccc cggtatgatg attgt 25

<210> 58
<211> 25
<212> DNA
<213> Homo sapiens

<400> 58
tcagtgggca cagttcttca gctac 25

<210> 59
<211> 25
<212> DNA
<213> Homo sapiens

<400> 59
actagctcat tatttccatc tttgg 25

<210> 60
<211> 25
<212> DNA
<213> Homo sapiens

<400> 60
aattatgagt ttctatctgt gtcca 25

<210> 61
<211> 25
<212> DNA
<213> Homo sapiens

<400> 61
gggaagtcca catagcgtca ttaat 25

<210> 62
<211> 25
<212> DNA
<213> Homo sapiens

<400> 62
ttcagctacc ctgactttct cagga 25

<210> 63
<211> 25
<212> DNA
<213> Homo sapiens

<400> 63
ggcatgatgt ccggtgattt ctgta 25

<210> 64
<211> 25
<212> DNA
<213> Homo sapiens

<400> 64
actcttgagg gttgattatg ctgca 25

<210> 65
<211> 25
<212> DNA
<213> Homo sapiens

<400> 65
ggacctcatc tctgagctga aacgg 25

<210> 66
<211> 25
<212> DNA
<213> Homo sapiens

<400> 66
gttggtgttt aaagatctga agtgt 25

<210> 67
<211> 25
<212> DNA
<213> Homo sapiens

<400> 67
gaagcaccat aactttgttt agccc 25

<210> 68
<211> 25
<212> DNA
<213> Homo sapiens

<400> 68
acagcatgag gcggccgggg agctg 25

<210> 69
<211> 25
<212> DNA
<213> Homo sapiens

<400>  69
gctatggtta tattagcacc aaact  25

<210>  70
<211>  25
<212>  DNA
<213>  Homo sapiens

<400>  70
ggccgacaac agctgcatct atgtc  25

<210>  71
<211>  25
<212>  DNA
<213>  Homo sapiens

<400>  71
tgaaaaaggg tttctattct ctctg  25

<210>  72
<211>  25
<212>  DNA
<213>  Homo sapiens

<400>  72
agtatcagtc ggtgcaacag ttggc  25

<210>  73
<211>  25
<212>  DNA
<213>  Homo sapiens

<400>  73
tgaagttgat cctgagactc ttgaa  25

<210>  74
<211>  25
<212>  DNA
<213>  Homo sapiens

<400>  74
tactcagagg tgtgaccctc gccag  25

<210>  75
<211>  25
<212>  DNA
<213>  Homo sapiens

<400>  75
gtcgtactat cttactgagc cacag  25

<210>  76
<211>  25
<212>  DNA
<213>  Homo sapiens

<400> 76
aaggcgtaac gagttcatct ttctt 25

<210> 77
<211> 25
<212> DNA
<213> Homo sapiens

<400> 77
ccttgatacc agctctctgt ggaaa 25

<210> 78
<211> 25
<212> DNA
<213> Homo sapiens

<400> 78
aaatcactaa acctcgtttt ctcag 25

<210> 79
<211> 25
<212> DNA
<213> Homo sapiens

<400> 79
agcgtcctta tctttcagag ctaca 25

<210> 80
<211> 25
<212> DNA
<213> Homo sapiens

<400> 80
aaaccccaga cgccatgaaa gctgc 25

<210> 81
<211> 25
<212> DNA
<213> Homo sapiens

<400> 81
gggaacactg ctctcagaca ttaca 25

<210> 82
<211> 25
<212> DNA
<213> Homo sapiens

<400> 82
ctttttcctg ggtcatgctg caaca 25

<210> 83
<211> 25
<212> DNA
<213> Homo sapiens

<400> 83
gatggggtgc atgtagtctt tggac 25

<210> 84
<211> 25
<212> DNA
<213> Homo sapiens

<400> 84
gaaggtgtga tctgtgggac tgtct 25

<210> 85
<211> 25
<212> DNA
<213> Homo sapiens

<400> 85
gtacgttttt actcagttca tgcgt 25

<210> 86
<211> 25
<212> DNA
<213> Homo sapiens

<400> 86
gcttctcgtg ctgcacatat ttcct 25

<210> 87
<211> 25
<212> DNA
<213> Homo sapiens

<400> 87
gtgaacagac ttgaaactcc agagc 25

<210> 88
<211> 25
<212> DNA
<213> Homo sapiens

<400> 88
atcattttca ggcttctgca gctgt 25

<210> 89
<211> 25
<212> DNA
<213> Homo sapiens

<400> 89
gcatgttgtt tgccaggaca ctgtg 25

<210> 90
<211> 25
<212> DNA
<213> Homo sapiens

<400> 90
ttgtgcttgc tcttcagatg gatgg 25

<210> 91
<211> 25
<212> DNA
<213> Homo sapiens

<400> 91
tagccaggat ccttggtgtc ctagg 25

<210> 92
<211> 25
<212> DNA
<213> Homo sapiens

<400> 92
gcggcgtgta taccaatgca tggcc 25

<210> 93
<211> 25
<212> DNA
<213> Homo sapiens

<400> 93
gaataacttt tggctgttgt gctaa 25

<210> 94
<211> 25
<212> DNA
<213> Homo sapiens

<400> 94
tggcccgcgt gattgtggca tttaa 25

<210> 95
<211> 25
<212> DNA
<213> Homo sapiens

<400> 95
cataactgtt agacttcccg tttct 25

<210> 96
<211> 25
<212> DNA
<213> Homo sapiens

<400> 96
tcaataaagt tcccctgtga cactc 25

<210> 97
<211> 25
<212> DNA
<213> Homo sapiens

<400> 97
ctcccctatg atgggcggct actgg 25

<210> 98
<211> 25
<212> DNA
<213> Homo sapiens

<400> 98
atcagttgat tcttgtgcca ttttt 25

<210> 99
<211> 25
<212> DNA
<213> Homo sapiens

<400> 99
tgagccagtg agggcagtcc tgcaa 25

<210> 100
<211> 25
<212> DNA
<213> Homo sapiens

<400> 100
gcatcatcac catagagtcc cagga 25

<210> 101
<211> 25
<212> DNA
<213> Homo sapiens

<400> 101
tcttgctcca aagggctccg tggag 25

<210> 102
<211> 25
<212> DNA
<213> Homo sapiens

<400> 102
atacgccctt ggcacagtcg gatga 25

<210> 103
<211> 25
<212> DNA
<213> Homo sapiens

<400> 103
gtctgctggg tgtgaccatg tttcc 25

<210> 104
<211> 25
<212> DNA
<213> Homo sapiens

<400> 104
gtgcgtggga cgaagacatc tttga 25

<210> 105
<211> 25
<212> DNA
<213> Homo sapiens

<400> 105
tggtcccctt cacctgggag aaaag 25

<210> 106
<211> 25
<212> DNA
<213> Homo sapiens

<400> 106
gggccaagca ggacatggcg cggca 25

<210> 107
<211> 25
<212> DNA
<213> Homo sapiens

<400> 107
agcttcagac tcaagtaccc attct 25

<210> 108
<211> 25
<212> DNA
<213> Homo sapiens

<400> 108
gagctgctga gacgacgctc acaga 25

<210> 109
<211> 25
<212> DNA
<213> Homo sapiens

<400> 109
gagaagcagt ttgacggacc ttgtg 25

<210> 110
<211> 25
<212> DNA
<213> Homo sapiens

<400> 110
ggtgattctg agcgagatct tcctg 25

<210> 111
<211> 25
<212> DNA
<213> Homo sapiens

<400> 111
gcagccttgg aaacctaacc tgcct 25

<210> 112
<211> 25
<212> DNA
<213> Homo sapiens

<400> 112
cctgcagcac cgacgtgtgt gacag 25

<210> 113
<211> 25
<212> DNA
<213> Homo sapiens

<400> 113
acttggctca gtggaagccc tcttt 25

<210> 114
<211> 25
<212> DNA
<213> Homo sapiens

<400> 114
acattgcccg gaaactcagt ctatt 25

<210> 115
<211> 25
<212> DNA
<213> Homo sapiens

<400> 115
gctgtggatc tgtttggcca gggtc 25

<210> 116
<211> 25
<212> DNA
<213> Homo sapiens

<400> 116
gttagagccg atatcactgg aagat 25

<210> 117
<211> 25
<212> DNA
<213> Homo sapiens

<400> 117
agatttgtca gccctatctc aaact 25

<210> 118
<211> 25
<212> DNA
<213> Homo sapiens

<400> 118
aggtcttccc agaggctgga tacca 25

<210> 119
<211> 25
<212> DNA
<213> Homo sapiens

<400> 119
gtcttcccta cctcaggcag gaagg 25

<210> 120
<211> 25
<212> DNA
<213> Homo sapiens

<400> 120
ctacccttat gatgacccat tttcc 25

<210> 121
<211> 25
<212> DNA
<213> Homo sapiens

<400> 121
aaatgctctt atttttccaa cagct 25

<210> 122
<211> 25
<212> DNA
<213> Homo sapiens

<400> 122
gtgtatagtg ttttaccctc ttctt 25

<210> 123
<211> 25
<212> DNA
<213> Homo sapiens

<400> 123
tcatcaaggg ctatgtgcct cccac 25

<210> 124
<211> 25
<212> DNA
<213> Homo sapiens

<400> 124
ccccgcacca gatcaagtag tttgg 25

<210> 125
<211> 25
<212> DNA
<213> Homo sapiens

<400> 125
ccctcctacc agatgacaca gacaa 25

<210> 126
<211> 25
<212> DNA
<213> Homo sapiens

<400> 126
tgtatggttt tcacctggac accgt 25

<210> 127
<211> 25
<212> DNA
<213> Homo sapiens

<400> 127
aactccgtct gtctactgtg tgaga 25

<210> 128
<211> 25
<212> DNA
<213> Homo sapiens

<400> 128
cccactggcc tgaatctaca ctgga 25

<210> 129
<211> 25
<212> DNA
<213> Homo sapiens

<400> 129
acattacatc cgtggattct cctgc 25

<210> 130
<211> 25
<212> DNA
<213> Homo sapiens

<400> 130
ggccctgggc cagggtgatt ggact 25

<210> 131
<211> 25
<212> DNA
<213> Homo sapiens

<400> 131
tttagccctc atgactgtat tttct 25

<210> 132
<211> 25
<212> DNA
<213> Homo sapiens

<400> 132
acacgctgca gatcgaggac tttct 25

<210> 133
<211> 25
<212> DNA
<213> Homo sapiens

<400> 133
accggcagcc ctggaagggg cactt 25

<210> 134
<211> 25
<212> DNA
<213> Homo sapiens

<400> 134
taccagcagg aggttctggg cctct 25

<210> 135
<211> 25
<212> DNA
<213> Homo sapiens

<400> 135
catctttcag ggctgccagt ttcgc 25

<210> 136
<211> 25
<212> DNA
<213> Homo sapiens

<400> 136
gggctacgcc aaggactttg accct 25

<210> 137
<211> 25
<212> DNA
<213> Homo sapiens

<400> 137
aatctagtca cctaaccttg tggtt 25

<210> 138
<211> 25
<212> DNA
<213> Homo sapiens

<400> 138
atttcaaaat ttctgcattc acgga 25

<210> 139
<211> 25
<212> DNA
<213> Homo sapiens

<400> 139
aacacctgtc cattgaagat ttcac 25

<210> 140
<211> 25
<212> DNA
<213> Homo sapiens

<400> 140
gacattctta tgcttctttt acaac 25

<210> 141
<211> 25
<212> DNA
<213> Homo sapiens

<400> 141
aatgtttcct aacagttgtg atgtt 25

<210> 142
<211> 25
<212> DNA
<213> Homo sapiens

<400> 142
gggtgaagag agactcggtg cgggc 25

<210> 143
<211> 25
<212> DNA
<213> Homo sapiens

<400> 143
cgaccgcctg tatgtttgtg taatt 25

<210> 144
<211> 25
<212> DNA
<213> Homo sapiens

<400> 144
aaggcctatc agcttctatc agccc 25

<210> 145
<211> 25
<212> DNA
<213> Homo sapiens

<400> 145
gtcactggtc tgtttgcatt tgata 25

<210> 146
<211> 25
<212> DNA
<213> Homo sapiens

<400> 146
agctcaaaac ttgctaggca tcaga 25

<210> 147
<211> 25
<212> DNA
<213> Homo sapiens

<400> 147
gcagcaaagc tggctccaac atgct 25

<210> 148
<211> 25
<212> DNA
<213> Homo sapiens

<400> 148
aaacactatc tacttccttt gtcat 25

<210> 149
<211> 25
<212> DNA
<213> Homo sapiens

<400> 149
gaggatcatg cccttagcaa gtact 25

<210> 150
<211> 25
<212> DNA
<213> Homo sapiens

<400> 150
aacatcattt tagcaaaggc cagga 25

<210> 151
<211> 25
<212> DNA
<213> Homo sapiens

<400> 151
tgtccttgtt acattgaggt taaga 25

<210> 152
<211> 25
<212> DNA
<213> Homo sapiens

<400> 152
tccctactgc atggggactt ccacg 25

<210> 153
<211> 25
<212> DNA
<213> Homo sapiens

<400> 153
ccaccttcac ctcggaggga cggag 25

<210> 154
<211> 25
<212> DNA
<213> Homo sapiens

<400> 154
taattacacg ttcaccgtcc aagca 25

<210> 155
<211> 25
<212> DNA
<213> Homo sapiens

<400> 155
ttccctttct accattgatt taaat 25

<210> 156
<211> 25
<212> DNA
<213> Homo sapiens

<400> 156
agtactggtt gaacctgacc acttc 25

<210> 157
<211> 25
<212> DNA
<213> Homo sapiens

<400> 157
aaatacataa gctagtttct gttct 25

<210> 158
<211> 25
<212> DNA
<213> Homo sapiens

<400> 158
gtaacgtgat tgattcagta tctta 25

<210> 159
<211> 25
<212> DNA
<213> Homo sapiens

<400> 159
aatacatccg atgcgtagat tcttg 25

<210> 160
<211> 25
<212> DNA
<213> Homo sapiens

<400> 160
agaatgctcg ggtgctagac tggat 25

<210> 161
<211> 25
<212> DNA
<213> Homo sapiens

<400> 161
ggacggactc tatgaggggc tcaca 25

<210> 162
<211> 25
<212> DNA
<213> Homo sapiens

<400> 162
ggaatgactc aaatgcccaa aacca 25

<210> 163
<211> 25
<212> DNA
<213> Homo sapiens

<400> 163
tcctctcagc tcctaaagca caact 25

<210> 164
<211> 25
<212> DNA
<213> Homo sapiens

<400> 164
acatgttcgc acccaagtgt ggcgg 25

<210> 165
<211> 25
<212> DNA
<213> Homo sapiens

<400> 165
tttcagatgg agtaccagca ccgaa 25

<210> 166
<211> 25
<212> DNA
<213> Homo sapiens

<400> 166
tggcactcgc ggttggagtc agcga 25

<210> 167
<211> 25
<212> DNA
<213> Homo sapiens

<400> 167
ggccatctat gggactgaac tttga 25

<210> 168
<211> 25
<212> DNA
<213> Homo sapiens

<400> 168
gaagaaactg gacaccaaca gtgat 25

<210> 169
<211> 25
<212> DNA
<213> Homo sapiens

<400> 169
gctgtagcca gtgcagacct cactg 25

<210> 170
<211> 25
<212> DNA
<213> Homo sapiens

<400> 170
aggtgtccag tacctaatca cgctc 25

<210> 171
<211> 25
<212> DNA
<213> Homo sapiens

<400> 171
ttgccgtgat gaccgtggct atcct 25

<210> 172
<211> 25
<212> DNA
<213> Homo sapiens

<400> 172
gaatacgtta ggagccagta cccca 25

<210> 173
<211> 25
<212> DNA
<213> Homo sapiens

<400> 173
gaagctgctc aacaaagtgt ggaag 25

<210> 174
<211> 25
<212> DNA
<213> Homo sapiens

<400> 174
ggaatgtccc agacagacct gtctc 25

<210> 175
<211> 25
<212> DNA
<213> Homo sapiens

<400> 175
ccttcatgga cgtcaacagc acctg 25

<210> 176
<211> 25
<212> DNA
<213> Homo sapiens

<400> 176
ggcccggata tggctcgtgg acagc 25

<210> 177
<211> 25
<212> DNA
<213> Homo sapiens

<400> 177
aggagtctcc accagaggga ggctc 25

<210> 178
<211> 25
<212> DNA
<213> Homo sapiens

<400> 178
gagcaccact actgctgcat tgtgc 25

<210> 179
<211> 25
<212> DNA
<213> Homo sapiens

<400> 179
actctgacat ttcttgttct caagc 25

<210> 180
<211> 25
<212> DNA
<213> Homo sapiens

<400> 180
ccagcatttc cagagcagag cctac 25

<210> 181
<211> 25
<212> DNA
<213> Homo sapiens

<400>  181
gcgtgtcttg agttccatgc aaatt    25

<210>  182
<211>  25
<212>  DNA
<213>  Homo sapiens

<400>  182
aatggtgaca gtccaaacca ctcca    25

<210>  183
<211>  25
<212>  DNA
<213>  Homo sapiens

<400>  183
ggccctgcat gtcagatggc gtggt    25

<210>  184
<211>  25
<212>  DNA
<213>  Homo sapiens

<400>  184
tgatcataaa ttctccccaa ctata    25

<210>  185
<211>  25
<212>  DNA
<213>  Homo sapiens

<400>  185
aaagttgcca agatgctcct tgttg    25

<210>  186
<211>  25
<212>  DNA
<213>  Homo sapiens

<400>  186
gtctcaccca actgcagttt actat    25

<210>  187
<211>  25
<212>  DNA
<213>  Homo sapiens

<400>  187
gacggagttc ctaagcttca tgaat    25

<210>  188
<211>  25
<212>  DNA
<213>  Homo sapiens

<400> 188
tcagcctggg cagtcttacc aaaat 25

<210> 189
<211> 25
<212> DNA
<213> Homo sapiens

<400> 189
tgctgtggct tcaccaacta tacgg 25

<210> 190
<211> 25
<212> DNA
<213> Homo sapiens

<400> 190
ccttgactcc tttggtattt cactg 25

<210> 191
<211> 25
<212> DNA
<213> Homo sapiens

<400> 191
acagcaggca tcgcaacttt ctgca 25

<210> 192
<211> 25
<212> DNA
<213> Homo sapiens

<400> 192
caagagctac aatgtcacct ccgtc 25

<210> 193
<211> 25
<212> DNA
<213> Homo sapiens

<400> 193
gtttgtctct tgttgttctg aagga 25

<210> 194
<211> 25
<212> DNA
<213> Homo sapiens

<400> 194
ctctctagtt agatatctga cttgg 25

<210> 195
<211> 25
<212> DNA
<213> Homo sapiens

<400> 195
tccagcctcg gggtgtaggt ttctg 25

<210> 196
<211> 25
<212> DNA
<213> Homo sapiens

<400> 196
actcgtctca cgccgtgttt gagat 25

<210> 197
<211> 25
<212> DNA
<213> Homo sapiens

<400> 197
gccagagttc aaatgtgact ccacc 25

<210> 198
<211> 25
<212> DNA
<213> Homo sapiens

<400> 198
caagcccctg tattttgctg atcgg 25

<210> 199
<211> 25
<212> DNA
<213> Homo sapiens

<400> 199
agaccacaga tatcttctag acata 25

<210> 200
<211> 25
<212> DNA
<213> Homo sapiens

<400> 200
gtcactgtaa atcattctta agccc 25

<210> 201
<211> 25
<212> DNA
<213> Homo sapiens

<400> 201
aaggctgtca gggcttctgt ttgtt 25

<210> 202
<211> 25
<212> DNA
<213> Homo sapiens

<400> 202
gtagacacct gcacgcatag gattg 25

<210> 203
<211> 25
<212> DNA
<213> Homo sapiens

<400> 203
ttgccacttt cttgcgatat gctgt 25

<210> 204
<211> 25
<212> DNA
<213> Homo sapiens

<400> 204
gccattccag aagtagctta tccta 25

<210> 205
<211> 25
<212> DNA
<213> Homo sapiens

<400> 205
ccaatacccc accgtgatga cttga 25

<210> 206
<211> 25
<212> DNA
<213> Homo sapiens

<400> 206
aattctctgt tatctttacg aggta 25

<210> 207
<211> 25
<212> DNA
<213> Homo sapiens

<400> 207
cacgagaagg actatgacag cctgg 25

<210> 208
<211> 25
<212> DNA
<213> Homo sapiens

<400> 208
tgatttgcca caatgtcctt aactc 25

<210> 209
<211> 25
<212> DNA
<213> Homo sapiens

<400> 209
gctgaaggca cctactcagt atctt 25

<210> 210
<211> 25
<212> DNA
<213> Homo sapiens

<400> 210
agacctggca cttcagtaac tcagc 25

<210> 211
<211> 25
<212> DNA
<213> Homo sapiens

<400> 211
gactcttgaa gtaatggctg atcct 25

<210> 212
<211> 25
<212> DNA
<213> Homo sapiens

<400> 212
gggactttgt caactgcagt acact 25

<210> 213
<211> 25
<212> DNA
<213> Homo sapiens

<400> 213
cctttcttta ctccatcatg gctgg 25

<210> 214
<211> 25
<212> DNA
<213> Homo sapiens

<400> 214
atcactaaca ggtctttgac tcagg 25

<210> 215
<211> 25
<212> DNA
<213> Homo sapiens

<400> 215
gaattcatgg tatcctggtt atttt 25

<210> 216
<211> 25
<212> DNA
<213> Homo sapiens

<400> 216
aactactgtg aaattctacc agcat 25

<210> 217
<211> 25
<212> DNA
<213> Homo sapiens

<400> 217
gacacctgag cctggatttt cactc 25

<210> 218
<211> 25
<212> DNA
<213> Homo sapiens

<400> 218
tcaaacggcc gaagttgcct ctttt 25

<210> 219
<211> 25
<212> DNA
<213> Homo sapiens

<400> 219
atactaggcc tgtctgtggc attct 25

<210> 220
<211> 25
<212> DNA
<213> Homo sapiens

<400> 220
ggatttagga taagctgtca cccag 25

<210> 221
<211> 25
<212> DNA
<213> Homo sapiens

<400> 221
ggtcagcaac ctcttttgat tttgt 25

<210> 222
<211> 25
<212> DNA
<213> Homo sapiens

<400> 222
gacaggtgaa catttccgcc tggtc 25

<210> 223
<211> 25
<212> DNA
<213> Homo sapiens

<400> 223
gattcctgaa ctcaaggtac cagca 25

<210> 224
<211> 25
<212> DNA
<213> Homo sapiens

<400> 224
tgttcctttt tgttcaaagt ctatt 25

<210> 225
<211> 25
<212> DNA
<213> Homo sapiens

<400> 225
aggtgttcga aatccgcacc actga 25

<210> 226
<211> 25
<212> DNA
<213> Homo sapiens

<400> 226
gtggagttga tgactttctg ttttc 25

<210> 227
<211> 25
<212> DNA
<213> Homo sapiens

<400> 227
tgcaggtcat gcacacagtt ttgat 25

<210> 228
<211> 25
<212> DNA
<213> Homo sapiens

<400> 228
gctccagaag cgcttggaca ggctg 25

<210> 229
<211> 25
<212> DNA
<213> Homo sapiens

<400> 229
ggcataatgg caaatccttc aagca 25

<210> 230
<211> 25
<212> DNA
<213> Homo sapiens

<400> 230
ccccatagag acccaagttc tgcgg 25

<210> 231
<211> 25
<212> DNA
<213> Homo sapiens

<400> 231
gtaaaccaca tcttttttgc acttt 25

<210> 232
<211> 25
<212> DNA
<213> Homo sapiens

<400> 232
cacttttgt atttatcgtt tcata 25

<210> 233
<211> 25
<212> DNA
<213> Homo sapiens

<400> 233
gacgcaggac gagctcagtt gtaga 25

<210> 234
<211> 25
<212> DNA
<213> Homo sapiens

<400> 234
tgttgtgcag tgtgcctgtc actac 25

<210> 235
<211> 25
<212> DNA
<213> Homo sapiens

<400> 235
cacttactga ctttgcattt tcgtt 25

<210> 236
<211> 25
<212> DNA
<213> Homo sapiens

<400> 236
ggtggtttct cttgagactc gttac 25

<210> 237
<211> 25
<212> DNA
<213> Homo sapiens

<400> 237
ttggcagttg cattagtaac tttga 25

<210> 238
<211> 25
<212> DNA
<213> Homo sapiens

<400> 238
tcatgtgcac atgccgttgc agcac 25

<210> 239
<211> 25
<212> DNA
<213> Homo sapiens

<400> 239
cgttggagaa ctgcagctgc tgtgc 25

<210> 240
<211> 25
<212> DNA
<213> Homo sapiens

<400> 240
agcagctgag gtctcttgag ggagc 25

<210> 241
<211> 25
<212> DNA
<213> Homo sapiens

<400> 241
cattggcctg agtttcttgt gcatt 25

<210> 242
<211> 25
<212> DNA
<213> Homo sapiens

<400> 242
gagcctacct ggagggcgag tgcgt 25

<210> 243
<211> 25
<212> DNA
<213> Homo sapiens

<400> 243
gttttcaatt ttgcatgctc gatta 25

<210> 244
<211> 25
<212> DNA
<213> Homo sapiens

<400> 244
ttatgtgtac attattgttg ctatt 25

<210> 245
<211> 25
<212> DNA
<213> Homo sapiens

<400> 245
cttcccggtc actggtaaca atagc 25

<210> 246
<211> 25
<212> DNA
<213> Homo sapiens

<400> 246
gtacctgtgg gttagcatca agttc 25

<210> 247
<211> 25
<212> DNA
<213> Homo sapiens

<400> 247
ctgagagcct acctggaggg cctgt 25

<210> 248
<211> 25
<212> DNA
<213> Homo sapiens

<400> 248
ttgggccttt tatctgtcta tccat 25

<210> 249
<211> 25
<212> DNA
<213> Homo sapiens

<400> 249
cagtgtgaca agtgccggga gatct 25

<210> 250
<211> 25
<212> DNA
<213> Homo sapiens

<400> 250
agaatctttt ctatgcctct attcc 25

<210> 251
<211> 25
<212> DNA
<213> Homo sapiens

<400> 251
gccatatagc aggcacgtcc gggtc 25

<210> 252
<211> 25
<212> DNA
<213> Homo sapiens

<400> 252
ggcaagggat aactcttcta acaca 25

<210> 253
<211> 25
<212> DNA
<213> Homo sapiens

<400> 253
ttccctcctt atagtcaagg accgt 25

<210> 254
<211> 25
<212> DNA
<213> Homo sapiens

<400> 254
aataacgcaa atggcttcct ctttc 25

<210> 255
<211> 25
<212> DNA
<213> Homo sapiens

<400> 255
ggcctagcac ggacatggtc tgtcc 25

<210> 256
<211> 25
<212> DNA
<213> Homo sapiens

<400> 256
tgatggacga atgccttttc cggtg 25

<210> 257
<211> 25
<212> DNA
<213> Homo sapiens

<400> 257
gatcctctgc aatgtgcttg aaaac 25

<210> 258
<211> 25
<212> DNA
<213> Homo sapiens

<400> 258
tcacaagcta ttccctcaaa tctga 25

<210> 259
<211> 25
<212> DNA
<213> Homo sapiens

<400> 259
tgatgagctt tcctttgatc cggac 25

<210> 260
<211> 25
<212> DNA
<213> Homo sapiens

<400> 260
gagacagctg tcttgtgagg gactg 25

<210> 261
<211> 25
<212> DNA
<213> Homo sapiens

<400> 261
tacacaccac catatatact agctg 25

<210> 262
<211> 25
<212> DNA
<213> Homo sapiens

<400> 262
gtgtaggtga atgcaaactc catcg 25

<210> 263
<211> 25
<212> DNA
<213> Homo sapiens

<400> 263
ttcctcttta aacacccgaa gcgca 25

<210> 264
<211> 25
<212> DNA
<213> Homo sapiens

<400> 264
aactgttcct gactttatac tattt 25

<210> 265
<211> 25
<212> DNA
<213> Homo sapiens

<400> 265
gcataatgat attcacatcc cctca 25

<210> 266
<211> 25
<212> DNA
<213> Homo sapiens

<400> 266
tgttgtttct gcactttata ataaa 25

<210> 267
<211> 25
<212> DNA
<213> Homo sapiens

<400> 267
agaggtgggg ctggatgtct ccatc 25

<210> 268
<211> 25
<212> DNA
<213> Homo sapiens

<400> 268
aagtgcaaag ttattcccca tcttc 25

<210> 269
<211> 25
<212> DNA
<213> Homo sapiens

<400> 269
tacttacacc caaacagatc ctgaa 25

<210> 270
<211> 25
<212> DNA
<213> Homo sapiens

<400> 270
ttgcgtgtgg agctgtattc ccgag 25

<210> 271
<211> 25
<212> DNA
<213> Homo sapiens

<400> 271
ccctttcact gttctcacag gacat 25

<210> 272
<211> 25
<212> DNA
<213> Homo sapiens

<400> 272
gttggctgat attggagtgc tcatt 25

<210> 273
<211> 25
<212> DNA
<213> Homo sapiens

<400> 273
cagcagggtt tcaggttcca atcag 25

<210> 274
<211> 25
<212> DNA
<213> Homo sapiens

<400> 274
ctgtatttgt ggtctctgta tttat 25

<210> 275
<211> 25
<212> DNA
<213> Homo sapiens

<400> 275
acatccaaaa tgacggctgc tatat 25

<210> 276
<211> 25
<212> DNA
<213> Homo sapiens

<400> 276
gtggacccta ctattcatgt tttga 25

<210> 277
<211> 25
<212> DNA
<213> Homo sapiens

<400> 277
gcttaaactt acgtgcctta caggt 25

<210> 278
<211> 25
<212> DNA
<213> Homo sapiens

<400> 278
catgcagact gttagctttt acctt 25

<210> 279
<211> 25
<212> DNA
<213> Homo sapiens

<400>  279
gtcagtgcat aaagacatac tccaa  25

<210>  280
<211>  25
<212>  DNA
<213>  Homo sapiens

<400>  280
ggagtcctat ttgcagaacc acttt  25

<210>  281
<211>  25
<212>  DNA
<213>  Homo sapiens

<400>  281
ataaccaacc tattgcctat gagaa  25

<210>  282
<211>  25
<212>  DNA
<213>  Homo sapiens

<400>  282
cgaattagtc tccagcctct aaata  25

<210>  283
<211>  25
<212>  DNA
<213>  Homo sapiens

<400>  283
tcgggtctct ccataattca gccca  25

<210>  284
<211>  25
<212>  DNA
<213>  Homo sapiens

<400>  284
acgattgcct tcagtttgtg ttgtg  25

<210>  285
<211>  25
<212>  DNA
<213>  Homo sapiens

<400>  285
aattcttttt attggtgcct atatt  25

<210>  286
<211>  25
<212>  DNA
<213>  Homo sapiens

<400> 286
aagctcactg gcatggcctt ccgtg 25

<210> 287
<211> 25
<212> DNA
<213> Homo sapiens

<400> 287
gagatatttc tgaattactg ttgta 25

<210> 288
<211> 25
<212> DNA
<213> Homo sapiens

<400> 288
tttgacgctg gggctggcat tgccc 25

<210> 289
<211> 25
<212> DNA
<213> Homo sapiens

<400> 289
ggacagcaga ctgccggtaa cgcgc 25

<210> 290
<211> 25
<212> DNA
<213> Homo sapiens

<400> 290
gctctgaagt caccaccaaa atgct 25

<210> 291
<211> 25
<212> DNA
<213> Homo sapiens

<400> 291
ggtgccaatt tcaagttcca agttg 25

<210> 292
<211> 25
<212> DNA
<213> Homo sapiens

<400> 292
tagggagccg caccttgtca tgtac 25

<210> 293
<211> 25
<212> DNA
<213> Homo sapiens

<400> 293
acgtatattt acctgtgact tgtat 25

<210> 294
<211> 25
<212> DNA
<213> Homo sapiens

<400> 294
taaactgctg cccgtagagg ccttt 25

<210> 295
<211> 25
<212> DNA
<213> Homo sapiens

<400> 295
tggatgttag cggtactctt ccact 25

<210> 296
<211> 25
<212> DNA
<213> Homo sapiens

<400> 296
ttttcctgga tatctgtgta ttttc 25

<210> 297
<211> 25
<212> DNA
<213> Homo sapiens

<400> 297
actgtgcgtt gtacatagtt ctaat 25

<210> 298
<211> 25
<212> DNA
<213> Homo sapiens

<400> 298
gaacactggc cataggaaat gctgt 25

<210> 299
<211> 25
<212> DNA
<213> Homo sapiens

<400> 299
gatcctttct gtaggctaat tcctc 25

<210> 300
<211> 25
<212> DNA
<213> Homo sapiens

<400>  300
aacgcagcag aacttgccac atcag                                          25

<210>  301
<211>  25
<212>  DNA
<213>  Homo sapiens

<400>  301
gaagcttggc tttagtggta gaatg                                          25

<210>  302
<211>  25
<212>  DNA
<213>  Homo sapiens

<400>  302
agcctggctc aatatctaat caatg                                          25

<210>  303
<211>  25
<212>  DNA
<213>  Homo sapiens

<400>  303
gaacttcaaa catttgggac cacct                                          25

<210>  304
<211>  25
<212>  DNA
<213>  Homo sapiens

<400>  304
ccaccctagt gtctcatgtt tgtat                                          25

<210>  305
<211>  25
<212>  DNA
<213>  Homo sapiens

<400>  305
tggtgatgtc tgctactatg ccagc                                          25

<210>  306
<211>  25
<212>  DNA
<213>  Homo sapiens

<400>  306
aagctggagg ctgagatcgc cacct                                          25

<210>  307
<211>  25
<212>  DNA
<213>  Homo sapiens

<400> 307
cactttaacc cacgcttcaa tgaga 25

<210> 308
<211> 25
<212> DNA
<213> Homo sapiens

<400> 308
tggaatgagt ctcctttagt ttcag 25

<210> 309
<211> 25
<212> DNA
<213> Homo sapiens

<400> 309
agagacatgt accttgacca tcgtc 25

<210> 310
<211> 25
<212> DNA
<213> Homo sapiens

<400> 310
tggaaatcct ctaggaggct cctcc 25

<210> 311
<211> 25
<212> DNA
<213> Homo sapiens

<400> 311
tgtgtgggtg ctgataattg tgtat 25

<210> 312
<211> 25
<212> DNA
<213> Homo sapiens

<400> 312
tactcttcca tcatctagaa ttgtt 25

<210> 313
<211> 25
<212> DNA
<213> Homo sapiens

<400> 313
gcaccctcag ggtcttaagg tcttc 25

<210> 314
<211> 25
<212> DNA
<213> Homo sapiens

<400> 314
gtacacagtc tgttttctat ttgtt 25

<210> 315
<211> 25
<212> DNA
<213> Homo sapiens

<400> 315
tgggctgcct tatattgtgt gtgtg 25

<210> 316
<211> 25
<212> DNA
<213> Homo sapiens

<400> 316
taacccagac ttaatcttga atgat 25

<210> 317
<211> 25
<212> DNA
<213> Homo sapiens

<400> 317
gagacttcgg cggaccatta ggaat 25

<210> 318
<211> 25
<212> DNA
<213> Homo sapiens

<400> 318
atgcacgtga aacttaacac tttat 25

<210> 319
<211> 25
<212> DNA
<213> Homo sapiens

<400> 319
agttgaacag tgtgccctag ctttt 25

<210> 320
<211> 25
<212> DNA
<213> Homo sapiens

<400> 320
gatcttgtat tcagtcaggt taaaa 25

<210> 321
<211> 25
<212> DNA
<213> Homo sapiens

<400> 321
ccaaagtggt cagggtggcc tctgg 25

<210> 322
<211> 25
<212> DNA
<213> Homo sapiens

<400> 322
ggacgagtcg gaccgaggct aggac 25

<210> 323
<211> 25
<212> DNA
<213> Homo sapiens

<400> 323
atttgtggcc actcactttg tagga 25

<210> 324
<211> 25
<212> DNA
<213> Homo sapiens

<400> 324
tcttgctcca aagggctccg tggag 25

<210> 325
<211> 25
<212> DNA
<213> Homo sapiens

<400> 325
ctcctccatg agtctgacat ctcgg 25

<210> 326
<211> 25
<212> DNA
<213> Homo sapiens

<400> 326
ggttgaggag aggctccaga cccgc 25

<210> 327
<211> 25
<212> DNA
<213> Homo sapiens

<400> 327
acagcaactt gtcaaaccta agcat 25

<210> 328
<211> 25
<212> DNA
<213> Homo sapiens

<400> 328
acgcagagga cgtctctatg ccggt 25

<210> 329
<211> 25
<212> DNA
<213> Homo sapiens

<400> 329
gtagtcattc atttctagct gtaca 25

<210> 330
<211> 25
<212> DNA
<213> Homo sapiens

<400> 330
gtagctgatg aagtatgtcg cattt 25

<210> 331
<211> 25
<212> DNA
<213> Homo sapiens

<400> 331
gatgatcttc tgtggtgctt aagga 25

<210> 332
<211> 25
<212> DNA
<213> Homo sapiens

<400> 332
ctgtgattgc tgccaggcac tgttc 25

<210> 333
<211> 25
<212> DNA
<213> Homo sapiens

<400> 333
ttcacgcgga aatacacgct gcccc 25

<210> 334
<211> 25
<212> DNA
<213> Homo sapiens

<400> 334
tcatctgctg gtccgtggga cggtg 25

<210> 335
<211> 25
<212> DNA
<213> Homo sapiens

<400> 335
gaaggagagc catgcgtact ttcta 25

<210> 336
<211> 25
<212> DNA
<213> Homo sapiens

<400> 336
gtgtatagtg ttttaccctc ttctt 25

<210> 337
<211> 25
<212> DNA
<213> Homo sapiens

<400> 337
caggactttt gttccaggtt gccag 25

<210> 338
<211> 25
<212> DNA
<213> Homo sapiens

<400> 338
gtgcagtttc tgacacttgt tgttg 25

<210> 339
<211> 25
<212> DNA
<213> Homo sapiens

<400> 339
gggagcaccg tgatggagag gacag 25

<210> 340
<211> 25
<212> DNA
<213> Homo sapiens

<400> 340
aaggccaaga tcaccatcgt ggcag 25

<210> 341
<211> 25
<212> DNA
<213> Homo sapiens

<400> 341
tattcaaatg gcccctccag aaagt 25

<210> 342
<211> 25
<212> DNA
<213> Homo sapiens

<400> 342
aagccacatc tgagcacttc aagac 25

<210> 343
<211> 25
<212> DNA
<213> Homo sapiens

<400> 343
gttccaagaa ctctggtgtc tgacc 25

<210> 344
<211> 25
<212> DNA
<213> Homo sapiens

<400> 344
gaggatcatg cccttagcaa gtact 25

<210> 345
<211> 25
<212> DNA
<213> Homo sapiens

<400> 345
ggtctacgcc tattacaacc tggag 25

<210> 346
<211> 25
<212> DNA
<213> Homo sapiens

<400> 346
aatcgttctc cttacaatca agttc 25

<210> 347
<211> 25
<212> DNA
<213> Homo sapiens

<400> 347
ggaactaggc tcttatgtgt gcctt 25

<210> 348
<211> 25
<212> DNA
<213> Homo sapiens

<400> 348
tctgtcctcc taagaaatct gccca 25

<210> 349
<211> 25
<212> DNA
<213> Homo sapiens

<400> 349
gaggctcagt tagcaacctg tgttg 25

<210> 350
<211> 25
<212> DNA
<213> Homo sapiens

<400> 350
agactgtccc tgaaacctag tactg 25

<210> 351
<211> 25
<212> DNA
<213> Homo sapiens

<400> 351
actttctgca tgttgatgcc cgaag 25

<210> 352
<211> 25
<212> DNA
<213> Homo sapiens

<400> 352
gttcttgtat tgtattgccc agggg 25

<210> 353
<211> 25
<212> DNA
<213> Homo sapiens

<400> 353
aaaaaccgca gcccaggttc tgatc 25

<210> 354
<211> 25
<212> DNA
<213> Homo sapiens

<400> 354
gacatgaacg gctgttactc acgcc 25

<210> 355
<211> 25
<212> DNA
<213> Homo sapiens

<400> 355
tgttcttggc gctgtatgtg caggc 25

<210> 356
<211> 25
<212> DNA
<213> Homo sapiens

<400> 356
cttctacagg cttttgggaa gtagg 25

<210> 357
<211> 25
<212> DNA
<213> Homo sapiens

<400> 357
tggaggacac agatgactct ttggt 25

<210> 358
<211> 25
<212> DNA
<213> Homo sapiens

<400> 358
gagtgggtgg ggaattttct cctct 25

<210> 359
<211> 25
<212> DNA
<213> Homo sapiens

<400> 359
ctgcaccttc atcagcggca tgatg 25

<210> 360
<211> 25
<212> DNA
<213> Homo sapiens

<400> 360
tataaaccac tcttcaacag ctggc 25

<210> 361
<211> 25
<212> DNA
<213> Homo sapiens

<400> 361
aatacatccg atgcgtagat tcttg 25

<210> 362
<211> 25
<212> DNA
<213> Homo sapiens

<400> 362
tggcttagcc tgggcaggtc gtgtc 25

<210> 363
<211> 25
<212> DNA
<213> Homo sapiens

<400> 363
gagcagggtc tctctaaagg ggact 25

<210> 364
<211> 25
<212> DNA
<213> Homo sapiens

<400> 364
gtctctgagt tacaaaagtg ctaat 25

<210> 365
<211> 25
<212> DNA
<213> Homo sapiens

<400> 365
accagttttt ggcttactcc tgaga 25

<210> 366
<211> 25
<212> DNA
<213> Homo sapiens

<400> 366
ttgtctacac tcaggctgca gtatt 25

<210> 367
<211> 25
<212> DNA
<213> Homo sapiens

<400> 367
tcctgagaga tacctcacat tccaa 25

<210> 368
<211> 25
<212> DNA
<213> Homo sapiens

<400> 368
ggttagcctt agtttctcag acttg 25

<210> 369
<211> 25
<212> DNA
<213> Homo sapiens

<400> 369
tgtcctcatc tctgcaaagt tcagc 25

<210> 370
<211> 25
<212> DNA
<213> Homo sapiens

<400> 370
ttcccaagga caggcttcag catca 25

<210> 371
<211> 25
<212> DNA
<213> Homo sapiens

<400> 371
ctcctacgca gatgctgtca ctggc 25

<210> 372
<211> 25
<212> DNA
<213> Homo sapiens

<400> 372
tgagcaaggg ggcccgaatc gacca 25

<210> 373
<211> 25
<212> DNA
<213> Homo sapiens

<400> 373
aagcctgctc tcttgcaaag acagc 25

<210> 374
<211> 25
<212> DNA
<213> Homo sapiens

<400> 374
taactataag gtgcctcagt tttcc 25

<210> 375
<211> 25
<212> DNA
<213> Homo sapiens

<400> 375
catatggcat ctctcaactt ttctt 25

<210> 376
<211> 25
<212> DNA
<213> Homo sapiens

<400> 376
gaaagttcag ccagaatctt cgtcc 25

<210> 377
<211> 25
<212> DNA
<213> Homo sapiens

<400> 377
gggcagcttc ctaatatgct ttaca 25

<210> 378
<211> 25
<212> DNA
<213> Homo sapiens

<400> 378
gcctgtcttg agtagacttg gaccc 25

<210> 379
<211> 25
<212> DNA
<213> Homo sapiens

<400> 379
gatccacctc atatgtgagt ccgtc 25

<210> 380
<211> 25
<212> DNA
<213> Homo sapiens

<400> 380
cgctgctgtg tcactcatga ctgtt 25

<210> 381
<211> 25
<212> DNA
<213> Homo sapiens

<400> 381
gcctgttgaa ggtacatcgt ttgca 25

<210> 382
<211> 25
<212> DNA
<213> Homo sapiens

<400> 382
tctcccgatt cccaactgag tgtta 25

<210> 383
<211> 25
<212> DNA
<213> Homo sapiens

<400> 383
aggcaatttt aatcccctgc actag 25

<210> 384
<211> 25
<212> DNA
<213> Homo sapiens

<400> 384
ggacaaactg ccagttttgt ttcct 25

<210> 385
<211> 25
<212> DNA
<213> Homo sapiens

<400> 385
gttcttcaat cagctggcac acact 25

<210> 386
<211> 25
<212> DNA
<213> Homo sapiens

<400> 386
accacccagg aaaccatcga caaga 25

<210> 387
<211> 25
<212> DNA
<213> Homo sapiens

<400> 387
aaggacagaa cattgccttc ctcgt 25

<210> 388
<211> 25
<212> DNA
<213> Homo sapiens

<400> 388
ggatatacaa gttctgtggt ttcag 25

<210> 389
<211> 25
<212> DNA
<213> Homo sapiens

<400> 389
gtggttggca ctagactggt ggcag 25

<210> 390
<211> 25
<212> DNA
<213> Homo sapiens

<400> 390
atgtacttgt tctacctgga ttgtc 25

<210> 391
<211> 25
<212> DNA
<213> Homo sapiens

<400> 391
cgcggctgca tgacactgga ggtca 25

<210> 392
<211> 25
<212> DNA
<213> Homo sapiens

<400> 392
ggtgcccgga tccagagtgt gaaga 25

<210> 393
<211> 25
<212> DNA
<213> Homo sapiens

<400> 393
ctccatccta tgctgctaca attgc 25

<210> 394
<211> 25
<212> DNA
<213> Homo sapiens

<400> 394
tgaatgccag tccagatgtg cctag 25

<210> 395
<211> 25
<212> DNA
<213> Homo sapiens

<400> 395
ttacattcat ttaacctgcc gatta 25

<210> 396
<211> 25
<212> DNA
<213> Homo sapiens

<400> 396
cacccagtgg tactttggtg cctac 25

<210> 397
<211> 25
<212> DNA
<213> Homo sapiens

<400> 397
agtgctggtc actaaccaac aaggt 25

<210> 398
<211> 25
<212> DNA
<213> Homo sapiens

<400> 398
ctctctgaaa cgcttcctat aactc 25

<210> 399
<211> 25
<212> DNA
<213> Homo sapiens

<400> 399
ttgtcagttc tcaatttcct gtgtt 25

<210> 400
<211> 25
<212> DNA
<213> Homo sapiens

<400> 400
attatcgctt tatgtttttg gttat 25

<210> 401
<211> 25
<212> DNA
<213> Homo sapiens

<400> 401
ggggctcaag ggcaagatca gcgag 25

<210> 402
<211> 25
<212> DNA
<213> Homo sapiens

<400> 402
gaataggtat cttgagcttg gttct 25

<210> 403
<211> 25
<212> DNA
<213> Homo sapiens

<400> 403
acactcgctt ctggaacgtc tgagg 25

<210> 404
<211> 25
<212> DNA
<213> Homo sapiens

<400> 404
aatctatatt tgtcttccga tcaac 25

<210> 405
<211> 25
<212> DNA
<213> Homo sapiens

<400> 405
acaaggccca ggctggggcc agggc 25

## Description

### FIELD OF THE INVENTION

The invention pertains to methods of treating cancer in subjects in need thereof and using biomarkers to predict responsiveness of a cancer to a cancer treatment.

### BACKGROUND

Cancer remains one of the deadliest threats to human health. In 2013, the global cancer burden was estimated to be at least 14.1 million new cases and 8.2 million cancer deaths. These statistics are predicted to increase further by 2025. An effective treatment strategy is needed.

Cisplatin, an inorganic platinum-based anti-neoplastic agent, is one of the most effective and widely used anticancer drugs in the world and is commonly used for the treatment of a wide variety of cancers, such as breast, testicular, lung and ovarian cancers. A major obstacle to widespread use of cisplatin is the persistence of severe toxic side effects. Thus, there exists a need for improved cisplatin formulations and dosage regimens for treating cancer that produce fewer toxic side effects. Methods for determining whether a cancer will be responsive to a cisplatin therapy are also needed.

### SUMMARY OF THE INVENTION

Featured are methods for treating cancer using two doses of a liposomal cisplatin formulation (e.g., LiPlaCis) given on day 1 and day 8 of a three week treatment cycle. Also featured are methods for determining the responsiveness of a subject (e.g., a human) with a cancer (e.g., breast cancer) to treatment with the liposomal cisplatin formulation (e.g., LiPlaCis) by detecting a level of one or more biomarkers of sensitivity and/or resistance, such as the biomarkers set forth in one or more of Tables 2-5.

In a first aspect is a method of treating a subject (e.g., a human) with a cancer by administering to the subject at least two doses (e.g., first and second doses) of a composition that contains a secretory phospholipase A2 ($sPLA_2$) hydrolysable, cisplatin-containing liposome (e.g., LiPlaCis) on day 1 and day 8, respectively, of at least one three week treatment cycle, in which each of the doses of the liposomal composition contain cisplatin in an amount of about 75 mg to about 90 mg, or cisplatin in an amount of about 40 $mg/m^2$ body surface area to about 55 $mg/m^2$ body surface area of the subject.

In some embodiments of the first aspect, the first and/or second doses of the liposomal composition contain about 75 mg cisplatin. In other embodiments, the first and/or second doses of the liposomal composition contain about 90 mg cisplatin.

In other embodiments of the first aspect, the first and/or second doses of the composition contain cisplatin in an amount of about 40 $mg/m^2$ body surface area of the subject. In other embodiments, the first and/or second doses of the liposomal composition contain cisplatin in an amount of about 55 $mg/m^2$ body surface area of the subject.

The method may also involve administering the liposomal composition in an amount that provides about 150 mg to about 180 mg cisplatin to the subject in each three week treatment cycle. In some embodiments, an amount of about 150 mg cisplatin or an amount of about 180 mg cisplatin is administered to the subject in each three week treatment cycle.

In some embodiments of the first aspect, the method further includes the step of administering one or more additional therapies to the subject prior to, concurrently with, or after administration of the liposomal composition. The additional therapies may include surgery, radiation, or a therapeutic agent. The therapeutic agent may be selected from the group consisting of docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, and rituximab.

In some embodiments of the first aspect, the liposomal composition is administered to the subject intravenously, intramuscularly, transdermally, intradermally, intra-arterially, intracranially, subcutaneously, intraorbitally, intraventricularly, intraspinally, intraperitoneally, or intranasally. For example, the liposomal composition is administered to the subject by intravenous infusion. In some embodiments, the liposomal composition is administered to the subject over a period of about 2-3 hours. For example, the composition is administered to the subject as a 2 or 3 hour infusion.

In some embodiments of the first aspect, the three week treatment cycle is repeated two to twenty times. For example, the three week treatment cycle can be repeated two times, three times, four times, five times, ten times, fifteen times, or twenty times. Each three week treatment cycle can begin immediately after the conclusion of the prior three week cycle or one or more of the three week cycles can be separated by a period of a day (e.g., 1-6 days), a week (e.g., 1-4 weeks), a month (e.g., 1-12 months), or a year.

In some embodiments of the first aspect, the subject has been determined to be responsive to the liposomal composition (e.g., LiPlaCis) prior to administration of the liposomal composition.

In other embodiments of the first aspect, the method of treating a subject with cancer with the liposomal composition (e.g., LiPlaCis) further includes the step of determining the responsiveness of the subject to the liposomal composition. Responsiveness of the subject to the liposomal composition can be determined, e.g., by contacting a sample from the subject (e.g., a sample containing one or more nucleic acid molecules from the subject, such as a tumor sample) with a device that contains (i) one or more single-stranded nucleic acid molecules capable of specifically hybridizing with nucleotides of one or more biomarkers of sensitivity selected from those listed in Tables 2 and/or 4, or a complement thereof; and/or (ii) one or more single-stranded nucleic acid molecules capable of specifically hybridizing with nucleotides of one or more biomarkers of resistance selected from those listed in Tables 3 and/or 5, or a complement thereof. The level of the one or more biomarkers of sensitivity or the complement thereof and/or the level of the one or more biomarkers of resistance, or a complement thereof, in the sample is detected by, e.g., detecting hybridization between the one or more single-stranded nucleic acid molecules of the device and the one or more nucleic acid molecules of the sample. In some embodiments, the one or more biomarkers of sensitivity is not C1QR1 (SEQ ID NO: 13), SLA (SEQ ID NO: 48), PTPN7 (SEQ ID NO: 77), CENTB1 (SEQ ID NO: 37), IFI16 (SEQ ID NO: 17 or 261), ARHGEF6 (SEQ ID NO: 36 or 294), CD3D (SEQ ID NO: 81), ARHGAP15 (SEQ ID NO: 30), HCLS1 (SEQ ID NO: 16 or 259), CD53 (SEQ ID NO: 282), PTPRCAP (SEQ ID NO: 8), and/or PTPRC (SEQ ID NO: 10, 18, 25, or243).

In some embodiments of the first aspect, the subject is determined to be responsive to the liposomal composition (e.g., LiPlaCis) if: i) the level of the biomarker(s) of sensitivity, or the complement thereof, is substantially similar to the level of the biomarker(s) of sensitivity, or the complement thereof, in a cell or tissue known to be sensitive to the liposomal composition; and/or ii) the level of the biomarker(s) of resistance, or the complement thereof, is substantially dissimilar to the level of the biomarker(s) of resistance, or the complement thereof, in a cell or tissue known to be resistant to the liposomal composition.

In some embodiments, the responsiveness of the subject to the liposomal composition is determined by detecting the level of PLA2G2A (SEQ ID NO: 380), or a complement thereof, in the sample from the subject. For example, the responsiveness of the subject to the liposomal composition can be determined by detecting the level of PLA2G2A (SEQ ID NO: 380), or a complement thereof by performing microarray analysis or qRT-PCR.

In other embodiments, the method of determining the responsiveness of the subject to the liposomal composition (e.g., LiPlaCis) includes the step of detecting $sPLA_2$ protein in a tumor sample from the subject. The sPLA2 protein can be detected by contacting the tumor sample with an anti-$sPLA_2$ antibody and detecting binding between the $sPLA_2$ protein and the anti-$sPLA_2$ antibody. The method may include detecting the level of one or more biomarkers of sensitivity and/or resistance (Tables 2-5) in a sample from the subject and detecting the level of $sPLA_2$ protein in a tumor sample from the subject. In yet other embodiments, the method further includes the step of administering one or more cancer therapies other than the liposomal composition (e.g., LiPlaCis) to the subject when the subject is determined to be responsive to the liposomal composition.

In some embodiments of the first aspect, the cell (e.g., a cancer cell) or tissue (e.g., a tumor tissue) known to be sensitive to the liposomal composition and/or the cell or tissue known to be resistant to the liposomal composition is of the same type as a cell or tissue in the sample from the patient or from which the one or more nucleic acid molecules of the sample are derived. In particular, the cell (e.g., a cancer cell) or tissue (e.g., a tumor tissue) known to be sensitive to the liposomal composition and/or the cell or tissue known to be resistant to the liposomal composition is of the same type of cancer (e.g., breast cancer) as a cell or tissue in the sample from the subject or from which the one or more nucleic acid molecules of the sample are derived, which can provide, e.g., a control from which to assess whether the subject will be sensitive or resistant to the liposomal composition.

In some embodiments, the sample from the subject is a tumor sample. In some embodiments, the subject is resistant to one or more cancer therapies (e.g., surgery, radiation, or a therapeutic agent) other than the liposomal composition (e.g., LiPlaCis).

In some embodiments of the first aspect, the cancer is selected from a solid tumor cancer and a hematological cancer. For example, the cancer can be breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, renal cell carcinoma (RCC), esophageal cancer, melanoma, glioma, pancreatic cancer, gastrointestinal stromal tumors (GIST), sarcoma, non-small cell lung carcinoma (NSCLC), prostate cancer, ovarian cancer, colon cancer, bladder cancer, and squamous cell carcinoma of the head and neck (SCCHN). In particular, the cancer can be breast cancer, such as an estrogen receptor-positive (ERpos) breast cancer and/or a metastatic form of breast cancer.

In some embodiments, the subject may exhibit cancer relapse (e.g., relapse of breast cancer), such as relapse after a first cancer treatment and prior to treatment with the liposomal composition (e.g., LiPlaCis). Alternatively, the subject may have not been administered any treatment for cancer prior to administration of the liposomal composition (e.g., LiPlaCis). Additionally, the responsiveness of the subject to the liposomal composition may not have been determined prior to treatment and/or may be determined during or after a cancer treatment (e.g., treatment with cisplatin, such as with LiPlaCis).

In some embodiments, the device for determining the responsiveness of a subject to treatment with a liposomal composition described herein (e.g., LiPlaCis) can include at least two, at least three, at least four, at least five, at least six, at least seven, at least eight, at least nine, at least ten, or more single-stranded nucleic acid molecules capable of specifically hybridizing with the nucleotides of one or more biomarkers of sensitivity selected from the biomarkers of Tables 2 and 4, or a complement thereof (e.g., COL5A2 (SEQ ID NO: 73 or 211); and/or at least two, at least three, at least four, at least five, at least six, at least seven, at least eight, at least nine, at least ten, or more single-stranded nucleic acid molecules capable of specifically hybridizing with the nucleotides of one or more biomarkers of resistance selected from the biomarkers of Tables 3 and 5, or a complement thereof (e.g., SFN (SEQ ID NO: 96 OR 324)). In particular, one or more of the single-stranded nucleic acid molecules of the device may have a length in the range of 10 to 100 nucleotides (e.g., .a length in the range of 20 to 60 nucleotides). The one or more single-stranded nucleic acid molecules may also be labeled and/or immobilized on a solid substrate.

In some embodiments, the method for determining the responsiveness of a subject to treatment with a liposomal composition described herein (e.g., LiPlaCis) may include converting the level of the one or more biomarkers of sensitivity, or the complement thereof (e.g., one, two, three, four, five, ten, twenty, or all of the biomarkers shown in Tables 2 and 4, such as COL5A2 (SEQ ID NO: 73 or 211)), and/or the one or more biomarkers of resistance, or the complement thereof (e.g., one, two, three, four, five, ten, twenty, or all of the biomarkers shown in Tables 3 and 5, such as SFN (SEQ ID NO: 96 OR 324)), into a mean score, in which the mean score indicates the responsiveness of the subject to the liposomal composition (e.g., LiPlaCis). The method can further include subtracting the mean score for one or more of the biomarkers of resistance (e.g., one, two, three, four, five, ten, twenty, or all of the biomarkers shown in Tables 3 and 5, such as SFN (SEQ ID NO: 96 OR 324)) from the mean score for one or more of the biomarkers of sensitivity (e.g., one, two, three, four, five, ten, twenty, or all of the biomarkers shown in Tables 2 and 4, such as COL5A2 (SEQ ID NO: 73 or 211) to obtain a difference score, in which the difference score indicates the responsiveness of the subject to the liposomal composition. In particular, the mean score and/or the difference score above a cutoff value (e.g., a cutoff value of about 0.1, about 0.15, about 0.2, about 0.25, about 0.3, about 0.35, about 0.4, about 0.45, about 0.5, or greater) indicates that the subject is responsive to the liposomal composition.

In other embodiments, the device is a microarray, such as a deoxyribonucleic acid (DNA)-based platform. Alternatively, the device is for performing a qRT-PCR reaction (e.g., the device is used with a system for detecting the amplification product, for example, by fluorescence or by another method). The methods may also utilize both a microarray and a qRT-PCR device. Thus, the level of the biomarker(s) of sensitivity (e.g., one, two, three, four, five, ten, twenty, or all of the biomarkers shown in Tables 2 and 4, such as COL5A2 (SEQ ID NO: 73 or 211), and/or the biomarker(s) of resistance (e.g., one, two, three, four, five, ten, twenty, or all of the biomarkers shown in Tables 3 and 5, such as SFN (SEQ ID NO: 96 OR 324)), can be measured using qRT-PCR. In particular, the level of the one or more biomarkers of sensitivity, or the complement thereof (e.g., one, two, three, four, five, ten, twenty, or all of the biomarkers shown in Tables 2 and 4, such as COL5A2 (SEQ ID NO: 73 or 211)), and/or the one or more biomarkers of resistance, or the complement thereof (e.g., one, two, three, four, five, ten, twenty, or all of the biomarkers shown in Tables 3 and 5, such as SFN (SEQ ID NO: 96 OR 324)), are detected by performing microarray analysis or qRT-PCR. Additionally, the nucleic acid molecules of the sample may include mRNA or a cDNA thereof.

In still other embodiments, the biomarker of sensitivity may be selected from one or more of COL5A2 (SEQ ID NO: 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), EBI2 (SEQ ID NO: 9), ANP32E (SEQ ID NO: 11), SFPQ (SEQ ID NO: 12, 38 or 272), FNBP1 (SEQ ID NO: 14 or 28), CBFB (SEQ ID NO: 15), SFRS7 (SEQ ID NO: 19 or 54), and CAP350 (SEQ ID NO: 20 or 61). The biomarker of resistance may be selected from one or more of S SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), and LRP5 (SEQ ID NO: 112).

For example, the biomarkers of sensitivity may include COL5A2 (SEQ ID NO 73 or 211) and ITGA4 (SEQ ID NO: 1). The biomarkers of sensitivity may include COL5A2 (SEQ ID NO 73 or 211), ITGA4 (SEQ ID NO: 1), and MSN (SEQ ID NO: 2). The biomarkers of sensitivity may include COL5A2 (SEQ ID NO 73 or 211), ITGA4 (SEQ ID NO: 1), MSN (SEQ ID NO: 2), and FAM46A (SEQ ID NO: 3 OR 280). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), and ITGB2 (SEQ ID NO: 4). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), and DOCK2 (SEQ ID NO: 5 OR 223). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), and EVL (SEQ ID NO: 6). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), and SACS (SEQ ID NO: 7). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), and PTPRCAP (SEQ ID NO: 8). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), PTPRCAP (SEQ ID NO: 8), and EBI2 (SEQ ID NO: 9). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), PTPRCAP (SEQ ID NO: 8), EBI2 (SEQ ID NO: 9), and PTPRC (SEQ ID NO: 10, 18, 25, OR 243). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, OR 243), and ANP32E (SEQ ID NO: 11). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, OR 243), ANP32E (SEQ ID NO: 11), and SFPQ (SEQ ID NO: 12, 38 OR 272). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, OR 243), ANP32E (SEQ ID NO: 11), SFPQ (SEQ ID NO: 12, 38 OR 272), and C1QR1 (SEQ ID NO: 13). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 OR 280), ITGB2 (SEQ ID NO: 4), DOCK2 (SEQ ID NO: 5 OR 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, OR 243), ANP32E (SEQ ID NO: 11), SFPQ (SEQ ID NO: 12, 38 OR 272), C1QR1 (SEQ ID NO: 13), and FNBP1 (SEQ ID NO: 14 OR 28). The biomarkers of sensitivity may include COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), and CBFB (SEQ ID NO: 15). The biomarkers of sensitivity may include COL5A2 (SEQ ID NO: 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), EBI2 (SEQ ID NO: 9), ANP32E (SEQ ID NO: 11), SFPQ (SEQ ID NO: 12, 38 or 272), FNBP1 (SEQ ID NO: 14 or 28), CBFB (SEQ ID NO: 15), and SFRS7 (SEQ ID NO: 19 or 54). The biomarkers of sensitivity may include COL5A2 (SEQ ID NO: 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), EBI2 (SEQ ID NO: 9), ANP32E (SEQ ID NO: 11), SFPQ (SEQ ID NO: 12, 38 or 272), FNBP1 (SEQ ID NO: 14 or 28), CBFB (SEQ ID NO: 15), SFRS7 (SEQ ID NO: 19 or 54), and CAP350 (SEQ ID NO: 20 or 61).

For example, the biomarkers of resistance may include SFN (SEQ ID NO: 96 or 324) and LISCH7 (SEQ ID NO: 97). The biomarkers of resistance may include SFN (SEQ ID NO: 96 or 324), LISCH7 (SEQ ID NO: 97), and EPB41L4B (SEQ ID NO: 98). The biomarkers of resistance may include SFN (SEQ ID NO: 96 or 324), LISCH7 (SEQ ID NO: 97), EPB41L4B (SEQ ID NO: 98), and MST1R (SEQ ID NO: 99). The biomarkers of resistance may include SFN (SEQ ID NO: 96 or 324), LISCH7 (SEQ ID NO: 97), EPB41L4B (SEQ ID NO: 98), MST1R (SEQ ID NO: 99), and ITGB4 (SEQ ID NO: 100). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), and DBNDD2 (SEQ ID NO: 102 OR 365). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), and TACSTD1 (SEQ ID NO: 104). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), and MISP (SEQ ID NO: 105). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), and KRT8 (SEQ ID NO: 106). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), KRT8 (SEQ ID NO: 106), and JUP (SEQ ID NO: 107 OR 400). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), KRT8 (SEQ ID NO: 106), JUP (SEQ ID NO: 107 OR 400), and KRT18 (SEQ ID NO: 108 OR 306. The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), KRT8 (SEQ ID NO: 106), JUP (SEQ ID NO: 107 OR 400), KRT18 (SEQ ID NO: 108 OR 306, and FA2H (SEQ ID NO: 109). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), KRT8 (SEQ ID NO: 106), JUP (SEQ ID NO: 107 OR 400), KRT18 (SEQ ID NO: 108 OR 306, FA2H (SEQ ID NO: 109), and MGAT4B (SEQ ID NO: 110). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), KRT8 (SEQ ID NO: 106), JUP (SEQ ID NO: 107 OR 400), KRT18 (SEQ ID NO: 108 OR 306, FA2H (SEQ ID NO: 109), MGAT4B (SEQ ID NO: 110), and DSG2 (SEQ ID NO:111 OR 312). The biomarkers of resistance may include SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 OR 365), TACSTD1 (SEQ ID NO: 104), MISP (SEQ ID NO: 105), KRT8 (SEQ ID NO: 106), JUP (SEQ ID NO: 107 OR 400), KRT18 (SEQ ID NO: 108 OR 306, FA2H (SEQ ID NO: 109), MGAT4B (SEQ ID NO: 110), DSG2 (SEQ ID NO:111 OR 312), and LRP5 (SEQ ID NO: 112).

A second aspect features a composition containing an $sPLA_2$ hydrolysable, cisplatin-containing liposome (e.g., LiPlaCis) for use in treating cancer in a subject (e.g., a human, such as a human with cancer), in which the composition is formulated for administration in at least two doses (e.g., first and second doses). Each of the doses contains cisplatin in an amount of about 75 mg to about 90 mg, or cisplatin in an amount of about 40 $mg/m^2$ body surface area to about 55 $mg/m^2$ body surface area. The doses of the formulation are characterized as being prepared for administration to the subject on day 1 and day 8, respectively, of at least one three week treatment cycle.

A third aspect features a use of a composition containing an $sPLA_2$ hydrolysable, cisplatin-containing liposome (e.g., LiPlaCis) in the manufacture of a medicament for treating cancer in a subject in need thereof (e.g., a human, such as a human with cancer). The composition is formulated for administration in at least two doses (e.g., first and second doses). Each of the doses contain cisplatin in an amount of about 75 mg to about 90 mg or cisplatin in an amount of about 40 $mg/m^2$ body surface area to about 55 $mg/m^2$ body surface area. The doses of the formulation are characterized as being prepared for administration on day 1 and day 8, respectively, of at least one three week treatment cycle.

A fourth aspect features a kit containing: i) a composition containing an $sPLA_2$ hydrolysable, cisplatin-containing liposome (e.g., LiPlaCis) for use in treating cancer in a subject in need thereof (e.g., a human, such as a human with cancer), in which the composition is present in the kit in a concentrated form that can be diluted into at least two doses (e.g., first and second doses). Each of the doses contain cisplatin in an amount of about 75 mg to about 90 mg or cisplatin in an amount of about 40 $mg/m^2$ body surface area to about 55 $mg/m^2$ body surface area. The liposomal composition in the kit may also be diluted to a ready to use form that can be divided into the two doses without the need for dilution. The kit also, optionally, contains instructions for administering the composition to the subject, e.g., a first dose of the compositionon day 1 and a second dose of the composition on day 8 of at least one three week treatment cycle.

All of the embodiments discussed above in connection with the first aspect are equally applicable to each of the second, third, and fourth aspects.

**Definitions**

As used herein, "a" or "an" means "at least one" or "one or more" unless otherwise indicated. In addition, the singular forms "a", "an", and "the" include plural referents unless the context clearly dictates otherwise.

As used herein, "about" refers to an amount that is $\pm$ 10% of the recited value.

By "biomarker" is meant a nucleic acid molecule (e.g., a mRNA or its complement, for example, a cDNA) or a protein encoded by the nucleic acid molecule that is present in, or is from, a cell or tissue (e.g., a cancer cell or a tumor tissue). The expression of the biomarker correlates to the responsiveness (e.g., sensitivity or resistance) of the cell or tissue (and, thus, the patient in which the cell or tissue resides or the patient from which the cell or tissue was obtained) to a cancer treatment (e.g., LiPlaCis). In particular, a biomarker of sensitivity is a nucleic acid molecule (e.g., a mRNA or its complement) expressed from any one of the genes shown in Tables 2 and 4, or the protein encoded by the nucleic acid molecule, and a biomarker of resistance is a nucleic acid molecule (e.g., a mRNA or its complement) expressed from any one of the genes shown in Tables 3 and 5, or the protein encoded by the nucleic acid molecule.

The terms "cancer" and "cancerous" refer to or describe the physiological condition in mammals (e.g., humans) that is typically characterized by unregulated cell proliferation. Examples of cancer include, but are not limited to, prostate cancer, ovarian cancer (e.g., ovarian adenocarcinoma or embryonal carcinoma), liver cancer (e.g., hepatocellular carcinoma (HCC) or hepatoma), myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. The term cancer includes solid tumors (e.g., breast cancer) and hematological cancers (e.g., cancer of the blood, such as lymphoma (e.g., cutaneous T-cell lymphoma (CTCL)).

The terms "expression level" and "level of expression," as used herein, refer to the amount of a gene product (e.g., DNA, RNA (e.g. messenger RNA (mRNA)), or a protein encoded by a given gene) in a cell (e.g., a cancer cell), a tissue (e.g., a tumor tissue), a biological sample, or a subject (e.g., a human, such as a human with cancer).

"Gene" as used herein indicates a coding or noncoding gene whose activity can be determined by measuring the produced RNA. Examples include protein coding genes, microRNAs, small nuclear RNAs and other RNAs with catalytic, regulatory or coding properties.

As used herein, "inhibit growth" means causing a reduction in cell growth (e.g., cancer cell growth, which can be assessed using, e.g., the NCI60 cancer cell lines) *in vivo* or *in vitro* by, e.g., 10%, 20%, 30%, 40%, 50%, 60%, 70%, 80%, 90%, 95%, or 99% or more, as evident by a reduction in the proliferation of cells exposed to a treatment (e.g., an $sPLA_2$ hydrolysable, cisplatin-containing liposome described herein), relative to the proliferation of cells in the absence of the treatment. Growth inhibition may be the result of a treatment (e.g., treatment with the $sPLA_2$ hydrolysable, cisplatin-containing liposome) that induces apoptosis in a cell, induces necrosis in a cell, slows cell cycle progression, disrupts cellular metabolism, induces cell lysis, or induces some other mechanism that reduces the proliferation of cells.

As used herein, the term "microarray" refers to a device employed by any method that quantifies one or more subject oligonucleotides, e.g., RNA, DNA, cDNA, or analogues thereof, at a time. For example, many DNA microarrays, including those made by Affymetrix (e.g., an Affymetrix HG-U133A or HG-U133_Plus_2 array), use several probes for determining the level of a single biomarker. The DNA microarray may contain oligonucleotide probes that may be, e.g., full-length cDNAs complementary to an RNA or cDNA fragments that hybridize to part of an RNA. The DNA microarray may also contain modified versions of DNA or RNA, such as locked nucleic acids or LNA. Exemplary RNAs include mRNA, miRNA, and miRNA precursors.

As used herein, the term "NCI60" refers to a panel of 60 cancer cell lines from lung, colon, breast, ovarian, leukemia, renal, melanoma, prostate, and brain cancers including the following cancer cell lines: NSCLC_NCIH23, NSCLC_NCIH522, NSCLC_A549ATCC, NSCLC_EKVX, NSCLC_NCIH226, NSCLC_NCIH332M, NSCLC_H460, NSCLC_HOP62, NSCLC_HOP92, COLON_HT29, COLON_HCC-2998, COLON_HCT116, COLON_SW620, COLON_COLO205, COLON_HCT15, COLON_KM12, BREAST_MCF7, BREAST_MCF7ADRr, BREAST_MDAMB231, BREAST_HS578T, BREAST_MDAMB435, BREAST_MDN, BREAST_BT549, BREAST_T47D, OVAR_OVCAR3, OVAR_OVCAR4, OVAR_OVCAR5, OVAR_OVCAR8, OVAR_IGROV1, OVAR_SKOV3, LEUK_CCRFCEM, LEUK_K562, LEUK_MOLT4, LEUK_HL60, LEUK_RPMI8266, LEUK_SR, RENAL_UO31, RENAL_SN12C, RENAL_A498, RENAL_CAKI1, RENAL_RXF393, RENAL_7860, RENAL_ACHN, RENAL_TK10, MELAN_LOXIMVI, MELAN_MALME3M, MELAN_SKMEL2, MELAN_SKMEL5, MELAN_SKMEL28, MELAN_M14, MELAN_UACC62, MELAN_UACC257, PROSTATE_PC3, PROSTATE_DU145, CNS_SNB19, CNS_SNB75, CNS_U251, CNS_SF268, CNS_SF295, and CNS_SF539.

The terms "patient" and "subject," as used interchangeably herein, refer to any animal (e.g., a mammal, such as a human, e.g., a human with a cancer). A patient to be treated or tested for responsiveness to a treatment (e.g., treatment with an $sPLA_2$ hydrolysable, cisplatin-containing liposome, such as LiPlaCis) according to the methods described herein may be one who has been diagnosed with a cancer, such as those described herein, e.g., breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, renal cell carcinoma (RCC), esophageal cancer, melanoma, glioma, pancreatic cancer, gastrointestinal stromal tumors (GIST), sarcoma, non-small cell lung carcinoma (NSCLC), prostate cancer, ovarian cancer, colon cancer, bladder cancer, or squamous cell carcinoma of the head and neck (SCCHN). Diagnosis may be performed by any method or technique known in the art, such as x-ray, MRI, or biopsy, and may also be confirmed by a physician. To minimize exposure of a patient to drug treatments that may not be therapeutic, the patient may be determined to be either responsive or non-responsive to a cancer treatment, such as treatment with an $sPLA_2$ hydrolysable, cisplatin-containing liposome, according to the methods described herein, prior to treatment.

As used herein, the term "percent (%) sequence identity" refers to the percentage of nucleic acid residues of a candidate sequence, e.g., a probe or primer of the invention, that are identical to the nucleic acid residues of a reference sequence, e.g., a biomarker sequence of the invention, after aligning the sequences and introducing gaps, if necessary, to achieve the maximum percent sequence identity (e.g., gaps can be introduced in one or both of the candidate and reference sequences for optimal alignment and non-homologous sequences can be disregarded for comparison purposes). Alignment for purposes of determining percent sequence identity can be achieved in various ways that are within the skill in the art, for instance, using computer software, such as BLAST, BLAST-2, BLAST-P, BLAST-N, BLAST-X, WU-BLAST-2, ALIGN, ALIGN-2, CLUSTAL, Megalign (DNASTAR). In addition, those skilled in the art can determine appropriate parameters for measuring alignment, including any algorithms needed to achieve optimal alignment over the length of the sequences being compared.

"Resistant" or "resistance" as used herein means that a cell (e.g., a cancer cell), a tissue containing the cell (e.g., a tumor), or the cell or tissue in a patient (e.g., a human with cancer) is non-responsive to treatment with an anti-cancer agent (e.g., an $sPLA_2$ hydrolysable, cisplatin-containing liposome, such as LiPlaCis). In particular, the treatment reduces the growth of a resistant cell (e.g., the cancer cell) *in vitro* by less than about 40%, 30%, 20%, 10%, 5%, 1%, or less, relative to the growth of a cell or tissue known to be resistant to the treatment or relative to a cell or tissue not exposed to the treatment. Resistance to treatment may be determined by a cell proliferation assay, e.g., a cell-based assay, which measures the growth of treated cells as a function of the absorbance of the cells of an incident light beam, such as the NCI60 assays described herein. In this assay, greater absorbance indicates greater cell growth, and thus, resistance to the treatment.

The terms "responsive" and "responsiveness," as used herein, refer to the likelihood that a cancer treatment (e.g., treatment with an $sPLA_2$ hydrolysable, cisplatin-containing liposome) has a desired effect in a cell (e.g., a cancer cell), a tissue (e.g., a tumor), or a patient with cancer (e.g., a human with cancer). For example, the desired effect can include inhibition of the growth of a cancer cell *in vitro* by more than 10%, 20%, 30%, 40%, 50%, 60%, 70%, 80%, 90%, or 100% relative to the growth of a cancer cell not exposed to the treatment. The desired effect can also include reduction in tumor mass by, e.g., about 10%, 20%, 30%, 40%, 50%, 60%, 70%, 80%, 90%, or 100%. Responsiveness to treatment may be determined by a cell proliferation assay, e.g., a cell-based assay, which measures the growth of treated cells as a function of the absorbance of the cells of an incident light beam, such as the NCI60 assays described herein. In this assay, lesser absorbance indicates lesser cell growth, and thus, sensitivity to the treatment. A greater reduction in growth indicates more sensitivity to the treatment. In particular, "responsiveness" is a measure of the sensitivity or resistance of a patient (e.g., the cancer cells in a patient) to a treatment for cancer (e.g., an $sPLA_2$ hydrolysable, cisplatin-containing liposome, such as LiPlaCis).

The term "sample," as used herein, refers to any specimen (such as cells, tissue (e.g., a tissue sample obtained by biopsy), blood, serum, plasma, urine, cerebrospinal fluid, or pancreatic fluid) taken from a subject (e.g., a subject with a cancer). Preferably, the sample is taken from a portion of the body affected by a cancer (e.g., a biopsy of the cancer tissue, such as breast cancer tissue). Biopsy may involve fine needle aspiration biopsy, core needle biopsy (e.g., stereotactic core needle biopsy, vacuum-assisted core biopsy, or magnetic resonance imaging (MRI) guided biopsy), or surgical biopsy (e.g., incisional biopsy or excisional biopsy). The sample may undergo additional purification and processing, for example, to remove cell debris and other unwanted molecules. Additional processing may further involve producing cDNA molecules corresponding to nucleic acid molecules (e.g., mRNA) in the sample and/or amplification of the nucleic acid molecules, e.g., using PCR, such as RT-PCR. The standard methods of sample purification, such as removal of unwanted molecules, are known in the art.

The terms "secretory phospholipase $A_2$ ($sPLA_2$) hydrolyzable, cisplatin-containing liposome," "$sPLA_2$ hydrolysable, cisplatin-containing liposome," "composition comprising liposomal formulation of cisplatin," "liposomal cisplatin formulation," "the liposomal composition," "the composition," and "the liposome," as used herein refer to an antitumor agent that is a liposomal formulation of cisplatin. The $sPLA_2$ hydrolysable, cisplatin-containing liposome is formulated to release an encapsulated drug (e.g., cisplatin) from the core of a hydrophobic layer into tumor tissue. Since $sPLA_2$ protein is associated with tumor tissue, $sPLA_2$ hydrolysable liposomes may be used to preferentially deliver encapsulated drugs (e.g., cisplatin) to the tumor tissue. Exemplary sPLA2 hydrolysable, cisplatin-containing liposomes include LiPlaCis (LiPlasome Pharma ApS). An $sPLA_2$ hydrolysable, cisplatin-containing liposome is described in, e.g., U.S. Patent Application Publication No. 2012/0177726 and de Jonge et al. (Eur J Cancer. 46(16):3016-21, 2010), each of which is hereby incorporated by reference.

The term "LiPlaCis" as used herein refers to an antitumor agent that is a liposomal formulation of cisplatin. The liposomes - called LiPlasomes - are designed to trigger the release of an encapsulated drug (e.g., cisplatin) specifically in the tumor tissue. An enzyme especially present on tumors called secretory phospholipase A2 (sPLA2), is utilized to break down the liposomes once they have accumulated in the cancer tissue. The lipid composition of LiPlaCis is tailored to be specifically sensitive to degradation by the sPLA2 enzyme and thereby for release of the encapsulated drug. LiPlaCis is also described in de Jonge et al. (Eur J Cancer. 2010 46(16):3016-21) and U.S. Patent Application Publication No. 2012/0177726, hereby incorporated by reference. Exemplary LiPlaCis include LiPlaCis®, LiPlasome Pharma. The liposomes of LiPlaCis contain ~70:25:5 mol% DSPC:DSPG:DSPE-PEG2000 and less than 1% cholesterol.

"Sensitive" and "sensitivity" as used herein refer to a cell (e.g., a cancer cell), a tissue containing the cell (e.g., a tumor), or a patient containing the cell or tissue having cancer (e.g., a human having cancer) that is responsive to treatment, such as an anti-cancer agent (e.g., an $sPLA_2$ hydrolysable, cisplatin-containing liposome) or radiation treatment. In particular, the treatment inhibits the growth of the cell (e.g., the cancer cell) *in vitro* by about 70%, 80%, 90%, 95%, 99% or 100% relative to the growth of a cell not exposed to the treatment. Sensitivity to treatment may be determined by a cell proliferation assay, e.g., a cell-based assay, which measures the growth of treated cells as a function of the absorbance of the cells of an incident light beam, such as the NCI60 assays described herein. In this assay, lesser absorbance indicates lesser cell growth, and thus, sensitivity to the treatment.

The term "specific hybridization" as used herein refers to when complementary nucleic acid sequences form a stable duplex under high stringency conditions, such as high hybridization temperature and low salt in hybridization buffers, which permit only hybridization between nucleic acid sequences that are highly similar. Nucleic acids are referred to as "complementary" that contain nucleotides or nucleotide homologues that can form hydrogen bonds according to Watson-Crick base-pairing rules (e.g., G with C, A with T or A with U) or other hydrogen bonding motifs such as for example diaminopurine with T, 5-methyl C with G, 2-thiothymidine with A, inosine with C, pseudoisocytosine with G, etc. Anti-sense RNA may be complementary to other oligonucleotides, e.g., mRNA.

"Treatment," "medical treatment," to "treat," and "therapy," as used interchangeably herein, refer to administering or exposing a patient with cancer (e.g., a human) to an anti-cancer agent (e.g., a drug, such as an $sPLA_2$ hydrolysable, cisplatin-containing liposome (e.g., LiPlaCis), a protein, an antibody, a nucleic acid, a chemotherapeutic agent, or a radioactive agent), or to some other form of medical intervention used to treat or prevent a disease, disorder, or condition (e.g., surgery, cryotherapy, radiation therapy, or combinations thereof). In particular, a medical treatment can be or can include administration of an $sPLA_2$ hydrolysable, cisplatin-containing liposome, as described herein. For example, the treatment may be of a cancer, such as a solid tumor or a hematological cancer. Examples of cancer include, e.g., breast cancer (e.g., medullary carcinoma or an ER-positive breast cancer), prostate cancer, ovarian cancer (e.g., ovarian adenocarcinoma or embryonal carcinoma), liver cancer (e.g., hepatocellular carcinoma (HCC) or hepatoma), myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, larynx cancer, kidney cancer (e.g., renal cell carcinoma and Wilms tumor), gastric cancer, blastoma (e.g.,

nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. Radiation therapy includes the administration of a radioactive agent to a patient or exposure of a patient to radiation. The radiation may be generated from sources, such as particle accelerators and related medical devices or agents that emit, e.g., X-radiation, gamma radiation, or electron (Beta radiation) beams. A treatment may be or further include surgery, e.g., to remove a tumor from a subject or living organism.

Other features and advantages of the invention will be apparent from the following Detailed Description, the drawings, and the claims.

## BRIEF DESCRIPTION OF THE DRAWINGS

Figure 1 is a graph grouping predicted sensitivity to LiPlaCis by cancer type. Each gray circle represents the predicted LiPlaCis sensitivity of one patient calculated as the difference between the mean of the expression levels of the biomarkers of sensitivity (Table 2) and the mean of the expression levels of the biomarkers of resistance for the patient (Table 3). Patients are grouped according to cancer type. The median predicted sensitivity (black bar) for a cancer type is related to the relative response rate for that cancer type. The predictions are used for relative comparisons to compare cancer types and cannot be used for absolute predictions of response rate for a given cancer type. The predictions are normalized to a scale of 0 to 100 for all 3,522 patients.

Figure 2 is a graph depicting the correlation between DRP score and clinical response (RECIST) in seven patients who had not received prior platinum treatment. When the response is encoded as 3,2,1 for partial response (PR), stable disease (SD), and progressive disease (PD), respectively, the one-sided Pearson correlation is 0.61 (P=0.07, below the significance level of 0.1 defined in the statistical analysis plan). Patients that received prior platinum treatment were excluded from analysis. There are no scores below 33 because patients with a score below 33 were excluded from the trial.

Figure 3 is a graph depicting the Cox proportional hazards of seven patients who had not received prior platinum treatment, stratified by DRP score. The DRP score was used to divide the population in two: those above a cutoff of 67 (upper tertile, N=5) and those between inclusion cutoff of 33 and stratification cutoff of 67 (middle tertile, N=2). These two populations have a dramatic difference in hazard rate (ratio 4e-10, P=0.008). The median time to progression is 25 weeks and 8 weeks, respectively. Because there were no deaths before progression in the evaluable population, time to progression (TTP) and progression-free survival (PFS) are identical in this population.

Figure 4 is a graph comparing the response to LiPlaCis with prior treatment. The hazard ratio is 0.22 (P=0.025 one sided), and median duration of treatment is 25 versus 17 weeks.

Figure 5 is a bar graph showing the response of DRP positive advanced breast cancer patients to LiPlaCis treatment (2 doses of 75 mg each, administered on day 1 and day 8 of three week treatment cycle/s).

Figure 6 is a bar graph showing the duration of LiPlaCis treatment in the DRP positive advanced breast cancer patients, whose response to the treatment has been depicted in the aforementioned Figure 5.

Figure 7 is a schematic showing protocols for a phase I/II clinical trial of LiPlaCis.

## DETAILED DESCRIPTION OF THE INVENTION

We have discovered that a liposomal formulation of cisplatin, e.g., LiPlaCis, exhibits an improved therapeutic efficacy and an improved safety and tolerability profile compared to conventional cisplatin, in particular in subjects with cancer (e.g., advanced or refractory tumors, such as breast cancer). Subjects administered the liposomal composition containing cisplatin (e.g., in an amount of about 75-90 mg) on day 1 and day 8 of a three week treatment cycle. We observed a 5-28-fold increase in DNA platinum adducts (GG-Pt) in tumor tissue compared to normal tissue of the same patient. Administration of conventional cisplatin produces only a 4-6-fold level of DNA-platinum (GG-Pt). Our results show that LiPlaCis effectively targets and delivers cisplatin to tumor tissue.

In addition, the efficacy of treatment can be improved when the cancer subject is assessed prior to treatment using our drug response predictor (DRP) (e.g., assessing the level of one or more of the biomarkers of sensitivity of Tables 2 and 4 and/or one or more of the biomarkers of resistance of Tables 3 and 5. The DRP is an assay that, based on samples from a tumor, can predict the likelihood that a tumor will respond to a specific drug (e.g., cisplatin). The DRP method builds on a comparison of sensitive and resistant cell lines, including genomic information from the NCI (USA) NCI60 cell lines, clinical tumor biology, and clinical correlates in a systems biology network. The DRP can be performed using mRNA measurements. Biomarker signatures of the DRP can be matched to the corresponding genes in a universal microarray (which contains all genes) in order to make prediction for a specific drug (e.g., cisplatin) for a specific patient.

**$sPLA_2$ Hydrolysable, Cisplatin-containing Liposome (e.g., LiPlaCis)**

$sPLA_2$ hydrolysable, cisplatin-containing liposome (e.g., LiPlaCis) used herein, is a liposomal formulation of cisplatin, designed to be specifically degraded by secretory phospholipase A2 ($sPLA_2$) which is over-expressed by tumor tissue. sPLA2 has been shown to be present in elevated levels in a number of different tumor tissues (e.g., prostate, lung, ovarian, and breast cancer). Thus, LiPlaCis is intended to improve the therapeutic index due to an improved therapeutic efficacy and possibly also an improved safety and tolerability profile.

LiPlaCis can be prepared by spray-drying a mixture of phospholipids:
70/25/5 mol % DSPC/DSPG/DSPE-PEG2000

The lipids are then dissolved in methanol and chloroform. The lipid intermediate is hydrated in an aqueous solution of cisplatin with agitation. At this step the liposomes are formed but they have a broad size distribution and have a mixture of single-layer and multiple-layer liposomes. In order to get a product with a narrow size distribution and mono-layer liposomes, the hydration mixture can be extruded by passing it through poly-carbonate filters of appropriate pore sizes. To remove un-encapsulated cisplatin, the mixture can be purified by a number of techniques available, for example by dialysis, gel-filtration, and ultra-filtration. For preparations ranging from a few liters and above, ultra-filtration is a preferred method. Preparations intended for parenteral administration may be sterilized, for example by sterile-filtration. Methods for formulating LiPlaCis have been described in detail in, e.g., U.S. Patent Application Publication No. 2012/0177726 and de Jonge et al. (Eur J Cancer. 46(16):3016-21, 2010), each of which is hereby incorporated by reference.

**Methods of Treating Cancer Using an $sPLA_2$ Hydrolysable, Cisplatin-Containing Liposome (e.g., LiPlaCis)**

Featured herein are methods of treating cancer using a liposomal formulation of cisplatin (e.g., LiPlaCis) administered on day 1 and day 8 of three week treatment cycle/s.

*Administration of $sPLA_2$ hydrolysable, cisplatin-containing liposome*

A cancer patient can be treated with a composition containing $sPLA_2$ hydrolysable, cisplatin-containing liposomes (e.g., LiPlaCis) according to the methods described herein. The $sPLA_2$ hydrolysable, cisplatin-containing liposome composition may be administered to the patient, for example, parenterally, enterally, or topically. Enteral routes of administration of the liposomal formulation of cisplatin include oral, buccal, sublabial, sublingual, or by inhalation. Parenteral routes of administration of the liposomal formulation of cisplatin include intravenous, transdermal, intradermal, intramuscular, intraarterial, intracranial, subcutaneous, intraorbital, intraventricular, intraspinal, intraperitoneal, or intranasal. The preferred route for administration of the $sPLA_2$ hydrolysable, cisplatin-containing liposome (e.g., LiPlaCis) may be intravenous, such as intravenous infusion. The $sPLA_2$ hydrolysable, cisplatin-containing liposome composition may be administered as an intravenous infusion over a period of about 2-3 hours (e.g., 0.1-0.5, 0.5-1, 1-1.5, 1.5-2, 2-2.5, 2.5-3, 3-3.5, 3.5-4, 4-4.5, 4.5-5, 5-5.5, or 5.5-6 hours). The $sPLA_2$ hydrolysable, cisplatin-containing liposome composition can be administered as an intravenous infusion over about 2 hours (e.g., 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, or 2.9 hours), or over about 3 hours (e.g., 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5 hours). Particularly, the $sPLA_2$ hydrolysable, cisplatin-containing liposome composition (e.g., LiPlaCis) can be administered as an intravenous infusion over about 2 hours.

The $sPLA_2$ hydrolysable, cisplatin-containing liposome composition (e.g., LiPlaCis) can be administered in one or more doses (e.g., one, two, three, four, five, six, seven , eight, nine, ten, or more doses), each dose containing about 40-225 mg of cisplatin (e.g., 40-45, 45-50, 50-55, 55-60, 60-65, 65-70, 70-75, 75-80, 80-85, 85-90, 90-95, 95-100, 100-105, 105-110, 110-115, 115-120, 120-125, 125-130, 130-135, 135-140, 140-145, 145-150, 150-155, 155-160, 160-165, 165-170, 170-175, 175-180, 180-185, 185-190, 190-195, 195-200, 200-205, 205-210, 210-2115, 215-220, or 220-225 mg cisplatin). The $sPLA_2$ hydrolysable, cisplatin-containing liposome composition (e.g., LiPlaCis) can be administered as two doses, each dose containing an amount of about 75 mg of cisplatin (e.g., 60, 61, 62, 63, 64, 65, 66, 67, 68, 69, 70, 71, 72, 73, 74, 75, 76, 77, 78, 79, or 80 mg of cisplatin), or about 90 mg cisplatin (e.g., 81, 82, 83, 84, 85, 86, 87, 88, 89, 90, 91, 92, 93, 94, 95, 96, 97, 98, 99, or 100 mg cisplatin). Particularly, the $sPLA_2$ hydrolysable, cisplatin-containing liposome composition (e.g., LiPlaCis) can be administered in two doses, each dose containing an amount of about 75 mg cisplatin. Alternatively, the $sPLA_2$ hydrolysable, cisplatin-containing liposome (e.g., LiPlaCis) can be administered in two doses, each dose containing an amount of about 90 mg cisplatin. The two doses of the liposome composition are preferably administered on days 1 and 8 of a three week treatment cycle. The doses can also be administered according to a different schedule, if desired (e.g., a first dose on day 1 and a second dose on any one of days 5-21 of a three week treatment cycle).

As an alternative, the $sPLA_2$ hydrolysable, cisplatin-containing liposome composition (e.g., LiPlaCis) can be administered in two doses, the first dose containing an amount of about 75 mg cisplatin, and the second dose containing an amount of about 90 mg cisplatin. The $sPLA_2$ hydrolysable, cisplatin-containing liposome composition (e.g., LiPlaCis) can also be administered in two doses, the first dose containing an amount of about 90 mg cisplatin, and the second dose containing an amount of about 75 mg cisplatin. Alternatively, the $sPLA_2$ hydrolysable, cisplatin-containing liposome composition can be administered as one or more doses (e.g., one, two, three, four, five, six, seven , eight, nine, ten, or more doses), each dose containing an amount of cisplatin of 20-125 mg/$m^2$ body surface area of the subject (e.g., 20-25, 25-30, 30-35, 35-40, 40-45, 45-50, 50-55, 55-60, 60-65, 65-70, 70-75, 75-80, 80-85, 85-90, 90-95, 95-100, 100-105, 105-110, 110-115, or 115-120 mg/$m^2$ body surface area). For example, the $sPLA_2$ hydrolysable, cisplatin-containing liposome composition can be administered in one or more doses, each dose containing an amount of cisplatin of 40-55 mg/$m^2$ body surface area of the subject (e.g., 40, 41, 42, 43, 44, 45, 46, 47, 48, 49, 50, 51, 52, 53, 54, or 55 mg/$m^2$ body surface area). Particularly, the $sPLA_2$ hydrolysable, cisplatin-containing liposome composition (e.g., LiPlaCis) can be administered in two doses, each dose containing an amount of cisplatin of about 40 mg/$m^2$ body surface area of the subject. Alternatively, the $sPLA_2$ hydrolysable, cisplatin-containing liposome composition (e.g., LiPlaCis) can be administered in two doses, each dose containing an amount of cisplatin of about 55 mg/$m^2$ body surface area of the subject. The two doses of the liposome composition are preferably administered on days 1 and 8 of a three week treatment cycle. The doses can also be administered according to a different schedule, if desired (e.g., a first dose on day 1 and a second dose on any one of days 5-21 of a three week treatment cycle).

The $sPLA_2$ hydrolysable, cisplatin-containing liposome composition (e.g., LiPlaCis) may be administered at a frequency of, e.g., at least once hourly, once daily, twice daily, once weekly, once every two weeks, once every three weeks, once every four weeks, once monthly, once every two months, once every three months, once every six months, or once every year. For example, the $sPLA_2$ hydrolysable, cisplatin-containing liposome (e.g., LiPlaCis) can be administered as one or more doses once every three weeks. Particularly, the $sPLA_2$ hydrolysable, cisplatin-containing liposome (e.g., LiPlaCis) can be administered as two doses once every three weeks. In particular, the $sPLA_2$ hydrolysable, cisplatin-containing liposome (e.g., LiPlaCis) can be administered as two doses (e.g., first dose and second dose) on day 1 and day 8 of a three week cycle.

The $sPLA_2$ hydrolysable, cisplatin-containing liposome is administered at one or more doses such that about 80-450 mg of cisplatin (e.g., 80-90, 90-100, 100-110, 110-120, 120-130, 130-140, 140-150, 150-160, 160-170, 170-180, 180-190, 190-200, 200-210, 210-220, 220-230, 230-240, 240-250, 250-260, 260-270, 270-280, 280-290, 290-300, 300-310, 310-320, 320-330, 330-340, 340-350, 350-360, 360-370, 370-380, 380-390, 390-400, 400-410, 410-420, 420-430, 430-440, or 440-450 mg of cisplatin), or cisplatin amounting to 40-250 mg/$m^2$ body surface area (e.g., 40-50, 50-60, 60-70, 70-80, 80-90, 90-100, 100-110, 110-120, 120-130, 130-140, 140-150, 150-160, 160-170, 170-180, 180-190, 190-200, 200-210, 210-220, 220-230, 230-240, or 240-250 mg/$m^2$ body surface area) is administered in each treatment cycle. In particular, the $sPLA_2$ hydrolysable, cisplatin-containing liposome (e.g., LiPlaCis) can be administered as two doses in a treatment cycle such that 150 mg of cisplatin is administered in every treatment cycle. Alternatively, the $sPLA_2$ hydrolysable, cisplatin-containing liposome (e.g., LiPlaCis) can be administered as two doses in a treatment cycle such that 180 mg of cisplatin is administered in every treatment cycle. As yet another alternative, the $sPLA_2$ hydrolysable, cisplatin-containing liposome (e.g., LiPlaCis) can be administered as two doses in a treatment cycle such that cisplatin amounting to 80 mg/$m^2$ body surface area is administered in every treatment cycle. Alternatively, the $sPLA_2$ hydrolysable, cisplatin-containing liposome (e.g., LiPlaCis) can be administered as two doses in a treatment cycle such that cisplatin amounting to 110 mg/$m^2$ body surface area is administered in every treatment cycle.

The $sPLA_2$ hydrolysable, cisplatin-containing liposome composition (e.g., LiPlaCis) may be administered according to a treatment regimen of, e.g., 75 mg, 90 mg, 45 mg/$m^2$, or 55 mg/$m^2$ per dose on day 1 and day 8 (1 cycle) for up to 3 cycles or more. The treatment regimen may be repeated one to five times, one to ten times, one to fifteen times, one to twenty times, or more (e.g., 1, 2, 3, 4, 5, 6, 7, 8, 9, 10, 11, 12, 13, 14, 15, 16, 17, 18, 19, 20, or more cycles). The administration of the $sPLA_2$ hydrolysable, cisplatin-containing liposome composition can be repeated at such a frequency for a selected period of time, followed by a period without treatment. Such repeated administrations can occur over a course of therapy lasting a specified length of time (e.g., at least 1 week, 2 weeks, 3 weeks, 1 month, 2 months, 3 months, 6 months, 8 months, 10 months, 12 months, 18 months, 24 months, 36 months, 48 months, or 60 months). Alternatively, the administration of the $sPLA_2$ hydrolysable, cisplatin-containing liposome composition (e.g., LiPlaCis) can be repeated at such a frequency (e.g., a three week treatment cycle) in consecutive treatment cycles, with no time interval (e.g., no non-treatment interval) in between.

The $sPLA_2$ hydrolysable, cisplatin-containing liposome composition (e.g., LiPlaCis) may be administered at a treatment regimen that involves escalation of the dose in subsequent treatment cycles. For example, a liposomal cisplatin formulation (e.g., LiPlaCis) may be administered as 2 doses, each of about 75 mg of cisplatin (e.g., 60, 61, 62, 63, 64, 65, 66, 67, 68, 69, 70, 71, 72, 73, 74, 75, 76, 77, 78, 79, or 80 mg of cisplatin) on day 1 and day 8 of the first three week treatment cycle, followed by two doses, each of about 90 mg of cisplatin (e.g., 81, 82, 83, 84, 85, 86, 87, 88, 89, 90, 91, 92, 93, 94, 95, 96, 97, 98, 99, or 100 mg of cisplatin) on day 1 and day 8 of the next treatment cycle. Alternatively, liposomal cisplatin formulation (e.g., LiPlaCis) may be administered as 2 doses, each comprising cisplatin amounting to about 40 $mg/mm^2$ body surface area on day 1 and day 8 of the first three week treatment cycle, followed by two doses, each comprising cisplatin amounting to about 55 $mg/mm^2$ body surface area on day 1 and day 8 of the next treatment cycle.

The $sPLA_2$ hydrolysable, cisplatin-containing liposome composition (e.g., LiPlaCis) can be administered in a pharmaceutical composition that includes one or more pharmaceutically acceptable carriers, excipients, or diluents. Examples of suitable carriers, excipients, or diluents of the liposomal composition (e.g., LiPlaCis) include, e.g., saline, sterile water, polyalkylene glycols, oils of vegetable origin, hydrogenated napthalenes, suitable buffer, 1,3-butanediol, Ringer's solution and/or sodium chloride solution. Exemplary formulations for parenteral administration can include solutions prepared in water suitably mixed with a surfactant, e.g., hydroxypropylcellulose. Dispersions can also be prepared in glycerol, liquid polyethylene glycols, DMSO and mixtures thereof with or without alcohol, and in oils. Under ordinary conditions of storage and use, these preparations may contain a preservative to prevent the growth of microorganisms. Other exemplary carriers, excipients, or diluents are described in the Handbook of Pharmaceutical Excipients, 6th Edition, Rowe et al., Eds., Pharmaceutical Press (2009), hereby incorporated by reference in its entirety.

In some embodiments, administration of the $sPLA_2$ hydrolysable, cisplatin-containing liposome composition (e.g., LiPlaCis) can be accompanied by a hydration program as a prophylaxis against infusion reactions and as an anti-emetic regimen. An exemplary treatment scheme is outlined in Table 1.

**Table 1.** $sPLA_2$ hydrolysable, cisplatin-containing liposome (e.g., LiPlaCis) treatment, infusion reaction prophylaxis, hydration schema, emesis prophylaxis.

| **Time definition** | Day = 1 at Time = 0 hour is start of LiPlaCis infusion |
|---|---|
| **Prophylaxis against infusion reaction:** | |
| Prednisolone 50 mg BID PO | Day = -1 |
| Solumedrol 40 mg IV | Day = 1 at Time=- 2 hour |
| Clemastine 2 mg IV | Day = 1 at Time = -2 hour |
| Paracetamol 1g PO<br>Ibuprofen 400 mg PO | Day = 1 at Time = -1 hour |
| **Pre-hydration:** | |
| NaCl 0.9% 1½ L over 2 hours*<br>Mg++ 6 mmol over 2 hours | Day = 1 at Time = -2 hour to Time = 0 hour |
| *NaCl 0.9% 1 L over 1 hour (depending on diuresis) | Day = 1 |
| **LiPlaCis:** | |
| LiPlaCis 75 mg in 2 x 500 ml NaCl (0.9%) by 2h infusion | Day = 1 at Time = 0 to Time = +2 |
| **Post-hydration:** | |
| NaCl 0.9% 2½ L over 12 hours IV or equivalent PO. | Day = 1 at Time = +2 to Time = +14 |
| **Emesis prophylaxis (A1):** | |
| Palonosetron 250 μg iv | Day = 1 at Time = -1 |
| (Solumedrol 40 mg iv)<br>Prednisolone 25 mg BID PO days 2-3<br>Prednisolone 25 mg OD days 4-5 | (Day = 1 at Time = -2) Also listed in prophylaxis section<br>Day 2 and day 3<br>Day 4 and day 5 |
| Aprepitant p.n. 125 mg PO + 80mg PO days 1+2 | Day 1 and day 2 |

*Preparation of $sPLA_2$ hydrolysable, cisplatin-containing liposome composition for administration*

The $sPLA_2$ hydrolysable, cisplatin-containing liposome composition (e.g., LiPlaCis) can be supplied as a concentrate for infusion (e.g., LiPlaCis Concentrate for Infusion), which can be aseptically diluted in sterile 0.9% NaCl (aq) in an infusion bag before administration. For example, the infusion bag (e.g., LiPlaCis infusion bag system) can be Fresenius FREEFLEX® Sodium Chloride 0.9%, 500 ml. Two such infusion bags can be used for each dose, each containing 50% of the dose.

The liposomal concentrate for infusion (e.g., LiPlaCis Concentrate for Infusion) can be supplied as a white to off-white opalescent dispersion in 30 ml vials, each containing 20 ml. The product can be stored at -80°C and the concentration (in mg/ml) can be marked on the label. The volume of liposomal concentrate for infusion (e.g., LiPlaCis Concentrate for Infusion) that is to be diluted in order to prepare the final liquid for infusion may vary from patient to patient depending on the desired dose.

The liposomal concentrate for infusion (e.g., LiPlaCis Concentrate for Infusion) may be diluted by the following procedure:

(i) For each dose, the total volume ($V_{tot}$) of the liposomal concentrate for infusion (e.g., LiPlaCis Concentrate for Infusion) to be used can be calculated according to the following formula:

$$V_{tot} = D/C$$

The volume ($V_{bag}$) to be added to each of the two infusion bags can be calculated according to the following formula:

$$V_{bag} = V_{tot}/2$$

Where, $V_{tot}$ is the volume of the liposomal concentrate for infusion (e.g., LiPlaCis Concentrate for Infusion) to be used, in ml; $V_{bag}$ is the volume of liposomal concentrate for infusion (e.g., LiPlaCis Concentrate for Infusion) to be added to each of the two infusions bags, in ml; D is the dose, in mg; and C is the concentration of cisplatin in the liposomal concentrate for infusion (e.g., LiPlaCis Concentrate for Infusion), in mg/ml, stated on the label.

(ii) An appropriate amount of the liposomal concentrate for infusion (e.g., LiPlaCis Concentrate for Infusion) (according to the calculation above) can be thawed prior to use. The thawing can be done in a water bath at 10-25°C. Once thawed, the liposomal concentrate for infusion (e.g., LiPlaCis Concentrate for Infusion) ishould not be refrozen.

(iii) The calculated total volume $V_{tot}$ is withdrawn, and the volume $V_{bag}$ is added to each of the two infusion bags via a medication valve.

(iv) The infusion liquid should be mixed thoroughly, kept protected from light, and used within about 8 hours.

*Cancer patients that can be treated with the $sPLA_2$ hydrolysable, cisplatin-containing liposome composition (e.g., LiPlaCis)*

A patient who can be treated with the dosage regimen of $sPLA_2$ hydrolysable, cisplatin-containing liposome (e.g., LiPlaCis) according to the methods described herein, may include, e.g., a patient that has been diagnosed with cancer, a patient that has not received a cancer treatment (e.g., the liposomal formulation of cisplatin, an anti-cancer agent other than the liposomal formulation of cisplatin, or radiation), a patient that has received a cancer treatment (e.g., an anti-cancer agent other than the liposomal formulation of cisplatin or radiation), or a patient during treatment with the liposomal formulation of cisplatin.

For example, the patient may have a solid tumor or a hematological cancer, such as a cancer type selected from prostate cancer, ovarian cancer (e.g., ovarian adenocarcinoma or embryonal carcinoma), liver cancer (e.g., hepatocellular carcinoma (HCC) or hepatoma), myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. In particular, the cancer of the patient is, e.g., prostate cancer, ovarian cancer, hepatocellular carcinoma (HCC), multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, cervical cancer, renal cell carcinoma (RCC), esophageal cancer, melanoma, glioma, pancreatic cancer, gastrointestinal stromal tumors (GIST), sarcoma, estrogen receptor-positive (ERpos) breast cancer, non-small cell lung carcinoma (NSCLC), colon cancer, bladder cancer, or squamous cell carcinoma of the head and neck (SCCHN).

The patient may have a cancer (e.g., breast cancer) that is resistant to one or more cancer therapies other than the liposomal formulation of cisplatin (e.g., docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfiizomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, conventional cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, and rituximab), surgery, or radiation. The patient may also have experienced a recurrence following surgery, radiation, or treatment with a cancer therapy other than the liposomal formulation of cisplatin.

## Methods of Predicting Responsiveness of Patients Prior to Treatment

Also featured herein are methods of determining responsiveness of a patient to the liposomal formulation of cisplatin (e.g., LiPlaCis), e.g., prior to treatment with the same. For example, a patient can be identified as responsive to the liposomal formulation of cisplatin by determining the expression level of one or more biomarkers (e.g., one or more of the biomarkers shown in Tables 2-5, such as COL5A2 (SEQ ID NO: 73 OR 211) in a biological sample (e.g., a tumor sample) obtained from the patient, and subsequently administered the liposomal formulation of cisplatin (e.g., LiPlaCis). Alternatively, a patient can be identified as less likely to be responsive to the liposomal formulation of cisplatin by determining the expression level of one or more biomarkers (e.g., one or more of the biomarkers shown in Tables 2-5, such as COL5A2 (SEQ ID NO: 73 OR 211) in a biological sample obtained from the patient. If the patient exhibits expression levels of one or more biomarkers indicative of non-responsiveness to the liposomal formulation of cisplatin, the patient may be treated with or offered a treatment with an agent other than the liposomal formulation of cisplatin. In particular, the patient may be treated with, e.g., radiation and/or administration of a therapeutic agent, such as docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, or rituximab.

Expression levels of the biomarkers shown in Tables 2-5 may be detected in a subject/patient having cancer and are useful for predicting the responsiveness of the patient to $sPLA_2$ hydrolysable, cisplatin-containing liposome (e.g., LiPlaCis). These patients may already be determined to be resistant to a therapy other than the $sPLA_2$ hydrolysable, cisplatin-containing liposome, such as docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, conventional cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, or rituximab.

A device, such as a microarray, with one or more single-stranded oligonucleotide probes that have substantial identity (e.g., at least 85%, 90%, 95%, 99%, or 100% sequence identity) to a sequence that is complementary or identical to the nucleic acid sequence of one or more biomarkers shown in Tables 2-5 can be used according to the methods described herein to assess the responsiveness of a cancer patient to treatment with the $sPLA_2$ hydrolysable, cisplatin-containing liposome (e.g., LiPlaCis). For example, the probes can be used to detect one or more (e.g., two, three, four, five, ten, twenty, or all) of the biomarkers of sensitivity listed in Tables 2 and 4, such as COL5A2 (SEQ ID NO 73 or 211), in a sample (e.g., a tumor sample) from a patient having cancer (e.g., breast cancer). Additionally, the probes can be used to detect one or more (e.g., two, three, four, five, ten, twenty, or all) of the biomarkers of resistance listed in Tables 3 and 5, such as SFN (SEQ ID NO: 96 or 324), in a sample (e.g., a tumor sample) from a patient having cancer (e.g., breast cancer).

Individual biomarkers (e.g., COL5A2 (SEQ ID NO 73 or 211) or SFN (SEQ ID NO: 96 or 324)) and sets of biomarkers shown in Tables 2-5 that can be used to determine the responsiveness of a cancer patient to $sPLA_2$ hydrolysable, cisplatin-containing liposome (e.g., LiPlaCis) at various stages of disease progression (e.g., patients diagnosed with cancer or patients after cancer recurrence) and at different times during the treatment process (e.g., prior to administration of any cancer treatment, after administration of one or more cancer treatments other than the $sPLA_2$ hydrolysable, cisplatin-containing liposome, prior to administration of the $sPLA_2$ hydrolysable, cisplatin-containing liposome, or during administration of the $sPLA_2$ hydrolysable, cisplatin-containing liposome). Additionally, the methods can be used to determine the responsiveness to $sPLA_2$ hydrolysable, cisplatin-containing liposome (e.g., LiPlaCis) in a patient with cancer that is resistant to one or more cancer therapies other than LiPlaCis, such as docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, conventional (e.g., free) cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, or rituximab.

In particular, featured are methods for determining whether a patient may be responsive to $sPLA_2$ hydrolysable, cisplatin-containing liposome composition (e.g., LiPlaCis) by, e.g., detecting the expression level (e.g., mRNA or protein produced therefrom) of one or more of the biomarkers shown in Tables 2-5 (e.g., COL5A2 (SEQ ID NO 73 or 211)) in a biological sample (e.g., a tumor biopsy) obtained from the subject using a device (e.g., a microarray or a protein array). The expression level of one or more of the biomarkers of sensitivity may then be compared to the expression level of the biomarkers in a cell or tissue known to be sensitive or resistant to the $sPLA_2$ hydrolysable, cisplatin-containing liposome (e.g., LiPlaCis) to determine the patient's responsiveness to the $sPLA_2$ hydrolysable, cisplatin-containing liposome. The patient may be responsive to the $sPLA_2$ hydrolysable, cisplatin-containing liposome if the expression level of the one or more of the biomarkers of sensitivity (e.g., one or more of COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15)) is substantially similar to the expression level of the biomarkers of sensitivity in a cell or tissue known to be sensitive to $sPLA_2$ hydrolysable, cisplatin-containing liposome (e.g., from a patient sensitive to LiPlaCis). The patient may also be responsive to $sPLA_2$ hydrolysable, cisplatin-containing liposome if the level of expression of one or more of the biomarkers of resistance (e.g., one or more of SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112)) is substantially dissimilar to the expression level of the biomarkers of resistance in a cell or tissue known to be resistant to $sPLA_2$ hydrolysable, cisplatin-containing liposome (e.g., from a patient resistant to LiPlaCis).

Also featured are methods of treating a patient having cancer, such as a patient having a cancer that is resistant to one or more cancer therapies other than the $sPLA_2$ hydrolysable, cisplatin-containing liposome (e.g., LiPlaCis), by detecting the expression levels of one or more of the biomarkers shown in Tables 2-5 (e.g., COL5A2 (SEQ ID NO: 73 OR 211) in a sample (e.g., a tumor sample) from the patient, and then administering the $sPLA_2$ hydrolysable, cisplatin-containing liposome (e.g., LiPlaCis) based on the expression levels of the biomarkers. In particular, a patient having cancer may be administered $sPLA_2$ hydrolysable, cisplatin-containing liposome if the expression level of one or more biomarkers of sensitivity is substantially similar to the expression level of the biomarkers of sensitivity in a cell or tissue known to be sensitive to the same. Additionally, a patient having cancer may be administered $sPLA_2$ hydrolysable, cisplatin-containing liposome (e.g., LiPlaCis) if the expression level of one or more biomarkers of resistance is substantially dissimilar to the expression level of the biomarkers of resistance in a cell or tissue known to be resistant to the same. Thus, the methods can be used to treat cancer patients predicted to be responsive to the $sPLA_2$ hydrolysable, cisplatin-containing liposome (e.g., LiPlaCis), such as patients having, e.g., breast cancer, prostate cancer, ovarian cancer, hepatocellular carcinoma (HCC), cervical cancer, renal cell carcinoma (RCC), esophageal cancer, melanoma, glioma, pancreatic cancer, gastrointestinal stromal tumors (GIST), sarcoma, estrogen receptor-positive (ERpos) breast cancer, non-small cell lung carcinoma (NSCLC), colon cancer, bladder cancer, squamous cell carcinoma of the head and neck (SCCHN), acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), and Hodgkin's lymphoma. Alternatively, a patient having cancer may not be administered $sPLA_2$ hydrolysable, cisplatin-containing liposome (e.g. LiPlaCis) if the expression level of one or more biomarkers of sensitivity is substantially dissimilar to the expression level of the biomarkers of sensitivity in a cell or tissue known to be sensitive to the $sPLA_2$ hydrolysable, cisplatin-containing liposome. Likewise, a patient having cancer may not be administered $sPLA_2$ hydrolysable, cisplatin-containing liposome (e.g., LiPlaCis) if the expression level of one or more biomarkers of resistance is substantially similar to the expression level of the biomarkers of resistance in a cell or tissue known to be resistant to the $sPLA_2$ hydrolysable, cisplatin-containing liposome.

Methods are described herein for identifying biomarkers of drug responsiveness, detecting biomarker gene expression in cancer patients, determining the responsiveness of a cancer patient to $sPLA_2$ hydrolysable, cisplatin-containing liposome (e.g., LiPlaCis), and treating cancer patients with $sPLA_2$ hydrolysable, cisplatin-containing liposome (e.g., LiPlaCis). Also described are devices and kits for use in these methods.

*Methods for identifying biomarkers of drug responsiveness*

Featured herein are methods for identifying biomarkers (e.g., one or more of the biomarkers of Tables 2-5) for determining the responsiveness of a cancer patient to a cancer treatment, such as $sPLA_2$ hydrolysable, cisplatin-containing liposome (e.g., LiPlaCis). Such methods can involve, for example, an algorithm based on growth inhibition values (GI50) of cell lines (e.g., NCI60 cell lines) subjected to treatment with $sPLA_2$ hydrolysable, cisplatin-containing liposome (e.g., LiPlaCis), followed by measurement of gene expression (e.g., using a microarray (e.g., an Affymetrix HG-U133A or HG-U133_Plus_2 array)).

*Methodology of the in vitro cancer growth inhibition screen*

The human tumor cell lines of the cancer screening panel may be grown in RPMI 1640 medium containing 5% fetal bovine serum and 2 mM L-glutamine. Cells may be inoculated into 96 well microtiter plates in 100 $\mu$L at plating densities ranging from 5,000 to 40,000 cells/well depending on the doubling time of individual cell lines. After cell inoculation, the microtiter plates may be incubated at 37°C, 5% CO2, 95% air and 100% relative humidity for 24 hours prior to addition of experimental compounds.

After 24 hours, two plates of each cell line may be fixed in situ with TCA, to represent a measurement of the cell population for each cell line at the time of compound addition (Tz). Experimental compounds may be solubilized in dimethyl sulfoxide at 400-fold the desired final maximum test concentration and stored frozen prior to use. At the time of compound (e.g., $sPLA_2$ hydrolysable, cisplatin-containing liposome, such as LiPlaCis) addition, an aliquot of frozen concentrate may be thawed and diluted to twice the desired final maximum test concentration with complete medium containing 50 $\mu$g/ml Gentamicin. A total of four additional 10-fold or ½ log serial dilutions are made to provide a total of five concentrations plus control. Aliquots of 100 $\mu$l of these different compound dilutions are added to the appropriate microtiter wells already containing 100 $\mu$l of medium, resulting in the required final compound concentrations.

Following compound (e.g., $sPLA_2$ hydrolysable, cisplatin-containing liposome, such as LiPlaCis) addition, the plates may be incubated for an additional 48 h at 37°C, 5% CO2, 95% air, and 100% relative humidity. For adherent cells, the assay may be terminated by the addition of cold TCA. Cells may be fixed in situ by the gentle addition of 50 μl of cold 50% (w/v) TCA (final concentration, 10% TCA) and incubated for 60 minutes at 4°C. The supernatant may be discarded, and the plates may be washed five times with tap water and air-dried. Sulforhodamine B (SRB) solution (100 μl) at 0.4% (w/v) in 1% acetic acid may be added to each well, and the plates may be incubated for 10 minutes at room temperature. After staining, unbound dye may be removed by washing five times with 1% acetic acid and the plates may be air-dried. Bound stain may be subsequently solubilized with 10 mM trizma base, and the absorbance may be read on an automated plate reader at a wavelength of 515 nm. For suspension cells, the methodology may be the same, except that the assay may be terminated by fixing settled cells at the bottom of the wells by gently adding 50 μl of 80% TCA (final concentration, 16 % TCA). Using the seven absorbance measurements [time zero, (Tz), control growth, (C), and test growth in the presence of compound (e.g., $sPLA_2$ hydrolysable, cisplatin-containing liposome, such as LiPlaCis) at the five concentration levels (Ti)], the percentage growth may be calculated at each of the compound concentrations levels. Percentage growth inhibition may be calculated as:

$$[(Ti-Tz)/(C-Tz)] \times 100$$

for concentrations for which Ti>/=Tz

$$[(Ti-Tz)/Tz] \times 100$$

for concentrations for which Ti<Tz

Three dose response parameters may be calculated for each experimental agent (e.g., $sPLA_2$ hydrolysable, cisplatin-containing liposome, such as LiPlaCis). Growth inhibition of 50% (GI50) is calculated from [(Ti-Tz)/(C-Tz)] x 100 = 50, which is the agent (e.g., $sPLA_2$ hydrolysable, cisplatin-containing liposome, such as LiPlaCis) concentration resulting in a 50% reduction in the net protein increase (as measured by SRB staining) in control cells during the compound incubation. The compound concentration resulting in total growth inhibition (TGI) is calculated from Ti = Tz. The LC50 (concentration of compound resulting in a 50% reduction in the measured protein at the end of the compound treatment as compared to that at the beginning) indicating a net loss of cells following treatment is calculated from [(Ti-Tz)/Tz] x 100 = -50. Values are calculated for each of these three parameters if the level of activity is reached; however, if the effect is not reached or is exceeded, the value for that parameter is expressed as greater or less than the maximum or minimum concentration tested.

*Gene Expression and Growth Inhibition Analysis*

The gene expression measurements of NCI60 cancer cell lines can be obtained from a publically available database (e.g., the National Cancer Institute and the Massachusetts Institute of Technology). Each dataset can be normalized so that sample expression measured by different chips can be compared. The preferred method of normalization is the logit transformation, which may be performed for each gene y on each chip, as follows:

$$\text{logit}(y) = \log [(y\text{-background}) / (\text{saturation} - y)],$$

where background is calculated as the minimum intensity measured on the chip minus 0.1% of the signal intensity range: min-0.001*(max-min), and saturation is calculated as the maximum intensity measured on the chip plus 0.1% of the signal intensity range: max+0.001*(max-min). The resulting logit transformed data may then be z-transformed to mean zero and standard deviation 1.

Next, gene expression can be correlated to cancer cell growth inhibition. Growth inhibition data (GI50) of the NCI60 cell lines in the presence of a cancer treatment, such as LiPlaCis, can be obtained from the NCI. The correlation between the logit-transformed expression level of each gene in each cell line and the logarithm of GI50 (the concentration of a given compound that results in a 50% inhibition of growth) can be calculated, e.g., using the Pearson correlation coefficient or the Spearman Rank-Order correlation coefficient. Instead of using GI50s, any other measure of patient sensitivity to a given treatment (e.g., $sPLA_2$ hydrolysable, cisplatin-containing liposome, such as LiPlaCis) may be correlated to gene expression levels of the patient. Since a plurality of measurements may be available for a single gene, the most accurate determination of correlation coefficient can be, e.g., the median of the correlation coefficients calculated for all probes measuring expression of the same gene.

For example, the median correlation coefficient of gene expression measured on a probe to growth inhibition or patient sensitivity to $sPLA_2$ hydrolysable, cisplatin-containing liposome (e.g., LiPlaCis) can be calculated for all genes of interest. Genes that have a median correlation above, e.g., 0.20, 0.21 0.22. 0.23, 0.24, 0.25, 0.26, 0.27, 0.28, 0.29, 0.30, 0.31, 0.32, 0.33, 0.34, 0.35, 0.36, 0.37, 0.38, 0.39, 0.40, or higher (e.g., 0.2 or higher), can be used as biomarkers of sensitivity for assessing responsiveness of a cancer patient (e.g., a patient having cancer that is resistant to one or more cancer therapies other than $sPLA_2$ hydrolysable, cisplatin-containing liposome, such as LiPlaCis) to $sPLA_2$ hydrolysable, cisplatin-containing liposome (e.g., LiPlaCis). Likewise, genes that have a median correlation below, e.g., -0.20, -0.21, -0.22. -0.23, -0.24, -0.25, -0.26, -0.27, -0.28, -0.29, -0.30, -0.31, - 0.32, -0.33, -0.34, -0.35, -0.36, -0.37, -0.38, -0.39, -0.40, or lower (e.g., -0.2 or lower), can be used as biomarkers of resistance for assessing responsiveness of a cancer patient (e.g., a patient having cancer that is resistant to one or more cancer therapies other than $sPLA_2$ hydrolysable, cisplatin-containing liposome, such as LiPlaCis) to $sPLA_2$ hydrolysable, cisplatin-containing liposome (e.g., LiPlaCis). Preferably, the correlation coefficient of a biomarker of sensitivity will exceed 0.2, while the correlation coefficient of a biomarker of resistance will be less than -0.2. The result is a list of biomarker genes that correlate to sensitivity or resistance to $sPLA_2$ hydrolysable, cisplatin-containing liposome (e.g., LiPlaCis), as shown in Tables 2 and 4 and Tables 3 and 5, respectively.

*Cancer types*

The methods, devices, and kits of the invention can be used for diagnosing, prognosing, monitoring, treating, and/or reducing cancer in a subject suffering from, diagnosed with, or susceptible to cancer. Non-limiting examples of cancers that can be diagnosed, prognosed, monitored, treated, or reduced using the methods include hematological and solid tumors. In particular, cancers include, e.g., breast cancer, prostate cancer, ovarian cancer (e.g., ovarian adenocarcinoma or embryonal carcinoma), liver cancer (e.g., hepatocellular carcinoma (HCC) or hepatoma), myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system.

In particular, the methods are useful for diagnosing, prognosing, monitoring, treating, or preventing, e.g., breast cancer, prostate cancer, ovarian cancer, hepatocellular carcinoma (HCC), cervical cancer, renal cell carcinoma (RCC), esophageal cancer, melanoma, glioma, pancreatic cancer, gastrointestinal stromal tumors (GIST), sarcoma, estrogen receptor-positive (ERpos) breast cancer, non-small cell lung carcinoma (NSCLC), colon cancer, bladder cancer, squamous cell carcinoma of the head and neck (SCCHN), acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), and Hodgkin's lymphoma.

For example, the cancer can be prostate cancer, such as Stage I, II (e.g., IIA or IIB), III, or IV prostate cancer. In particular, the cancer may be prostate cancer that is resistant to one or more cancer therapies, such as docetaxel, cabazitaxel, mitoxantrone, estramustine, prednisone, and/or surgery. Alternatively, the cancer is an ovarian cancer. The ovarian cancer can be, for example, a Stage I (e.g., Stage IA, IB, or IC), Stage II (e.g., Stage IIA or IIB), Stage III (e.g., Stage IIIA1, IIIA2, IIIB, or IIIC), or Stage IV (e.g., Stage IVA or IVB) ovarian cancer. In particular, the cancer can be ovarian cancer that is resistant to one or more cancer therapies, such as docetaxel, carboplatin, bevacizumab, paclitaxel, gemcitabine, doxorubicin, topotecan, etoposide, tamoxifen, and/or letrozole. Additionally, the cancer can be HCC, such as Stage I, Stage II, Stage III (e.g., Stage IIIA, IIIB, or IIIC), or Stage IV (e.g., Stage IVA or IVB) HCC. In particular, the cancer can be HCC that is resistant to one or more cancer therapies, such as sorafenib, doxorubicin, cisplatin, gemcitabine, capecitabine, oxaliplatin, interferon-alpha, and/or 5-fluorouracil (5-FU).

*Methods for detecting biomarker gene expression in cancer patients*

A cancer patient can be assessed for sensitivity or resistance to $sPLA_2$ hydrolysable, cisplatin-containing liposome (e.g., LiPlaCis) by detecting gene expression of a biomarker (e.g., one or more of the biomarkers of Tables 2-5) in a biological sample obtained from the cancer patient (e.g., a patient having cancer that is resistant to one or more cancer therapies other than the $sPLA_2$ hydrolysable, cisplatin-containing liposome such as LiPlaCis). The biological sample can include, for example, cells, tissue (e.g., a tissue sample obtained by biopsy), blood, serum, plasma, urine, sputum, cerebrospinal fluid, lymph tissue or fluid, or pancreatic fluid. For example, the biological sample can be fresh frozen or formalin-fixed paraffin embedded (FFPE) tissue obtained from the subject, such as a tumor sample (e.g., a biopsy) from the tissue of interest (e.g., prostate, ovarian, lung, lymph nodes, thymus, spleen, bone marrow, breast, colorectal, pancreatic, cervical, bladder, gastrointestinal, head, or neck tissue).

*RNA extraction and biomarker expression measurement*

Cell samples or tissue samples may be snap frozen in liquid nitrogen until processing. RNA may be extracted using, e.g., Trizol Reagent from Invitrogen following manufacturer's instructions, and detected directly or converted to cDNA for detection. RNA may be amplified using, e.g., MessageAmp kit from Ambion following manufacturer's instructions. Amplified RNA may be quantified using, e.g., HG-U133A or HG-U133_Plus2 GeneChip from Affymetrix Inc. or a compatible apparatus, e.g., the GCS3000Dx GENECHIP® System from Affymetrix Inc., using the manufacturer's instructions. The resulting biomarker expression measurements may be further analyzed as described herein. The procedures described can be implemented using, e.g., R software available from R-Project and supplemented with packages available from Bioconductor.

One or more of the biomarkers shown in Tables 2-5 (e.g., COL5A2 (SEQ ID NO: 73 OR 211) may be measured in a biological sample (e.g., a tumor sample) obtained from the cancer patient (e.g., a patient with any of the cancer types described herein, such as a patient having cancer that is resistant to one or more cancer therapies other than $sPLA_2$ hydrolysable, cisplatin-containing liposome, such as LiPlaCis) using, e.g., polymerase chain reaction (PCR), reverse transcriptase PCR (RT-PCR), quantitative real-time PCR (qRT-PCR), an array (e.g., a microarray), a genechip, pyrosequencing, nanopore sequencing, sequencing by synthesis, sequencing by expansion, single molecule real time technology, sequencing by ligation, microfluidics, infrared fluorescence, next generation sequencing (e.g., RNA-Seq techniques), Northern blots, Western blots, Southern blots, NanoString nCounter technologies (e.g., those described in U.S. Patent Application Nos. US 2011/0201515, US 2011/0229888, and US 2013/0017971, each of which is incorporated by reference in its entirety), proteomic techniques (e.g., mass spectrometry or protein arrays), and combinations thereof.

*Devices*

Devices of the invention can be used for detecting the level of expression of one or more biomarkers shown in Tables 2-5. The device may include at least one single-stranded nucleic acid (e.g., a probe) having at least 85% sequence identity (e.g., 85%, 90%, 95%, 97%, 98%, 99%, or 100% sequence identity) to a nucleic acid sequence that is complementary or identical to at least 5 (e.g., at least 10, at least 15, at least 20, or more) consecutive nucleotides of one or more biomarkers shown in Tables 2-5 (e.g., COL5A2 (SEQ ID NO 73 or 211) or SFN (SEQ ID NO: 96 or 324)), in which the at least one single-stranded nucleic acid is sufficient for the detection of the expression level of the one or more biomarkers. The device may be used to detect the expression level of a given biomarker by specific hybridization between the single-stranded nucleic acid and the biomarker (e.g., an mRNA, genomic DNA, or non-coding RNA), a nucleic acid encoding the biomarker (e.g., an mRNA), or a complementary nucleic acid thereof. The device may be or include a microarray. The device may also include or be used with reagents and materials for next generation sequence (e.g., sequencing by synthesis). The device may also include or be used with NanoString reagents and at least one nCounter cartridge. The device may be or include a protein array, which contains one or more protein binding moieties (e.g., proteins, antibodies, nucleic acids, aptamers, affibodies, lipids, phospholipids, small molecules, labeled variants of any of the above, and any other moieties useful for protein detection as well known in the art) capable of detectably binding to the polypeptide product(s) of one or more biomarkers shown in Tables 2-5. The device may also be a cartridge for measuring an amplification product resulting from hybridization between one or more nucleic acid molecules from the patient and at least one single-stranded nucleic acid single-stranded nucleic acid molecules of the device, such as a device for performing qRT-PCR.

*Microarrays*

The expression levels of the biomarkers (e.g., the biomarkers listed in Tables 2-5 (e.g., COL5A2 (SEQ ID NO: 73 OR 211) may be determined using high-throughput expression profiling platforms, such as microarrays. In particular, a microarray for use in the methods for assessing the responsiveness of a cancer patient (e.g., a patient having cancer that is resistant to one or more cancer therapies other than $sPLA_2$ hydrolysable, cisplatin-containing liposome, such as LiPlaCis) to $sPLA_2$ hydrolysable, cisplatin-containing liposome (e.g., LiPlaCis) contains or is produced by generating oligonucleotide probes (e.g., DNA, cDNA, or RNA probes) capable of hybridizing to one or more biomarkers of interest (e.g., one or more of the biomarkers of Tables 2-5) or the complement sequences thereof. Each probe can have, e.g., at least 10, 15, 20, 25, 30, or more contiguous nucleic acid residues (e.g., at least 15) that are complementary or identical to a nucleic acid sequence of a selected biomarker. The probe nucleic sequence can also have at least 85% (e.g., 90%, 95%, 99%, or 100%) sequence identity to the nucleic acid sequence of the gene coding the biomarker (e.g., COL5A2 (SEQ ID NO 73 or 211)) or the complement sequence thereof. In particular, the probe sequences can be complementary to all or a portion of the nucleic acid sequence of the biomarker(s).

For example, microarrays of the invention for determining responsiveness to $sPLA_2$ hydrolysable, cisplatin-containing liposome (e.g., LiPlaCis) can include probes for one or more (e.g., at least 5, 10, 15, or 20 or more (e.g., all)) biomarkers of sensitivity shown in Tables 2 and 4, such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15)

Microarrays for determining responsiveness to $sPLA_2$ hydrolysable, cisplatin-containing liposome (e.g., LiPlaCis) can also include probes for one or more (e.g., at least 5, 10, 15, or 20 or more (e.g., all)) biomarkers of resistance listed in Tables 3 and 5, such as SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112).

Microarrays for determining responsiveness to $sPLA_2$ hydrolysable, cisplatin-containing liposome (e.g., LiPlaCis) can also include probes for one or more (e.g., at least 5, 10, 15, or 20 or more (e.g., all)) biomarkers of sensitivity and biomarkers of resistance shown in Tables 2-5, such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112)..

A microarray probe may be single-stranded or double-stranded. The probe may be labeled (e.g., detectably labeled with a fluorescent molecule, dye molecule, small molecule, epitope tag, barcode sequence, polypeptide, or any other detectable molecule). Probes can be detectably labeled and immobilized on a solid support to form the microarray. For example, probes can be either prefabricated and spotted to the surface or directly synthesized on to the surface (*in situ*) of the microarray. The microarray can also be configured such that the sequence and position of each member (e.g., probe) of the array is known. For example, a selection of biomarkers whose expression correlates with an increased likelihood of responsiveness to $sPLA_2$ hydrolysable, cisplatin-containing liposome (e.g., LiPlaCis) can be arrayed on a solid support. Hybridization of a labeled probe with a particular target nucleic acid (e.g., an mRNA corresponding to one or more biomarkers of Tables 2-5) indicates that the sample from which the mRNA was derived expresses that biomarker (e.g., the biomarker of sensitivity or resistance to $sPLA_2$ hydrolysable, cisplatin-containing liposome, such as LiPlaCis).

*PCR-based techniques*

As few as one to thirty (e.g., 5 to 30 or 10 to 30, or at least the first 15 of the biomarkers listed in Tables 2-5) biomarkers may be used to determine patient responsiveness to $sPLA_2$ hydrolysable, cisplatin-containing liposome (e.g., LiPlaCis) using the methods described herein. Tissue or cell samples from a cancer patient (e.g., a patient having cancer that is resistant to one or more cancer therapies other than $sPLA_2$ hydrolysable, cisplatin-containing liposome (e.g., LiPlaCis) can be conveniently assayed for gene expression levels using polymerase chain reaction (PCR) analysis, such as quantitative real-time PCR (qPCR), or quantitative loop-mediated isothermal amplification (q-LAMP). For example, an mRNA corresponding to a biomarker of Tables 2-5 can be detected in a biological sample by (a) producing cDNA from the sample by reverse transcription using at least one primer; (b) amplifying the cDNA so produced using a target polynucleotide as sense and antisense primers to amplify target cDNAs therein; and (c) detecting the presence of the amplified target cDNA using polynucleotide probes. The primers and probes including the target sequences shown in Tables 2-5, such as COL5A2 (SEQ ID NO 73 or 211) and/or SFN (SEQ ID NO: 96 or 324), may be used to detect expression of one or more of the indicated biomarkers using PCR. The methods can include one or more steps that allow determination of the levels of target mRNA in a biological sample (e.g., by simultaneously examining the levels of a comparative control mRNA sequence or "housekeeping" gene, such as an actin family member or GAPDH). The primers for these PCR-based assays may be labeled for detection according to methods known in the art.

*Sequencing*

The expression levels of the biomarkers shown in Tables 2-5, such as COL5A2 (SEQ ID NO 73 or 211) and/or SFN (SEQ ID NO: 96 or 324), may be determined using sequencing technologies, such as next generation sequencing platforms (e.g., RNA-Seq), as described in Mortazavi et al., Nat. Methods 5: 621-628, 2008, hereby incorporated by reference. RNA-Seq is a robust technology for monitoring expression by direct sequencing of the RNA molecules in a sample. This methodology may include fragmentation of RNA to an average length of, e.g., 200 nucleotides, conversion to cDNA by random priming, and synthesis of double-stranded cDNA (e.g., using the PROTOSCRIPT® First Strand cDNA Synthesis Kit from New England Biosciences). The cDNA may then be converted into a molecular library for sequencing by addition of sequence adapters for each library (e.g., from ILLUMINA®/Solexa), and the resulting 50 to 100 nucleotide reads are mapped onto the genome. Exemplary sequencing platforms suitable for use according to the methods include, e.g., pyrosequencing, ILLUMINA® sequencing by synthesis, SOLID® sequencing, ION TORRENT® sequencing, and SMRT® sequencing.

*Methods of determining the responsiveness of a patient to $sPLA_2$ hydrolysable, cisplatin-containing liposome*

Featured are methods for determining the responsiveness of a cancer patient to treatment with one or more cancer therapies, in particular, a liposomal cisplatin composition, such as LiPlaCis. The patient may also be resistant to one or more cancer therapies other than $sPLA_2$ hydrolysable, cisplatin-containing liposome, such as LiPlaCis). The diagnostic methods include assaying the level of expression of one or more of the biomarkers shown in Tables 2-5 (e.g., COL5A2 (SEQ ID NO 73 or 211) or SFN (SEQ ID NO: 96 or 324)). The methods of the invention may be used for predicting a patient's responsiveness to $sPLA_2$ hydrolysable, cisplatin-containing liposome (e.g., LiPlaCis), and optionally, treating the cancer patient throughout the progression of cancer and/or in cases of recurrence (e.g., after a first line treatment, a second line treatment, and/or a third line treatment).

The invention provides individual biomarkers (e.g., COL5A2 (SEQ ID NO: 73 OR 211) and sets of biomarkers (e.g., two or more of the biomarkers listed in Tables 2-5), the expression levels of which, as detected in a biological sample (e.g., a tumor sample, such as a biopsy) obtained from a cancer patient (e.g., a human with cancer), are indicative of responsiveness to $sPLA_2$ hydrolysable, cisplatin-containing liposome (e.g., LiPlaCis). The biomarkers were identified using methods similar to those previously described in, e.g., Chen et al. (Mol. Cancer Ther. 11:34-33, 2012), Wang et al. (J. Nat. Cancer Inst. 105: 1284-1291, 2013), and Knudsen et al. (PLoS One, 9: e87415, 2014), each of which are incorporated by reference herein in their entirety. In particular, an algorithm based on growth inhibition values (GI50) of a cell line (e.g., NCI60 cells) is subjected to treatment with $sPLA_2$ hydrolysable, cisplatin-containing liposome (e.g., LiPlaCis) and gene expression is determined (e.g., by microarray analysis, reverse transcriptase polymerase chain reaction (RT-PCR), quantitative real-time PCR (qPCR), or next generation sequencing). After normalization, genes with, e.g., a Pearson correlation coefficient greater than about 0.2 or below about -0.2 can be classified as biomarkers of sensitivity or resistance, respectively. In particular, a correlation coefficient of about 0.2 or greater is a statistically significant cutoff known in the art for establishing whether the expression level of A GENE, e.g., the genes shown in Tables 2-5, correlate with the likelihood of cancer treatment sensitivity, such as sensitivity to $sPLA_2$ hydrolysable, cisplatin-containing liposome (e.g., LiPlaCis). Thus, a correlation coefficient of about 0.2 or greater or about -0.2 or lower can be used to estimate the statistical significance of the expression level of the genes of Tables 2-5 for predicting patient responsiveness to treatment with $sPLA_2$ hydrolysable, cisplatin-containing liposome (e.g., LiPlaCis) according to the methods described herein.

*Comparison of biomarker expression levels*

One or more biomarkers of sensitivity and/or resistance, identified as described herein, can be used to predict responsiveness to $sPLA_2$ hydrolysable, cisplatin-containing liposome (e.g., LiPlaCis) by measuring the expression level of the biomarkers in a biological sample obtained from the cancer patient. A single biomarker (e.g., any of the biomarkers of Tables 2-5, such as COL5A2 (SEQ ID NO: 73 OR 211) may be used to determine the responsiveness of a cancer patient (e.g., a patient having cancer that is resistant to one or more cancer therapies other than LiPlaCis) to $sPLA_2$ hydrolysable, cisplatin-containing liposome (e.g., LiPlaCis). After determining the expression level of the biomarker(s) in a sample (e.g., a tumor sample) from the cancer patient, the expression level of the biomarker(s) in the sample may be compared to the expression level of the biomarker(s) in a cell (e.g., a cancer cell) or tissue (e.g., a tumor tissue) known to be sensitive to treatment with $sPLA_2$ hydrolysable, cisplatin-containing liposome (e,g., LiPlaCis). If the expression level of the biomarker(s) in the sample from the cancer patient is substantially similar (e.g., identical to or has the same trend of expression level) to the expression level of the biomarker(s) in the cell or tissue known to be sensitive to $sPLA_2$ hydrolysable, cisplatin-containing liposome (e.g., LiPlaCis), then the cancer patient is predicted to be responsive to treatment with the $sPLA_2$ hydrolysable, cisplatin-containing liposome. Alternatively, if the expression level of the biomarker(s) in the sample from the cancer patient is substantially dissimilar to the expression level of the biomarker(s) in the cell or tissue known to be sensitive to $sPLA_2$ hydrolysable, cisplatin-containing liposome (e.g., LiPlaCis), then the cancer patient is predicted to be non-responsive to treatment with the $sPLA_2$ hydrolysable, cisplatin-containing liposome.

The expression level of the biomarker (e.g., COL5A2 (SEQ ID NO: 73 OR 211) in a sample from the cancer patient may also be compared to the expression level of the biomarker in a cell (e.g., a cancer cell) or tissue (e.g., a tumor tissue) known to be resistant to treatment with $sPLA_2$ hydrolysable, cisplatin-containing liposome (e.g., LiPlaCis). If the expression level of the biomarker in the sample from the cancer patient is substantially similar to the expression level of the biomarker in the cell or tissue known to be resistant to $sPLA_2$ hydrolysable, cisplatin-containing liposome (e.g., LiPlaCis), then the cancer patient is predicted to be non-responsive to treatment with the $sPLA_2$ hydrolysable, cisplatin-containing liposome. Alternatively, if the expression level of the biomarker in the sample from the cancer patient is substantially dissimilar to the expression level of the biomarker in the cell or tissue known to be sensitive to $sPLA_2$ hydrolysable, cisplatin-containing liposome (e.g., LiPlaCis), then the cancer patient is predicted to be responsive to treatment with the $sPLA_2$ hydrolysable, cisplatin-containing liposome.

The responsiveness of a cancer patient (e.g., a patient having cancer that is resistant to one or more cancer therapies other than $sPLA_2$ hydrolysable, cisplatin-containing liposome) to $sPLA_2$ hydrolysable, cisplatin-containing liposome (e.g., LiPlaCis) can also be predicted by comparing the expression level of a biomarker (e.g., COL5A2 (SEQ ID NO: 73 OR 211) to the expression level of the biomarker in one or more cells or tissues (e.g., from a cancer patient population) known to be sensitive to treatment with $sPLA_2$ hydrolysable, cisplatin-containing liposome and one or more cells or tissues (e.g., from a cancer patient population) known to be resistant to treatment with $sPLA_2$ hydrolysable, cisplatin-containing liposome. In particular, the patient may be responsive to treatment with $sPLA_2$ hydrolysable, cisplatin-containing liposome (e.g., LiPlaCis) if the expression level of the biomarker is more similar to the expression level of the biomarker in a cell or tissue known to be sensitive to treatment with the $sPLA_2$ hydrolysable, cisplatin-containing liposome than to a cell or tissue known to be resistant to treatment with the same. Alternatively, the patient may be non-responsive to treatment with $sPLA_2$ hydrolysable, cisplatin-containing liposome (e.g., LiPlaCis) if the expression level of the biomarker is more similar to the expression level of the biomarker in a cell or tissue known to be resistant to treatment with the $sPLA_2$ hydrolysable, cisplatin-containing liposome than to a cell or tissue known to be sensitive to treatment with the same.

Additionally, one or more biomarkers of sensitivity (e.g., one or more of COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15)) and one or more biomarkers of resistance (e.g., one or more of SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112)) may be used in combination to determine the responsiveness of a cancer patient (e.g., a patient having cancer that is resistant to one or more cancer therapies other than $sPLA_2$ hydrolysable, cisplatin-containing liposome) to treatment with $sPLA_2$ hydrolysable, cisplatin-containing liposome (e.g., LiPlaCis). For example, the predicted responsiveness of a cancer patient may be determined from, e.g., the difference score, which may be defined as the difference between the mean of the expression level of the one or more biomarkers of sensitivity of Tables 2 and 4 and the mean of the expression level of the one or more biomarkers of resistance of Tables 3 and 5.

The difference score of the cancer patient can then be compared to the difference score based on the expression level of the biomarkers in a cell (e.g., a cancer cell) or tissue (e.g., a tumor tissue) known to be sensitive or resistant to treatment with $sPLA_2$ hydrolysable, cisplatin-containing liposome. In particular, the patient may be responsive to treatment with $sPLA_2$ hydrolysable, cisplatin-containing liposome (e.g., LiPlaCis) if the difference score is substantially similar to the expression level of the biomarkers in a cell or tissue known to be sensitive to treatment with the $sPLA_2$ hydrolysable, cisplatin-containing liposome. Alternatively, the patient may be non-responsive to treatment with $sPLA_2$ hydrolysable, cisplatin-containing liposome (e.g., LiPlaCis) if the difference score is substantially similar to the expression level of the biomarkers in a cell or tissue known to be resistant to treatment with the $sPLA_2$ hydrolysable, cisplatin-containing liposome.

Additionally, the patient may be responsive to treatment with $sPLA_2$ hydrolysable, cisplatin-containing liposome (e.g., LiPlaCis) if the difference score is substantially similar to the expression level of the biomarkers in a cell or tissue known to be sensitive to treatment with the $sPLA_2$ hydrolysable, cisplatin-containing liposome than a cell or tissue known to be resistant to treatment with the same. Alternatively, the patient may be non-responsive to treatment with $sPLA_2$ hydrolysable, cisplatin-containing liposome (e.g., LiPlaCis) if the difference score is substantially similar to the expression level of the biomarkers in a cell or tissue known to be resistant to treatment with the $sPLA_2$ hydrolysable, cisplatin-containing liposome than a cell or tissue known to be sensitive to treatment with the same.

One or more biomarkers of sensitivity and/or resistance, identified as described herein, can be used to predict responsiveness to $sPLA_2$ hydrolysable, cisplatin-containing liposome (e.g., LiPlaCis) by measuring the expression level of the biomarkers in a biological sample obtained from the cancer patient. A single biomarker (e.g., any of the biomarkers of Tables 2-5, such as COL5A2 (SEQ ID NO: 73 OR 211) may be used to determine the responsiveness of a cancer patient (e.g., a patient having cancer that is resistant to one or more cancer therapies other than $sPLA_2$ hydrolysable, cisplatin-containing liposome) to $sPLA_2$ hydrolysable, cisplatin-containing liposome (e.g., LiPlaCis). After determining the expression level of the biomarker(s) in a sample (e.g., a tumor sample) from the cancer patient, the expression level of the biomarker(s) in the sample may be compared to the expression level of the biomarker(s) in a cell (e.g., a cancer cell) or tissue (e.g., a tumor tissue) known to be sensitive to treatment with $sPLA_2$ hydrolysable, cisplatin-containing liposome. If the expression level of the biomarker(s) in the sample from the cancer patient corresponds to (e.g., is identical to or has the same trend of expression level as) the expression level of the biomarker(s) in the cell or tissue known to be sensitive to $sPLA_2$ hydrolysable, cisplatin-containing liposome, then the cancer patient is predicted to be responsive to treatment with the $sPLA_2$ hydrolysable, cisplatin-containing liposome. Alternatively, if the expression level of the biomarker(s) in the sample from the cancer patient is substantially dissimilar to the expression level of the biomarker(s) in the cell or tissue known to be sensitive to $sPLA_2$ hydrolysable, cisplatin-containing liposome, then the cancer patient is predicted to be non-responsive to treatment with the $sPLA_2$ hydrolysable, cisplatin-containing liposome.

The expression level of the biomarker (e.g., COL5A2 (SEQ ID NO: 73 OR 211) in a sample from the cancer patient may also be compared to the expression level of the biomarker in a cell (e.g., a cancer cell) or tissue (e.g., a tumor tissue) known to be resistant to treatment with $sPLA_2$ hydrolysable, cisplatin-containing liposome (e.g., LiPlaCis). If the expression level of the biomarker in the sample from the cancer patient corresponds to the expression level of the biomarker in the cell or tissue known to be resistant to $sPLA_2$ hydrolysable, cisplatin-containing liposome, then the cancer patient is predicted to be non-responsive to treatment with the $sPLA_2$ hydrolysable, cisplatin-containing liposome. Alternatively, if the expression level of the biomarker in the sample from the cancer patient is substantially dissimilar to the expression level of the biomarker in the cell or tissue known to be resistant to $sPLA_2$ hydrolysable, cisplatin-containing liposome, then the cancer patient is predicted to be responsive to treatment with the $sPLA_2$ hydrolysable, cisplatin-containing liposome.

The responsiveness of a cancer patient (e.g., a patient having cancer that is resistant to one or more cancer therapies other than $sPLA_2$ hydrolysable, cisplatin-containing liposome) to $sPLA_2$ hydrolysable, cisplatin-containing liposome (e.g., LiPlaCis) can also be predicted by comparing the expression level of a biomarker (e.g., COL5A2 (SEQ ID NO: 73 OR 211) to the expression level of the biomarker in one or more cells or tissues (e.g., from a cancer patient population) known to be sensitive to treatment with $sPLA_2$ hydrolysable, cisplatin-containing liposome and one or more cells or tissues (e.g., from a cancer patient population) known to be resistant to treatment with the same. In particular, the patient may be responsive to treatment with $sPLA_2$ hydrolysable, cisplatin-containing liposome (e.g., LiPlaCis) if the expression level of the biomarker(s) corresponds to the expression level of the biomarker(s) in a cell or tissue known to be sensitive to treatment with the $sPLA_2$ hydrolysable, cisplatin-containing liposome relative to the expression level of the biomarkers in a cell or tissue known to be resistant to treatment with the same. Alternatively, the patient may be non-responsive to treatment with $sPLA_2$ hydrolysable, cisplatin-containing liposome (e.g., LiPlaCis) if the expression level of the biomarker(s) corresponds to the expression level of the biomarker(s) in a cell or tissue known to be resistant to treatment with the $sPLA_2$ hydrolysable, cisplatin-containing liposome relative to the expression level of the biomarkers in a cell or tissue known to be resistant to treatment with the same.

Additionally, one or more biomarkers of sensitivity (e.g., one or more of COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15)) and one or more biomarkers of resistance (e.g., one or more of SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112)) may be used in combination to determine the responsiveness of a cancer patient (e.g., a patient having cancer that is resistant to one or more cancer therapies other than $sPLA_2$ hydrolysable, cisplatin-containing liposome) to treatment with $sPLA_2$ hydrolysable, cisplatin-

containing liposome (e.g., LiPlaCis). For example, the predicted responsiveness of a cancer patient may be determined from, e.g., the difference score, which may be defined as the difference between the mean of the expression level of the one or more biomarkers of sensitivity of Tables 2 and 4 and the mean of the expression level of the one or more biomarkers of resistance of Tables 3 and 5.

The difference score of the cancer patient can then be compared to the difference score based on the expression level of the biomarkers in a cell (e.g., a cancer cell) or tissue (e.g., a tumor tissue) known to be sensitive or resistant to treatment with $sPLA_2$ hydrolysable, cisplatin-containing liposome. In particular, the patient may be responsive to treatment with $sPLA_2$ hydrolysable, cisplatin-containing liposome (e.g., LiPlaCis) if the difference score corresponds to the expression level of the biomarkers in a cell or tissue known to be sensitive to treatment with the $sPLA_2$ hydrolysable, cisplatin-containing liposome. Alternatively, the patient may be non-responsive to treatment with $sPLA_2$ hydrolysable, cisplatin-containing liposome (e.g., LiPlaCis) if the difference score corresponds to the expression level of the biomarkers in a cell or tissue known to be resistant to treatment with the $sPLA_2$ hydrolysable, cisplatin-containing liposome. Additionally, the patient may be responsive to treatment with $sPLA_2$ hydrolysable, cisplatin-containing liposome (e.g., LiPlaCis) if the difference score corresponds to the expression level of the biomarkers in a cell or tissue known to be sensitive to treatment with the $sPLA_2$ hydrolysable, cisplatin-containing liposome relative to the expression level of the biomarkers in a cell or tissue known to be resistant to treatment with the same. Alternatively, the patient may be non-responsive to treatment with $sPLA_2$ hydrolysable, cisplatin-containing liposome (e.g., LiPlaCis) if the difference score corresponds to the expression level of the biomarkers in a cell or tissue known to be resistant to treatment with the $sPLA_2$ hydrolysable, cisplatin-containing liposome relative to the expression level of the biomarkers in a cell or tissue known to be sensitive to treatment with the same.

Preferably, the cell or tissue known to be either sensitive or resistant to $sPLA_2$ hydrolysable, cisplatin-containing liposome (e.g., LiPlaCis) is of the same cancer type as the cancer patient with an unknown responsiveness to $sPLA_2$ hydrolysable, cisplatin-containing liposome. For example, the cancer patient and the cell or tissue known to be either sensitive or resistant to $sPLA_2$ hydrolysable, cisplatin-containing liposome (e.g., LiPlaCis) may both have a cancer type selected from a solid tumor or a hematological cancer, e.g., prostate cancer, ovarian cancer (e.g., ovarian adenocarcinoma or embryonal carcinoma), liver cancer (e.g., hepatocellular carcinoma (HCC) or hepatoma), myeloma (e.g., multiple myeloma), colorectal cancer (e.g., colon cancer and rectal cancer), leukemia (e.g., acute myeloid leukemia, acute lymphoid leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, Waldenstrom's macroglobulinemia, and lymphocytic lymphoma), cervical cancer, esophageal cancer, melanoma, glioma (e.g., oligodendroglioma), pancreatic cancer (e.g., adenosquamous carcinoma, signet ring cell carcinoma, hepatoid carcinoma, colloid carcinoma, islet cell carcinoma, and pancreatic neuroendocrine carcinoma), gastrointestinal stromal tumor, sarcoma (e.g., fibrosarcoma, myxosarcoma, liposarcoma, chondrosarcoma, osteogenic sarcoma, angiosarcoma, endotheliosarcoma, lymphangiosarcoma, lymphangioendotheliosarcoma, leiomyosarcoma, Ewing's sarcoma, and rhabdomyosarcoma), breast cancer (e.g., medullary carcinoma), ER-positive cancer, bladder cancer, head and neck cancer (e.g., squamous cell carcinoma of the head and neck), lung cancer (e.g., non-small cell lung carcinoma, large cell carcinoma, bronchogenic carcinoma, and papillary adenocarcinoma), metastatic cancer, oral cavity cancer, uterine cancer, testicular cancer (e.g., seminoma and embryonal carcinoma), skin cancer (e.g., squamous cell carcinoma and basal cell carcinoma), thyroid cancer (e.g., papillary carcinoma and medullary carcinoma), brain cancer (e.g., astrocytoma and craniopharyngioma), stomach cancer, intra-epithelial cancer, bone cancer, biliary tract cancer, eye cancer, larynx cancer, kidney cancer (e.g., renal cell carcinoma and Wilms tumor), gastric cancer, blastoma (e.g., nephroblastoma, medulloblastoma, hemangioblastoma, neuroblastoma, and retinoblastoma), polycythemia vera, chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, cancer of the endocrine system, cancer of the penis, cancer of the urethra, cutaneous or intraocular melanoma, a gynecologic tumor, solid tumors of childhood, and neoplasms of the central nervous system. In particular, the cancer of the patient and the cell or tissue with known resistance or sensitivity to LiPlaCis is, e.g., prostate cancer, ovarian cancer, hepatocellular carcinoma (HCC), multiple myeloma, breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, cervical cancer, renal cell carcinoma (RCC), esophageal cancer, melanoma, glioma, pancreatic cancer, gastrointestinal stromal tumors (GIST), sarcoma, estrogen receptor-positive (ERpos) breast cancer, non-small cell lung carcinoma (NSCLC), colon cancer, bladder cancer, or squamous cell carcinoma of the head and neck (SCCHN).

Machine learning techniques such as Neural Networks, Support Vector Machines, K Nearest Neighbor, and Nearest Centroids may also be employed to develop models that discriminate patients sensitive to treatment with $sPLA_2$

hydrolysable, cisplatin-containing liposome from those resistant to treatment with $sPLA_2$ hydrolysable, cisplatin-containing liposome using biomarker expression as model variables which assign each patient a classification as sensitive or resistant to treatment with the same. Machine learning techniques used to classify patients using various measurements are described in U.S. Patent No. 5,822,715; U.S. Patent Application Publication Nos. 2003/0073083, 2005/0227266, 2005/0208512, 2005/0123945, 2003/0129629, and 2002/0006613; and in Vapnik V N. Statistical Learning Theory, John Wiley & Sons, New York, 1998; Hastie et al., 2001, The Elements of Statistical Learning: Data Mining, Inference, and Prediction, Springer, N.Y.; Agresti, 1996, An Introduction to Categorical Data Analysis, John Wiley & Sons, New York; V. Tresp et al., "Neural Network Modeling of Physiological Processes," in Hanson S. J. et al. (Eds.), Computational Learning Theory and Natural Learning Systems 2, MIT Press, 1994, each of which are hereby incorporated by reference in their entirety.

*Biomarkers of sensitivity and resistance*

The expression levels of one or more biomarkers of Tables 2-5 can be used to determine cancer patient responsiveness to treatment with $sPLA_2$ hydrolysable, cisplatin-containing liposome (e.g., LiPlaCis). Once determined to be responsive, the patient can be treated with $sPLA_2$ hydrolysable, cisplatin-containing liposome (e.g., LiPlaCis). In particular, the biomarker COL5A2 (SEQ ID NO 73 or 211) may be used to assess a cancer patient's (e.g., a patient with cancer that is resistant to one or more cancer therapies other than $sPLA_2$ hydrolysable, cisplatin-containing liposome) responsiveness to $sPLA_2$ hydrolysable, cisplatin-containing liposome. The expression level of the biomarker COL5A2 (SEQ ID NO 73 or 211) may be assessed using nucleic acid amplification methods (e.g., PCR) or a device (e.g., a microarray). As is described above, the expression level of COL5A2 (SEQ ID NO 73 or 211) in the patient sample may then be compared, e.g., to the expression level of COL5A2 (SEQ ID NO 73 or 211) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker COL5A2 (SEQ ID NO 73 or 211) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The expression level of the biomarker ITGA4 (SEQ ID NO: 1) may be assessed using nucleic acid amplification methods (e.g., PCR) or a device (e.g., a microarray). As is described above, the expression level of ITGA4 (SEQ ID NO: 1) in the patient sample may then be compared, e.g., to the expression level of ITGA4 (SEQ ID NO: 1) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker ITGA4 (SEQ ID NO: 1) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker MSN (SEQ ID NO: 2) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker MSN (SEQ ID NO: 2) may be assessed using nucleic acid amplification methods (e.g., PCR) or a device (e.g., a microarray). As is described above, the expression level of MSN (SEQ ID NO: 2) in the patient sample may then be compared, e.g., to the expression level of MSN (SEQ ID NO: 2) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker MSN (SEQ ID NO: 2) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker FAM46A (SEQ ID NO: 3 or 280) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker FAM46A (SEQ ID NO: 3 or 280) may be assessed using nucleic acid amplification methods (e.g., PCR) or a device (e.g., a microarray). As is described above, the expression level of FAM46A (SEQ ID NO: 3 or 280) in the patient sample may then be compared, e.g., to the expression level of FAM46A (SEQ ID NO: 3 or 280) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker FAM46A (SEQ ID NO: 3 or 280) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker ITGB2 (SEQ ID NO: 4) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker ITGB2 (SEQ ID NO: 4) may be assessed using nucleic acid amplification methods (e.g., PCR) or a device (e.g., a microarray). As is described above, the expression level of ITGB2 (SEQ ID NO: 4) in the patient sample may then be compared, e.g., to the expression level of ITGB2 (SEQ ID NO: 4) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker ITGB2 (SEQ ID NO: 4) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker DOCK2 (SEQ ID NO: 5 or 223) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker DOCK2 (SEQ ID NO: 5 or 223) may be assessed using nucleic acid amplification methods (e.g., PCR) or a device (e.g., a microarray). As is described above, the expression level of DOCK2 (SEQ ID NO: 5 or 223) in the patient sample may then be compared, e.g., to the expression level of DOCK2 (SEQ ID NO: 5 or 223) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker DOCK2 (SEQ ID NO: 5 or 223) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker EVL (SEQ ID NO: 6) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker EVL (SEQ ID NO: 6) may be assessed using nucleic acid amplification methods (e.g., PCR) or a device (e.g., a microarray). As is described above, the expression level of EVL (SEQ ID NO: 6) in the patient sample may then be compared, e.g., to the expression level of EVL (SEQ ID NO: 6) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker EVL (SEQ ID NO: 6) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker SACS (SEQ ID NO: 7) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker SACS (SEQ ID NO: 7) may be assessed using nucleic acid amplification methods (e.g., PCR) or a device (e.g., a microarray). As is described above, the expression level of SACS (SEQ ID NO: 7) in the patient sample may then be compared, e.g., to the expression level of SACS (SEQ ID NO: 7) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker SACS (SEQ ID NO: 7) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The expression levels of one or more biomarkers of Tables 2-5 can be used to determine cancer patient responsiveness to treatment with $sPLA_2$ hydrolysable, cisplatin-containing liposome. Once determined to be responsive, the patient can be treated with $sPLA_2$ hydrolysable, cisplatin-containing liposome (e.g., LiPlaCis). In particular, the biomarker PTPRCAP (SEQ ID NO: 8) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome. The expression level of the biomarker PTPRCAP (SEQ ID NO: 8) may be assessed using nucleic acid amplification methods (e.g., PCR) or a device (e.g., a microarray). As is described above, the expression level of PTPRCAP (SEQ ID NO: 8) in the patient sample may then be compared, e.g., to the expression level of PTPRCAP (SEQ ID NO: 8) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker PTPRCAP (SEQ ID NO: 8) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO:7), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41 L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker EBI2 (SEQ ID NO: 9) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker EBI2 (SEQ ID NO: 9) may be assessed using nucleic acid amplification methods (e.g., PCR) or a device (e.g., a microarray). As is described above, the expression level of EBI2 (SEQ ID NO: 9) in the patient sample may then be compared, e.g., to the expression level of EBI2 (SEQ ID NO: 9) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker EBI2 (SEQ ID NO: 9) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker PTPRC (SEQ ID NO: 10, 18, 25, or 243) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker PTPRC (SEQ ID NO: 10, 18, 25, or 243) may be assessed using nucleic acid amplification methods (e.g., PCR) or a device (e.g., a microarray). As is described above, the expression level of PTPRC (SEQ ID NO: 10, 18, 25, or 243) in the patient sample may then be compared, e.g., to the expression level of PTPRC (SEQ ID NO: 10, 18, 25, or 243) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker PTPRC (SEQ ID NO: 10, 18, 25, or 243) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker ANP32E (SEQ ID NO: 11) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker ANP32E (SEQ ID NO: 11) may be assessed using nucleic acid amplification methods (e.g., PCR) or a device (e.g., a microarray). As is described above, the expression level of ANP32E (SEQ ID NO: 11) in the patient sample may then be compared, e.g., to the expression level of ANP32E (SEQ ID NO: 11) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker ANP32E (SEQ ID NO: 11) may be used alone to predict cancer patient responsiveness to treatment with LiPlaCis or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker SFPQ (SEQ ID NO: 12, 38 or 272) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker SFPQ (SEQ ID NO: 12, 38 or 272) may be assessed using nucleic acid amplification methods (e.g., PCR) or a device (e.g., a microarray). As is described above, the expression level of SFPQ (SEQ ID NO: 12, 38 or 272) in the patient sample may then be compared, e.g., to the expression level of SFPQ (SEQ ID NO: 12, 38 or 272) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker SFPQ (SEQ ID NO: 12, 38 or 272) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker C1QR1 (SEQ ID NO: 13) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker C1QR1 (SEQ ID NO: 13) may be assessed using nucleic acid amplification methods (e.g., PCR) or a device (e.g., a microarray). As is described above, the expression level of C1QR1 (SEQ ID NO: 13) in the patient sample may then be compared, e.g., to the expression level of C1QR1 (SEQ ID NO: 13) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker C1QR1 (SEQ ID NO: 13) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), FNBP1 (SEQ ID NO: 14 or 28), CBFB (SEQ ID NO: 15), SFN (SEQ ID NO: 96 or 324), LISCH7 (SEQ ID NO: 97), EPB41 L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker FNBP1 (SEQ ID NO: 14 or 28) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker FNBP1 (SEQ ID NO: 14 or 28) may be assessed using nucleic acid amplification methods (e.g., PCR) or a device (e.g., a microarray). As is described above, the expression level of FNBP1 (SEQ ID NO: 14 or 28) in the patient sample may then be compared, e.g., to the expression level of FNBP1 (SEQ ID NO: 14 or 28) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker FNBP1 (SEQ ID NO: 14 or 28) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO:7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), CBFB (SEQ ID NO: 15), SFN (SEQ ID NO: 96 or 324), LISCH7 (SEQ ID NO: 97), EPB41 L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker SFN (SEQ ID NO: 96 or 324) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker SFN (SEQ ID NO: 96 or 324) may be assessed using nucleic acid amplification methods (e.g., PCR) or a device (e.g., a microarray). As is described above, the expression level of SFN (SEQ ID NO: 96 or 324) in the patient sample may then be compared, e.g., to the expression level of SFN (SEQ ID NO: 96 or 324) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker SFN (SEQ ID NO: 96 or 324) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO:7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker LISCH7 (SEQ ID NO: 97) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker LISCH7 (SEQ ID NO: 97) may be assessed using nucleic acid amplification methods (e.g., PCR) or a device (e.g., a microarray). As is described above, the expression level of LISCH7 (SEQ ID NO: 97) in the patient sample may then be compared, e.g., to the expression level of LISCH7 (SEQ ID NO: 97) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker LISCH7 (SEQ ID NO: 97) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO:7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), EPB41 L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker EPB41 L4B (SEQ ID NO: 98) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker EPB41 L4B (SEQ ID NO: 98) may be assessed using nucleic acid amplification methods (e.g., PCR) or a device (e.g., a microarray). As is described above, the expression level of EPB41 L4B (SEQ ID NO: 98) in the patient sample may then be compared, e.g., to the expression level of EPB41 L4B (SEQ ID NO: 98) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker EPB41L4B (SEQ ID NO: 98) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO:7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker MST1R (SEQ ID NO: 99) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker MST1R (SEQ ID NO: 99) may be assessed using nucleic acid amplification methods (e.g., PCR) or a device (e.g., a microarray). As is described above, the expression level of MST1R (SEQ ID NO: 99) in the patient sample may then be compared, e.g., to the expression level of MST1R (SEQ ID NO: 99) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker MST1R (SEQ ID NO: 99) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker ITGB4 (SEQ ID NO: 100) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker ITGB4 (SEQ ID NO: 100) may be assessed using nucleic acid amplification methods (e.g., PCR) or a device (e.g., a microarray). As is described above, the expression level of ITGB4 (SEQ ID NO: 100) in the patient sample may then be compared, e.g., to the expression level of ITGB4 (SEQ ID NO: 100) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker ITGB4 (SEQ ID NO: 100) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41 L4B (SEQ ID NO: 98), MST1R (SEQ ID NO: 99), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker DBNDD2 (SEQ ID NO: 102 or 365) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker DBNDD2 (SEQ ID NO: 102 or 365) may be assessed using nucleic acid amplification methods (e.g., PCR) or a device (e.g., a microarray). As is described above, the expression level of DBNDD2 (SEQ ID NO: 102 or 365) in the patient sample may then be compared, e.g., to the expression level of DBNDD2 (SEQ ID NO: 102 or 365) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker DBNDD2 (SEQ ID NO: 102 or 365) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker TACSTD1 (SEQ ID NO: 104) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker TACSTD1 (SEQ ID NO: 104) may be assessed using nucleic acid amplification methods (e.g., PCR) or a device (e.g., a microarray). As is described above, the expression level of TACSTD1 (SEQ ID NO: 104) in the patient sample may then be compared, e.g., to the expression level of TACSTD1 (SEQ ID NO: 104) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker TACSTD1 (SEQ ID NO: 104) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker MISP (SEQ ID NO: 105) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker MISP (SEQ ID NO: 105) may be assessed using nucleic acid amplification methods (e.g., PCR) or a device (e.g., a microarray). As is described above, the expression level of MISP (SEQ ID NO: 105) in the patient sample may then be compared, e.g., to the expression level of MISP (SEQ ID NO: 105) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker MISP (SEQ ID NO: 105) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker KRT8 (SEQ ID NO: 106) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis) using nucleic acid amplification methods (e.g., PCR) or a device (e.g., a microarray). As is described above, the expression level of KRT8 (SEQ ID NO: 106) in the patient sample may then be compared, e.g., to the expression level of KRT8 (SEQ ID NO: 106) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker KRT8 (SEQ ID NO: 106) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker JUP (SEQ ID NO: 107 or 400) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker JUP (SEQ ID NO: 107 or 400) may be assessed using nucleic acid amplification methods (e.g., PCR) or a device (e.g., a microarray). As is described above, the expression level of JUP (SEQ ID NO: 107 or 400) in the patient sample may then be compared, e.g., to the expression level of JUP (SEQ ID NO: 107 or 400) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker JUP (SEQ ID NO: 107 or 400) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker KRT18 (SEQ ID NO: 108 or 306) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker KRT18 (SEQ ID NO: 108 or 306) may be assessed using nucleic acid amplification methods (e.g., PCR) or a device (e.g., a microarray). As is described above, the expression level of KRT18 (SEQ ID NO: 108 or 306) in the patient sample may then be compared, e.g., to the expression level of KRT18 (SEQ ID NO: 108 or 306) in a cell (e.g., a cancer cell) or tissue (e.g., a tumor tissue) known to be sensitive or resistant to treatment with $sPLA_2$ hydrolysable,

cisplatin-containing liposome and used to determine the cancer patient's responsiveness to the same. The biomarker KRT18 (SEQ ID NO: 108 or 306) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), FA2H (SEQ ID NO: 109), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker FA2H (SEQ ID NO: 109) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker FA2H (SEQ ID NO: 109) may be assessed using nucleic acid amplification methods (e.g., PCR) or a device (e.g., a microarray). As is described above, the expression level of FA2H (SEQ ID NO: 109) in the patient sample may then be compared, e.g., to the expression level of FA2H (SEQ ID NO: 109) in a cell (e.g., a cancer cell) or tissue (e.g., a tumor tissue) known to be sensitive or resistant to treatment with $sPLA_2$ hydrolysable, cisplatin-containing liposome s and used to determine the cancer patient's responsiveness to the same. The biomarker FA2H (SEQ ID NO: 109) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), MGAT4B (SEQ ID NO: 110), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker MGAT4B (SEQ ID NO: 110) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker MGAT4B (SEQ ID NO: 110) may be assessed using nucleic acid amplification methods (e.g., PCR) or a device (e.g., a microarray). As is described above, the expression level of MGAT4B (SEQ ID NO: 110) in the patient sample may then be compared, e.g., to the expression level of MGAT4B (SEQ ID NO: 110) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker MGAT4B (SEQ ID NO: 110) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), DSG2 (SEQ ID NO:111 or 312), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker DSG2 (SEQ ID NO:111 or 312) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker DSG2 (SEQ ID NO:111 or 312) may be assessed using nucleic acid amplification methods (e.g., PCR) or a device (e.g., a microarray). As is described above, the expression level of DSG2 (SEQ ID NO:111 or 312) in the patient sample may then be compared, e.g., to the expression level of DSG2 (SEQ ID NO:111 or 312) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker DSG2 (SEQ ID NO:111 or 312) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), LRP5 (SEQ ID NO: 112). The expression level of the biomarker(s) may be determined using, e.g., a microarray, PCR, or other techniques described herein, for example, using a nucleic acid probe sequence based on the target sequences shown in Tables 2-5.

The biomarker LRP5 (SEQ ID NO: 112) may be used to assess a cancer patient's (e.g., a patient having cancer that is resistant to one or more cancer therapies other than $sPLA_2$ hydrolysable, cisplatin-containing liposome) responsiveness to $sPLA_2$ hydrolysable, cisplatin-containing liposome (e.g., LiPlaCis). The expression level of the biomarker LRP5 (SEQ ID NO: 112) may be assessed using nucleic acid amplification methods (e.g., PCR) or a device (e.g., a microarray). As is described above, the expression level of LRP5 (SEQ ID NO: 112) in the patient sample may then be compared, e.g., to the expression level of LRP5 (SEQ ID NO: 112) in a cell (e.g., a cancer cell) or tissue (e.g., a tumor tissue) known to be sensitive or resistant to treatment with $sPLA_2$ hydrolysable, cisplatin-containing liposome and used to determine the cancer patient's responsiveness to the same. The biomarker LRP5 (SEQ ID NO: 112) may be used alone to predict cancer patient responsiveness to treatment with $sPLA_2$ hydrolysable, cisplatin-containing liposome or in combination with one or more additional biomarkers (e.g., one, two, three, four, five, ten, or all of the biomarkers shown in Tables 2-5), such as COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or272), C1QR1 (SEQ ID NO: 13), FNBP1 (SEQ ID NO: 14 or 28), CBFB (SEQ ID NO: 15), SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312). The expression level of the biomarker(s) may be determined using, e.g., a microarray, PCR, or other techniques described herein, for example, using a nucleic acid probe sequence based on the target sequences shown in Tables 2-5.

**Table 2.** mRNA biomarkers of sensitivity to cisplatin. Dashes mean that the Affymetrix probeset has not been mapped to a specific gene. Affymetrix IDs refer to the array type HG-U133A.

| Gene | Affymetrix ID | Correlation | Affymetrix Probe Sequence | SEQ ID NO: |
|---|---|---|---|---|
| ITGA4 | 213416_at | 0.46 | CAGGCCTCTCAGATACAAGGGGAAC | 1 |
| MSN | 200600_at | 0.45 | ATAGCTGCCTTAAAGTCAGTAACTT | 2 |
| FAM46A | 221766_s_at | 0.41 | CACCATGCTGGCTATCCGGGTGTTA | 3 |
| ITGB2 | 202803_s_at | 0.39 | CTCCACTCTGACTGGCACAGTCTTT | 4 |
| DOCK2 | 213160_at | 0.39 | GATTCCTGAACTCAAGGTACCAGCA | 5 |
| EVL | 217838_s_at | 0.39 | GATCATCGACGCCATCAGGCAGGAG | 6 |
| SACS | 213262_at | 0.38 | GTGTGGTTGAACAGGATGCAATCTT | 7 |
| PTPRCAP | 204960_at | 0.37 | GCTTCCCAAGATGCCATGGCTGGAC | 8 |
| EBI2 | 205419_at | 0.37 | GCAGGACTTCCCTTATAAAGCAAAA | 9 |
| PTPRC | 212587_s_at | 0.37 | GATTATAACCGTGTTGAACTCTCTG | 10 |
| ANP32E | 221505_at | 0.37 | GTTTTCGGTCCTATTTTAATGCTCT | 11 |
| SFPQ | 201586_s_at | 0.36 | AAAGACCAACAAATCTCAAGCCCTA | 12 |

(continued)

| Gene | Affymetrix ID | Correlation | Affymetrix Probe Sequence | SEQ ID NO: |
|---|---|---|---|---|
| C1QR1 | 202878_s_at | 0.36 | GGTCTGTTCTTGTAGATAATGCCCT | 13 |
| FNBP1 | 213940_s_at | 0.36 | TGCTGGCCACGGATTTTGACGACGA | 14 |
| CBFB | 202370_s_at | 0.35 | GGTGTTGTACAGCTCACATGTTTAC | 15 |
| HCLS1 | 202957_at | 0.35 | GGTTTGCCTCATTGTGCTATTTGCC | 16 |
| IFI16 | 208965_s_at | 0.35 | ATAAGCATTGATTCCTGCATTTCTG | 17 |
| PTPRC | 212588_at | 0.35 | GCATTTAGTCCAATGTCTTTTTAAG | 18 |
| SFRS7 | 213649_at | 0.35 | ATCATGCTGAGGCGCCTTGCAAATC | 19 |
| CAP350 | 204373_s_at | 0.34 | ATGACTGGTATGATAGCTCTTGACA | 20 |
| IGLL1 | 206660_at | 0.34 | CAATCCAAGCATAACTCAGTGACGC | 21 |
| DOCK10 | 219279_at | 0.34 | GAATGTGTAGCTCAAATGCAAACCA | 22 |
| WASPIP | 202664_at | 0.33 | TTCCCTCCTTATAGTCAAGGACCGT | 23 |
| FLI1 | 204236_at | 0.33 | TGACCTCGGTCACAAAAGCAGTTTT | 24 |
| PTPRC | 207238_s_at | 0.33 | GAACAGTTTGTACAGACGTATGCTT | 25 |
| IFI16 | 208966_x_at | 0.33 | TACAACACTATACATACACACCACC | 26 |
| HDGFRP3 | 209524_at | 0.33 | TTATGCCAGCTTATATTGTGAGAAC | 27 |
| FNBP1 | 212288_at | 0.33 | GAGTTGCCTGTTTGTCTCTGGAGAT | 28 |
| SEPT6 | 212414_s_at | 0.33 | GCTGCAGTGTAGATGGCTCTTGTTT | 29 |
| ARHGAP15 | 218870_at | 0.33 | ACGTTGTCACCGGAGCACTGAAGAT | 30 |
| RASSF2 | 203185_at | 0.32 | ATAGCAGCACACATTTTCACGTTTC | 31 |
| GMFG | 204220_at | 0.32 | AAGACCGGCAGATGGTGGTGCTGGA | 32 |
| SYNCRIP | 209025_s_at | 0.32 | ATTTGGCTCAAGTCCATTTGGCTGT | 33 |
| HDGFRP3 | 209526_s_at | 0.32 | GCATGAAGTTGCCCTTAACCACTAA | 34 |
| ARHGEF6 | 209539_at | 0.32 | TAACCATGCTTACACACTAAACTAT | 35 |
| TMEM5 | 204808_s_at | 0.31 | TGCCCGGTCGGAGTAAACACAGAAT | 36 |
| CENTB1 | 205213_at | 0.31 | GATGTCAACTGGGTCAATGGGGGCC | 37 |
| SFPQ | 214016_s_at | 0.31 | GTTGGCTGATATTGGAGTGCTCATT | 38 |
| BCAT1 | 214452_at | 0.31 | CCTTTTGTACTTCACTCAGATACTA | 39 |
| LCP1 | 208885_at | 0.3 | TAAGCATCCTTAGGGTTCTGCCTCT | 40 |
| COR01A | 209083_at | 0.3 | CTCATCTCCCTCAAGGATGGCTACG | 41 |
| SLC4A7 | 209884_s_at | 0.3 | TGTGAATCATCCTGCCTTTCAAATT | 42 |
| RAFTLIN | 212646_at | 0.3 | TACAAACCACATTACTTCTGTCACT | 43 |
| CKIP-1 | 218223_s_at | 0.3 | GTCCCGGATCCAGGACCTGGTAGCA | 44 |
| SNRP70 | 201221_s_at | 0.29 | AGTGAAGAGGTCGTCCTCTCCATCT | 45 |
| BNIP3 | 201849_at | 0.29 | GCTGAAGGCACCTACTCAGTATCTT | 46 |
| SLA | 203761_at | 0.29 | TAAGCATTCCGTCCATCTAAGCTCA | 47 |
| MFNG | 204153_s_at | 0.29 | TGATGGAGCATAACGGGTCCCAGCC | 48 |
| LOC57821 | 206721_at | 0.29 | ATGATTTCTTAGGGTCTGTGTACTT | 49 |

(continued)

| Gene | Affymetrix ID | Correlation | Affymetrix Probe Sequence | SEQ ID NO: |
|---|---|---|---|---|
| CBLB | 209682_at | 0.29 | GTTCCATTTCTCTCATTCACAAGAT | 50 |
| QKI | 212636_at | 0.29 | GAGGCCAAGAAATTCCATGTTGTTT | 51 |
| ZRF1 | 213097_s_at | 0.29 | AAAGCTGTGAATCTGTTCCCTGCTG | 52 |
| FTL | 213187_x_at | 0.29 | ATGAGCTCCCAGATTCGTCAGAATT | 53 |
| SFRS7 | 214141_x_at | 0.29 | TCCCCATCAGGAAGTCCTCGCAGAA | 54 |
| VIM | 201426_s_at | 0.28 | TGAGTCCCTGGAACGCCAGATGCGT | 55 |
| PWP1 | 201606_s_at | 0.28 | TTAGAGCCAGTCTTCACACTCGGAA | 56 |
| AKAP7 | 205771_s_at | 0.28 | AAAACTTCCCCGGTATGATGATTGT | 57 |
| AF1Q | 211071_s_at | 0.28 | TCAGTGGGCACAGTTCTTCAGCTAC | 58 |
| DICER1 | 213229_at | 0.28 | ACTAGCTCATTATTTCCATCTTTGG | 59 |
| PDE4DIP | 213388_at | 0.28 | AATTATGAGTTTCTATCTGTGTCCA | 60 |
| CAP350 | 213956_at | 0.28 | GGGAAGTCCACATAGCGTCATTAAT | 61 |
| AIF1 | 215051_x_at | 0.28 | TTCAGCTACCCTGACTTTCTCAGGA | 62 |
| TRAF3 | 221571_at | 0.28 | GGCATGATGTCCGGTGATTTCTGTA | 63 |
| MBNL1 | 201152_s_at | 0.27 | ACTCTTGAGGGTTGATTATGCTGCA | 64 |
| FMNL1 | 204789_at | 0.27 | GGACCTCATCTCTGAGCTGAAACGG | 65 |
| TMEFF1 | 205122_at | 0.27 | GTTGGTGTTTAAAGATCTGAAGTGT | 66 |
| IL6R | 205945_at | 0.27 | GAAGCACCATAACTTTGTTTAGCCC | 67 |
| SIVA | 210792_x_at | 0.27 | ACAGCATGAGGCGGCCGGGGAGCTG | 68 |
| MCAM | 211340_s_at | 0.27 | GCTATGGTTATATTAGCACCAAACT | 69 |
| POLR2I | 212955_s_at | 0.27 | GGCCGACAACAGCTGCATCTATGTC | 70 |
| T3JAM | 213888_s_at | 0.27 | TGAAAAAGGGTTTCTATTCTCTCTG | 71 |
| C1orf24 | 217967_s_at | 0.27 | AGTATCAGTCGGTGCAACAGTTGGC | 72 |
| COL5A2 | 221730_at | 0.27 | TGAAGTTGATCCTGAGACTCTTGAA | 73 |
| LAPTM5 | 201720_s_at | 0.26 | TACTCAGAGGTGTGACCCTCGCCAG | 74 |
| JARID1A | 202040_s_at | 0.26 | GTCGTACTATCTTACTGAGCCACAG | 75 |
| CUGBP2 | 202156_s_at | 0.26 | AAGGCGTAACGAGTTCATCTTTCTT | 76 |
| PTPN7 | 204852_s_at | 0.26 | CCTTGATACCAGCTCTCTGTGGAAA | 77 |
| LCP2 | 205269_at | 0.26 | AAATCACTAAACCTCGTTTTCTCAG | 78 |
| RASA4 | 212706_at | 0.26 | AGCGTCCTTATCTTTCAGAGCTACA | 79 |
| FTL | 212788_x_at | 0.26 | AAACCCCAGACGCCATGAAAGCTGC | 80 |
| CD3D | 213539_at | 0.26 | GGGAACACTGCTCTCAGACATTACA | 81 |
| EIF4A1 | 214805_at | 0.26 | CTTTTTCCTGGGTCATGCTGCAACA | 82 |
| NKTR | 215338_s_at | 0.26 | GATGGGGTGCATGTAGTCTTTGGAC | 83 |
| C1orf24 | 217966_s_at | 0.26 | GAAGGTGTGATCTGTGGGACTGTCT | 84 |
| C2orf33 | 219137_s_at | 0.26 | GTACGTTTTTACTCAGTTCATGCGT | 85 |
| TMEM22 | 219569_s_at | 0.26 | GCTTCTCGTGCTGCACATATTTCCT | 86 |

(continued)

| Gene | Affymetrix ID | Correlation | Affymetrix Probe Sequence | SEQ ID NO: |
|---|---|---|---|---|
| GIMAP6 | 219777_ at | 0.26 | GTGAACAGACTTGAAACTCCAGAGC | 87 |
| RAP1B | 200833_s_at | 0.25 | ATCATTTTCAGGCTTCTGCAGCTGT | 88 |
| SRRM1 | 201225_s_at | 0.25 | GCATGTTGTTTGCCAGGACACTGTG | 89 |
| PWP1 | 201608_s_at | 0.25 | TTGTGCTTGCTCTTCAGATGGATGG | 90 |
| EDG1 | 204642_at | 0.25 | TAGCCAGGATCCTTGGTGTCCTAGG | 91 |
| CD47 | 211075_s_at | 0.25 | GCGGCGTGTATACCAATGCATGGCC | 92 |
| CG018 | 213375_s_at | 0.25 | GAATAACTTTTGGCTGTTGTGCTAA | 93 |
| TPK1 | 221218_s_at | 0.25 | TGGCCCGCGTGATTGTGGCATTTAA | 94 |
| COL5A2 | 221729_at | 0.25 | CATAACTGTTAGACTTCCCGTTTCT | 95 |

**Table 3.** mRNA biomarkers of resistance to cisplatin. Dashes mean that the Affymetrix probeset has not been mapped to a specific gene. Affymetrix IDs refer to the array type HG-U133A.

| Gene | Affymetrix ID | Correlation | Affymetrix Probe Sequence | SEQ ID NO: |
|---|---|---|---|---|
| SFN | 33323_r_at | -0.48 | TCAATAAAGTTCCCCTGTGACACTC | 96 |
| LISCH7 | 208190_s_at | -0.47 | CTCCCCTATGATGGGCGGCTACTGG | 97 |
| EPB41L4B | 220161_s_at | -0.47 | ATCAGTTGATTCTTGTGCCATTTTT | 98 |
| MST1R | 205455_at | -0.46 | TGAGCCAGTGAGGGCAGTCCTGCAA | 99 |
| ITGB4 | 204990_s_at | -0.45 | GCATCATCACCATAGAGTCCCAGGA | 100 |
| SFN | 209260_at | -0.45 | TCTTGCTCCAAAGGGCTCCGTGGAG | 101 |
| C20orf35 | 218094_s_at | -0.45 | ATACGCCCTTGGCACAGTCGGATGA | 102 |
| SFN | 33322_i_at | -0.45 | GTCTGCTGGGTGTGACCATGTTTCC | 103 |
| TACSTD1 | 201839_s_at | -0.43 | GTGCGTGGGACGAAGACATCTTTGA | 104 |
| C19orf21 | 212925_at | -0.42 | TGGTCCCCTTCACCTGGGAGAAAAG | 105 |
| KRT8 | 209008_x_at | -0.41 | GGGCCAAGCAGGACATGGCGCGGCA | 106 |
| JUP | 201015_s_at | -0.4 | AGCTTCAGACTCAAGTACCCATTCT | 107 |
| KRT18 | 201596_x_at | -0.4 | GAGCTGCTGAGACGACGCTCACAGA | 108 |
| FA2H | 219429_at | -0.39 | GAGAAGCAGTTTGACGGACCTTGTG | 109 |
| MGAT4B | 220189_s_at | -0.38 | GGTGATTCTGAGCGAGATCTTCCTG | 110 |
| DSG2 | 217901_at | -0.37 | GCAGCCTTGGAAACCTAACCTGCCT | 111 |
| LRP5 | 209468_at | -0.36 | CCTGCAGCACCGACGTGTGTGACAG | 112 |
| GJB3 | 215243_s_at | -0.36 | ACTTGGCTCAGTGGAAGCCCTCTTT | 113 |
| TACSTD2 | 202286_s_at | -0.35 | ACATTGCCCGGAAACTCAGTCTATT | 114 |
| LAD1 | 203287_at | -0.35 | GCTGTGGATCTGTTTGGCCAGGGTC | 115 |
| AGR2 | 209173_at | -0.35 | GTTAGAGCCGATATCACTGGAAGAT | 116 |
| HTATIP2 | 209448_at | -0.35 | AGATTTGTCAGCCCTATCTCAAACT | 117 |
| LOC57228 | 209679_s_at | -0.35 | AGGTCTTCCCAGAGGCTGGATACCA | 118 |

(continued)

| Gene | Affymetrix ID | Correlation | Affymetrix Probe Sequence | SEQ ID NO: |
|---|---|---|---|---|
| BCL2L1 | 212312_at | -0.35 | GTCTTCCCTACCTCAGGCAGGAAGG | 119 |
| GPX2 | 202831_at | -0.34 | CTACCCTTATGATGACCCATTTTCC | 120 |
| SOX9 | 202935_s_at | -0.34 | AAATGCTCTTATTTTTCCAACAGCT | 121 |
| TPBG | 203476_at | -0.34 | GTGTATAGTGTTTTACCCTCTTCTT | 122 |
| LGALS4 | 204272_at | -0.34 | TCATCAAGGGCTATGTGCCTCCCAC | 123 |
| PHLDA1 | 217996_at | -0.34 | CCCCGCACCAGATCAAGTAGTTTGG | 124 |
| PLEK2 | 218644_at | -0.34 | CCCTCCTACCAGATGACACAGACAA | 125 |
| TNFRSF21 | 218856_at | -0.34 | TGTATGGTTTTCACCTGGACACCGT | 126 |
| IER3 | 201631_s_at | -0.33 | AACTCCGTCTGTCTACTGTGTGAGA | 127 |
| RAI3 | 203108_at | -0.33 | CCCACTGGCCTGAATCTACACTGGA | 128 |
| BENE | 209373_at | -0.33 | ACATTACATCCGTGGATTCTCCTGC | 129 |
| MGC50853 | 212400_at | -0.33 | GGCCCTGGGCCAGGGTGATTGGACT | 130 |
| RAI3 | 212444_at | -0.33 | TTTAGCCCTCATGACTGTATTTTCT | 131 |
| CLIC3 | 219529_at | -0.33 | ACACGCTGCAGATCGAGGACTTTCT | 132 |
| CLDN3 | 203954_x_at | -0.32 | ACCGGCAGCCCTGGAAGGGGCACTT | 133 |
| FGFR4 | 204579_at | -0.32 | TACCAGCAGGAGGTTCTGGGCCTCT | 134 |
| PPARG | 208510_s_at | -0.32 | CATCTTTCAGGGCTGCCAGTTTCGC | 135 |
| FBP1 | 209696_at | -0.32 | GGGCTACGCCAAGGACTTTGACCCT | 136 |
| CPNE3 | 202119_s_at | -0.31 | AATCTAGTCACCTAACCTTGTGGTT | 137 |
| AREG | 205239_at | -0.31 | ATTTCAAAATTTCTGCATTCACGGA | 138 |
| VIL1 | 205506_at | -0.31 | AACACCTGTCCATTGAAGATTTCAC | 139 |
| GATA6 | 210002_at | -0.31 | GACATTCTTATGCTTCTTTTACAAC | 140 |
| TCF7L2 | 212761_at | -0.31 | AATGTTTCCTAACAGTTGTGATGTT | 141 |
| PP1201 | 217730_at | -0.31 | GGGTGAAGAGAGACTCGGTGCGGGC | 142 |
| FLJ20847 | 219053_s_at | -0.31 | CGACCGCCTGTATGTTTGTGTAATT | 143 |
| GPR172A | 222155_s_at | -0.31 | AAGGCCTATCAGCTTCTATCAGCCC | 144 |
| ITGA6 | 201656_at | -0.3 | GTCACTGGTCTGTTTGCATTTGATA | 145 |
| ZNF165 | 206683_at | -0.3 | AGCTCAAAACTTGCTAGGCATCAGA | 146 |
| FLNB | 208613_s_at | -0.3 | GCAGCAAAGCTGGCTCCAACATGCT | 147 |
| MCCC2 | 209623_at | -0.3 | AAACACTATCTACTTCCTTTGTCAT | 148 |
| FLJ20273 | 218035_s_at | -0.3 | GAGGATCATGCCCTTAGCAAGTACT | 149 |
| TMEM16A | 218804_at | -0.3 | AACATCATTTTAGCAAAGGCCAGGA | 150 |
| RAB11FIP1 | 219681_s_at | -0.3 | TGTCCTTGTTACATTGAGGTTAAGA | 151 |
| SLC3A2 | 200924_s_at | -0.29 | TCCCTACTGCATGGGGACTTCCACG | 152 |
| EFNA1 | 202023_at | -0.29 | CCACCTTCACCTCGGAGGGACGGAG | 153 |
| SORL1 | 203509_at | -0.29 | TAATTACACGTTCACCGTCCAAGCA | 154 |
| PLS1 | 205190_at | -0.29 | TTCCCTTTCTACCATTGATTTAAAT | 155 |

(continued)

| Gene | Affymetrix ID | Correlation | Affymetrix Probe Sequence | SEQ ID NO: |
|---|---|---|---|---|
| GALIG | 208949_s_at | -0.29 | AGTACTGGTTGAACCTGACCACTTC | 156 |
| EHD1 | 209038_s_at | -0.29 | AAATACATAAGCTAGTTTCTGTTCT | 157 |
| NR2F2 | 209120_at | -0.29 | GTAACGTGATTGATTCAGTATCTTA | 158 |
| SERPINB1 | 213572_s_at | -0.29 | AATACATCCGATGCGTAGATTCTTG | 159 |
| PCK2 | 202847_at | -0.28 | AGAATGCTCGGGTGCTAGACTGGAT | 160 |
| ARF6 | 203311_s_at | -0.28 | GGACGGACTCTATGAGGGGCTCACA | 161 |
| TGFA | 205016_at | -0.28 | GGAATGACTCAAATGCCCAAAACCA | 162 |
| CST6 | 206595_at | -0.28 | TCCTCTCAGCTCCTAAAGCACAACT | 163 |
| PXN | 211823_s_at | -0.28 | ACATGTTCGCACCCAAGTGTGGCGG | 164 |
| SORL1 | 212560_at | -0.28 | TTTCAGATGGAGTACCAGCACCGAA | 165 |
| SLC39A4 | 219215_s_at | -0.28 | TGGCACTCGCGGTTGGAGTCAGCGA | 166 |
| GCNT3 | 219508_at | -0.28 | GGCCATCTATGGGACTGAACTTTGA | 167 |
| S100A11 | 200660_at | -0.27 | GAAGAAACTGGACACCAACAGTGAT | 168 |
| ITPR3 | 201189_s_at | -0.27 | GCTGTAGCCAGTGCAGACCTCACTG | 169 |
| DHCR7 | 201790_s_at | -0.27 | AGGTGTCCAGTACCTAATCACGCTC | 170 |
| TCIRG1 | 204158_s_at | -0.27 | TTGCCGTGATGACCGTGGCTATCCT | 171 |
| NR2F2 | 209121_x_at | -0.27 | GAATACGTTAGGAGCCAGTACCCCA | 172 |
| SLC25A1 | 210010_s_at | -0.27 | GAAGCTGCTCAACAAAGTGTGGAAG | 173 |
| SERPINB6 | 211474_s_at | -0.27 | GGAATGTCCCAGACAGACCTGTCTC | 174 |
| ARTN | 216052_x_at | -0.27 | CCTTCATGGACGTCAACAGCACCTG | 175 |
| LOC51123 | 218059_at | -0.27 | GGCCCGGATATGGCTCGTGGACAGC | 176 |
| S100A14 | 218677_at | -0.27 | AGGAGTCTCCACCAGAGGGAGGCTC | 177 |
| FCGRT | 218831_s_at | -0.27 | GAGCACCACTACTGCTGCATTGTGC | 178 |
| RAB20 | 219622_at | -0.27 | ACTCTGACATTTCTTGTTCTCAAGC | 179 |
| SPDEF | 220192_x_at | -0.27 | CCAGCATTTCCAGAGCAGAGCCTAC | 180 |
| PNAS-4 | 221648_s_at | -0.27 | GCGTGTCTTGAGTTCCATGCAAATT | 181 |
| PXN | 201087_at | -0.26 | AATGGTGACAGTCCAAACCACTCCA | 182 |
| TPD52L2 | 201379_s_at | -0.26 | GGCCCTGCATGTCAGATGGCGTGGT | 183 |
| ALDH3A2 | 202054_s_at | -0.26 | TGATCATAAATTCTCCCCAACTATA | 184 |
| ARF6 | 203312_x_at | -0.26 | AAAGTTGCCAAGATGCTCCTTGTTG | 185 |
| GPA33 | 205929_at | -0.26 | GTCTCACCCAACTGCAGTTTACTAT | 186 |
| --- | 208540_x_at | -0.26 | GACGGAGTTCCTAAGCTTCATGAAT | 187 |
| FLNB | 208614_s_at | -0.26 | TCAGCCTGGGCAGTCTTACCAAAAT | 188 |
| TSPAN-1 | 209114_at | -0.26 | TGCTGTGGCTTCACCAACTATACGG | 189 |
| CDH17 | 209847_at | -0.26 | CCTTGACTCCTTTGGTATTTCACTG | 190 |
| SERPINB1 | 212268_at | -0.26 | ACAGCAGGCATCGCAACTTTCTGCA | 191 |
| LCN2 | 212531_at | -0.26 | CAAGAGCTACAATGTCACCTCCGTC | 192 |

(continued)

| Gene | Affymetrix ID | Correlation | Affymetrix Probe Sequence | SEQ ID NO: |
|---|---|---|---|---|
| KIAA0984 | 213913_s_at | -0.26 | GTTTGTCTCTTGTTGTTCTGAAGGA | 193 |
| ACSL5 | 218322_s_at | -0.26 | CTCTCTAGTTAGATATCTGACTTGG | 194 |
| MUC13 | 218687_s_at | -0.26 | TCCAGCCTCGGGGTGTAGGTTTCTG | 195 |
| FAM11B | 219253_at | -0.26 | ACTCGTCTCACGCCGTGTTTGAGAT | 196 |
| SH2D3A | 219513_s_at | -0.26 | GCCAGAGTTCAAATGTGACTCCACC | 197 |
| ANXA2 | 201590_x_at | -0.25 | CAAGCCCCTGTATTTTGCTGATCGG | 198 |
| TM4SF3 | 203824_at | -0.25 | AGACCACAGATATCTTCTAGACATA | 199 |
| NT5E | 203939_at | -0.25 | GTCACTGTAAATCATTCTTAAGCCC | 200 |
| TETRAN | 209215_at | -0.25 | AAGGCTGTCAGGGCTTCTGTTTGTT | 201 |
| CTBP2 | 210835_s_at | -0.25 | GTAGACACCTGCACGCATAGGATTG | 202 |
| SCD | 211708_s_at | -0.25 | TTGCCACTTTCTTGCGATATGCTGT | 203 |
| DNMBP | 212838_at | -0.25 | GCCATTCCAGAAGTAGCTTATCCTA | 204 |
| TMC5 | 219580_s_at | -0.25 | CCAATACCCCACCGTGATGACTTGA | 205 |

**Table 4** mRNA biomarkers of sensitivity to LiPlaCis. Dashes mean that the Affymetrix probeset has not been mapped to a specific gene. Affymetrix IDs refer to the array type HG-U133A.

| Gene | Affymetrix ID | Covariance | Affymetrix Probe Sequence | SEQ ID NO: |
|---|---|---|---|---|
| CALD1 | 212077_at | 10861321835689.1 | AATTCTCTGTTATCTTTACGAGGTA | 206 |
| COL6A2 | 209156_s_at | 8535698909744.43 | CACGAGAAGGACTATGACAGCCTGG | 207 |
| FERMT2 | 209210_s_at | 5291552917682.63 | TGATTTGCCACAATGTCCTTAACTC | 208 |
| BNIP3 | 201849_at | 5145685657339.48 | GCTGAAGGCACCTACTCAGTATCTT | 209 |
| RAB31 | 217762_s_at | 4734688539598.5 | AGACCTGGCACTTCAGTAACTCAGC | 210 |
| COL5A2 | 221730_at | 4647176466910.36 | GACTCTTGAAGTAATGGCTGATCCT | 211 |
| MPO | 203948_s_at | 4518211644157.6 | GGGACTTTGTCAACTGCAGTACACT | 212 |
| SRPX | 204955_at | 4340511505629.07 | CCTTTCTTTACTCCATCATGGCTGG | 213 |
| ARHGDIB | 201288_at | 4263392661779.67 | ATCACTAACAGGTCTTTGACTCAGG | 214 |
| TMEM47 | 209656_s_at | 4156685173988.01 | GAATTCATGGTATCCTGGTTATTTT | 215 |
| CSRP2 | 207030_s_at | 3960151282910.27 | AACTACTGTGAAATTCTACCAGCAT | 216 |
| DPYSL3 | 201431_s_at | 3876388962016.02 | GACACCTGAGCCTGGATTTTCACTC | 217 |
| HTRA1 | 201185_at | 3845854940391.73 | TCAAACGGCCGAAGTTGCCTCTTTT | 218 |
| SLC39A6 | 202088_at | 3547148987590.88 | ATACTAGGCCTGTCTGTGGCATTCT | 219 |
| LAT2 | 221581_s_at | 3545380986375.43 | GGATTTAGGATAAGCTGTCACCCAG | 220 |
| ENAH | 217820_s_at | 3385939870513.75 | GGTCAGCAACCTCTTTTGATTTTGT | 221 |
| RPS4Y1 | 201909_at | 3384951327956.31 | GACAGGTGAACATTTCCGCCTGGTC | 222 |
| DOCK2 | 213160_at | 3367491706976.35 | GATTCCTGAACTCAAGGTACCAGCA | 223 |
| COL1A1 | 202311_s_at | 3222960671378.67 | TGTTCCTTTTTGTTCAAAGTCTATT | 224 |

(continued)

| Gene | Affymetrix ID | Covariance | Affymetrix Probe Sequence | SEQ ID NO: |
|---|---|---|---|---|
| GMFG | 204220_at | 3013566458581.29 | AGGTGTTCGAAATCCGCACCACTGA | 225 |
| CYR61 | 201289_at | 2999506373414.97 | GTGGAGTTGATGACTTTCTGTTTTC | 226 |
| RHOB | 212099_at | 2978300392812.93 | TGCAGGTCATGCACACAGTTTTGAT | 227 |
| CORO1A | 209083_at | 2968352455386.15 | GCTCCAGAAGCGCTTGGACAGGCTG | 228 |
| ID4 | 209291_at | 2948241975028.96 | GGCATAATGGCAAATCCTTCAAGCA | 229 |
| RARRES2 | 209496_at | 2907180844659.6 | CCCCATAGAGACCCAAGTTCTGCGG | 230 |
| SOX4 | 201417_at | 2862450307972.36 | GTAAACCACATCTTTTTTCGACTTT | 231 |
| NID1 | 202007_at | 2798544570884.12 | CACTTTTTGTATTTATCGTTTCATA | 232 |
| CALD1 | 201616_s_at | 2776573094080.12 | GACGCAGGACGAGCTCAGTTGTAGA | 233 |
| SERPINE2 | 212190_at | 2767126943194.04 | TGTTGTGCAGTGTGCCTGTCACTAC | 234 |
| CTSL1 | 202087_s_at | 2681524741399.96 | CACTTACTGACTTTGCATTTTCGTT | 235 |
| C3orf14 | 219288_at | 2679480387909.32 | GGTGGTTTCTCTTGAGACTCGTTAC | 236 |
| DKK3 | 202196_s_at | 2608335983440.84 | TTGGCAGTTGCATTAGTAACTTTGA | 237 |
| SCRN1 | 201462_at | 2582074623391.62 | TCATGTGCACATGCCGTTGCAGCAC | 238 |
| MT1M | 217546_at | 2555792977629.17 | CGTTGGAGAACTGCAGCTGCTGTGC | 239 |
| PLAU | 205479_s_at | 2529115320523.6 | AGCAGCTGAGGTCTCTTGAGGGAGC | 240 |
| NREP | 201310_s_at | 2514590941976.06 | CATTGGCCTGAGTTTCTTGTGCATT | 241 |
| HLA-B | 208729_x_at | 2501423496784.03 | GAGCCTACCTGGAGGGCGAGTGCGT | 242 |
| PTPRC | 212588_at | 2494855639496.51 | GTTTTCAATTTTGCATGCTCGATTA | 243 |
| HDGFRP3 | 209524_at | 2438222715080.89 | TTATGTGTACATTATTGTTGCTATT | 244 |
| CELF2 | 202157_s_at | 2427790438608.2 | CTTCCCGGTCACTGGTAACAATAGC | 245 |
| SFRP1 | 202037_s_at | 2413217767593.8 | GTACCTGTGGGTTAGCATCAAGTTC | 246 |
| HLA-B | 211911_x_at | 2358346288074.42 | CTGAGAGCCTACCTGGAGGGCCTGT | 247 |
| LOX | 215446_s_at | 2354236167712.24 | TTGGGCCTTTTATCTGTCTATCCAT | 248 |
| CLU | 208791_at | 2341547177698.15 | CAGTGTGACAAGTGCCGGGAGATCT | 249 |
| SH3BGRL | 201312_s_at | 2249866543302.91 | AGAATCTTTTCTATGCCTCTATTCC | 250 |
| INHBA | 210511_s_at | 2238550007854.02 | GCCATATAGCAGGCACGTCCGGGTC | 251 |
| MMP1 | 204475_at | 2203074303300.14 | GGCAAGGGATAACTCTTCTAACACA | 252 |
| WIPF1 | 202664_at | 2194537285288.12 | TTCCCTCCTTATAGTCAAGGACCGT | 253 |
| ADAMTS1 | 222162_s_at | 2144423953975.08 | AATAACGCAAATGGCTTCCTCTTTC | 254 |
| THY1 | 208850_s_at | 2141423198789.74 | GGCCTAGCACGGACATGGTCTGTCC | 255 |
| UCHL1 | 201387_s_at | 2140899985376.98 | TGATGGACGAATGCCTTTTCCGGTG | 256 |
| MYH10 | 212372_at | 2139390916542.17 | GATCCTCTGCAATGTGCTTGAAAAC | 257 |
| TYMS | 202589_at | 2131876162229.91 | TCACAAGCTATTCCCTCAAATCTGA | 258 |
| HCLS1 | 202957_at | 2089924252642.24 | TGATGAGCTTTCCTTTGATCCGGAC | 259 |
| HLA-B | 209140_x_at | 2085546519988.6 | GAGACAGCTGTCTTGTGAGGGACTG | 260 |
| IFI16 | 208966_x_at | 2061722348570.95 | TACACACCACCATATATACTAGCTG | 261 |

(continued)

| Gene | Affymetrix ID | Covariance | Affymetrix Probe Sequence | SEQ ID NO: |
|---|---|---|---|---|
| PRKCB | 207957_s_at | 2037662863122.06 | GTGTAGGTGAATGCAAACTCCATCG | 262 |
| BNIP3 | 201848_s_at | 2008580245730.46 | TTCCTCTTTAAACACCCGAAGCGCA | 263 |
| TUSC3 | 213423_x_at | 1987545095813.27 | AACTGTTCCTGACTTTATACTATTT | 264 |
| WNT5A | 205990_s_at | 1982235386738.35 | GCATAATGATATTCACATCCCCTCA | 265 |
| CALD1 | 201617_x_at | 1981280027254.5 | TGTTGTTTCTGCACTTTATAATAAA | 266 |
| HLA-C | 216526_x_at | 1955999731784.71 | AGAGGTGGGGCTGGATGTCTCCATC | 267 |
| IL1R1 | 202948_at | 1955342562611.76 | AAGTGCAAAGTTATTCCCCATCTTC | 268 |
| AUTS2 | 212599_at | 1927738178390.84 | TACTTACACCCAAACAGATCCTGAA | 269 |
| THBS2 | 203083_at | 1912997768879.9 | TTGCGTGTGGAGCTGTATTCCCGAG | 270 |
| CHRDL1 | 209763_at | 1895325557387.3 | CCCTTTCACTGTTCTCACAGGACAT | 271 |
| SFPQ | 214016_s_at | 1886539698542.15 | GTTGGCTGATATTGGAGTGCTCATT | 272 |
| CXCL12 | 209687_at | 1857308403453.12 | CAGCAGGGTTTCAGGTTCCAATCAG | 273 |
| HOXC6 | 206858_s_at | 1831591158444.48 | CTGTATTTGTGGTCTCTGTATTTAT | 274 |
| PLAGL1 | 209318_x_at | 1827870818957.99 | ACATCCAAAATGACGGCTGCTATAT | 275 |
| RDX | 212397_at | 1815278384492.07 | GTGGACCCTACTATTCATGTTTTGA | 276 |
| HNRNPH1 | 213619_at | 1813815711802.08 | GCTTAAACTTACGTGCCTTACAGGT | 277 |
| KRAS | 214352_s_at | 1802923545775.42 | CATGCAGACTGTTAGCTTTTACCTT | 278 |
| IL8 | 211506_s_at | 1788698391848.43 | GTCAGTGCATAAAGACATACTCCAA | 279 |
| FAM46A | 221766_s_at | 1787987145165.06 | GGAGTCCTATTTGCAGAACCACTTT | 280 |
| QKI | 212265_at | 1787672566876.18 | ATAACCAACCTATTGCCTATGAGAA | 281 |
| CD53 | 203416_at | 1777870731216.97 | CGAATTAGTCTCCAGCCTCTAAATA | 282 |
| LAPTM5 | 201720_s_at | 1763708973603.65 | TCGGGTCTCTCCATAATTCAGCCCA | 283 |
| FOXG1 | 206018_at | 1752375753099.1 | ACGATTGCCTTCAGTTTGTGTTGTG | 284 |
| MST4 | 218499_at | 1732353014841.79 | AATTCTTTTTATTGGTGCCTATATT | 285 |
| GAPDH GAPDH | AFFX-HUMGAPDH/M33197_M_at | 1692594771893.01 | AAGCTCACTGGCATGGCCTTCCGTG | 286 |
| TUBB2B | 214023_x_at | 1672014039622.35 | GAGATATTTCTGAATTACTGTTGTA | 287 |
| GAPDH | 212581_x_at | 1649610188507.54 | TTTGACGCTGGGGCTGGCATTGCCC | 288 |
| CEBPD | 203973_s_at | 1623762464226.23 | GGACAGCAGACTGCCGGTAACGCGC | 289 |
| PLAU | 211668_s_at | 1604895332856.59 | GCTCTGAAGTCACCACCAAAATGCT | 290 |
| CAV1 | 203065_s_at | 1604187716818.41 | GGTGCCAATTTCAAGTTCCAAGTTG | 291 |
| GAPDH GAPDH | AFFX-HUMGAPDH/M33197_3_at | 1601834913853.31 | TAGGGAGCCGCACCTTGTCATGTAC | 292 |
| - | 213158_at | 1597303398144.17 | ACGTATATTTACCTGTGACTTGTAT | 293 |
| ARHGEF6 | 209539_at | 1586970619512.16 | TAAACTGCTGCCCGTAGAGGCCTTT | 294 |
| PRKCB | 209685_s_at | 1580850725622.13 | TGGATGTTAGCGGTACTCTTCCACT | 295 |

(continued)

| Gene | Affymetrix ID | Covariance | Affymetrix Probe Sequence | SEQ ID NO: |
|---|---|---|---|---|
| SRGN | 201859_at | 1549790579490.15 | TTTTCCTGGATATCTGTGTATTTTC | 296 |
| TLE4 | 204872_at | 1549011037374.17 | ACTGTGCGTTGTACATAGTTCTAAT | 297 |
| LOC100506558 MATN2 | 202350_s_at | 1544181853329.71 | GAACACTGGCCATAGGAAATGCTGT | 298 |
| BHLHE40 | 201170_s_at | 1537151135133.25 | GATCCTTTCTGTAGGCTAATTCCTC | 299 |
| SGCE | 204688_at | 1519398433064.38 | AACGCAGCAGAACTTGCCACATCAG | 300 |
| --- | 222288_at | 1511518722955.02 | GAAGCTTGGCTTTAGTGGTAGAATG | 301 |
| PCBP2 | 204031_s_at | 1507948521040.68 | AGCCTGGCTCAATATCTAATCAATG | 302 |
| TFAP2A | 204653_at | 1493277682055.65 | GAACTTCAAACATTTGGGACCACCT | 303 |
| SPON1 | 209436_at | 1472949317341.51 | CCACCCTAGTGTCTCATGTTTGTAT | 304 |
| COL4A2 | 211966_at | 1468135692764.19 | TGGTGATGTCTGCTACTATGCCAGC | 305 |

**Table 5.** mRNA biomarkers of resistance to LiPlaCis. Dashes mean that the Affymetrix probeset has not been mapped to a specific gene. Affymetrix IDs refer to the array type HG-U133A.

| Gene | Affymetrix ID | Covariance | Affymetrix Probe Sequence | SEQ ID NO: |
|---|---|---|---|---|
| KRT18 | 201596_x_at | -22426211704708.5 | AAGCTGGAGGCTGAGATCGCCACCT | 306 |
| LGALS3 | 208949_s_at | -11456296973610.8 | CACTTTAACCCACGCTTCAATGAGA | 307 |
| DSP | 200606_at | -10269594517738.5 | TGGAATGAGTCTCCTTTAGTTTCAG | 308 |
| IGFBP4 | 201508_at | -8435796702432.14 | AGAGACATGTACCTTGACCATCGTC | 309 |
| SPINT2 | 210715_s_at | -8294729535462.05 | TGGAAATCCTCTAGGAGGCTCCTCC | 310 |
| CDH1 | 201131_s_at | -7786548077136.61 | TGTGTGGGTGCTGATAATTGTGTAT | 311 |
| DSG2 | 217901_at | -7061991934030.4 | TACTCTTCCATCATCTAGAATTGTT | 312 |
| RAB25 | 218186_at | -6195270978776.59 | GCACCCTCAGGGTCTTAAGGTCTTC | 313 |
| PTPRF | 200636_s_at | -6131832886305.69 | GTACACAGTCTGTTTTCTATTTGTT | 314 |
| SOX9 | 202936_s_at | -5835576205162.92 | TGGGCTGCCTTATATTGTGTGTGTG | 315 |
| LYZ | 213975_s_at | -5458342909996.32 | TAACCCAGACTTAATCTTGAATGAT | 316 |
| IER3 | 201631_s_at | -5365171123958.73 | GAGACTTCGGCGGACCATTAGGAAT | 317 |
| PERP | 217744_s_at | -5097068499548.16 | ATGCACGTGAAACTTAACACTTTAT | 318 |
| SOX9 | 202935_s_at | -5050052756141.07 | AGTTGAACAGTGTGCCCTAGCTTTT | 319 |

(continued)

| Gene | Affymetrix ID | Covariance | Affymetrix Probe Sequence | SEQ ID NO: |
|---|---|---|---|---|
| ATP1B1 | 201243_s_at | -4753436553865.35 | GATCTTGTATTCAGTCAGGTTAAAA | 320 |
| IFI27 | 202411_at | -4636709898452.9 | CCAAAGTGGTCAGGGTGGCCTCTGG | 321 |
| PHLDA2 | 209803_s_at | -4623467982538.76 | GGACGAGTCGGACCGAGGCTAGGAC | 322 |
| CTTN | 201059_at | -4563342040423.69 | ATTTGTGGCCACTCACTTTGTAGGA | 323 |
| SFN | 209260_at | -4455761701170.73 | TCTTGCTCCAAAGGGCTCCGTGGAG | 324 |
| MALL | 209373_at | -4327230558082.54 | CTCCTCCATGAGTCTGACATCTCGG | 325 |
| S100A11 | 200660_at | -4322815561525.15 | GGTTGAGGAGAGGCTCCAGACCCGC | 326 |
| TSPAN13 | 217979_at | -4261036366041.2 | ACAGCAACTTGTCAAACCTAAGCAT | 327 |
| AKR1C3 | 209160_at | -4207721689216.25 | ACGCAGAGGACGTCTCTATGCCGGT | 328 |
| FAT1 | 201579_at | -4082641838983.11 | GTAGTCATTCATTTCTAGCTGTACA | 329 |
| DSTN | 201021_s_at | -4020978397283.39 | GTAGCTGATGAAGTATGTCGCATTT | 330 |
| EFEMP1 | 201842_s_at | -3992766849062.55 | GATGATCTTCTGTGGTGCTTAAGGA | 331 |
| TFF3 | 204623_at | -3853023482644 | CTGTGATTGCTGCCAGGCACTGTTC | 332 |
| HSPB1 | 201841_s_at | -3835026328384.26 | TTCACGCGGAAATACACGCTGCCCC | 333 |
| SDC1 | 201286_at | -3731984524505.92 | TCATCTGCTGGTCCGTGGGACGGTG | 334 |
| PLAC8 | 219014_at | -3720610591317.68 | GAAGGAGAGCCATGCGTACTTTCTA | 335 |
| TPBG | 203476_at | -3655713541808.07 | GTGTATAGTGTTTTACCCTCTTCTT | 336 |
| LCN2 | 212531_at | -3340240709988.96 | CAGGACTTTTGTTCCAGGTTGCCAG | 337 |
| CEACAM6 | 203757_s_at | -3279054777343.26 | GTGCAGTTTCTGACACTTGTTGTTG | 338 |
| ELF3 | 210827_s_at | -3241469160886.13 | GGGAGCACCGTGATGGAGAGGACAG | 339 |
| CLDN3 | 203953_s_at | -3192796314939.69 | AAGGCCAAGATCACCATCGTGGCAG | 340 |
| TPD52L1 | 203786_s_at | -3049121447681.89 | TATTCAAATGGCCCCTCCAGAAAGT | 341 |
| VAMP8 | 202546_at | -2969692217517 | AAGCCACATCTGAGCACTTCAAGAC | 342 |
| C1orf106 | 219010_at | -2931724791122.81 | GTTCCAAGAACTCTGGTGTCTGACC | 343 |
| RBM47 | 218035_s_at | -2891974033193.95 | GAGGATCATGCCCTTAGCAAGTACT | 344 |
| C3 | 217767_at | -2846605120573.62 | GGTCTACGCCTATTACAACCTGGAG | 345 |
| CAPN2 | 208683_at | -2829130992700.86 | AATCGTTCTCCTTACAATCAAGTTC | 346 |
| ERBB3 | 202454_s_at | -2788407249074.31 | GGAACTAGGCTCTTATGTGTGCCTT | 347 |

(continued)

| Gene | Affymetrix ID | Covariance | Affymetrix Probe Sequence | SEQ ID NO: |
|---|---|---|---|---|
| SLPI | 203021_at | -2755718313124.09 | TCTGTCCTCCTAAGAAATCTGCCCA | 348 |
| SPATS2L | 222154_s_at | -2729322838596.83 | GAGGCTCAGTTAGCAACCTGTGTTG | 349 |
| ERBB2 | 216836_s_at | -2698032874395.93 | AGACTGTCCCTGAAACCTAGTACTG | 350 |
| SERPINB1 | 212268_at | -2694341115802.62 | ACTTTCTGCATGTTGATGCCCGAAG | 351 |
| CEACAM6 | 211657_at | -2643169692661.57 | GTTCTTGTATTGTATTGCCCAGGGG | 352 |
| AKR1B10 | 206561_s_at | -2617913243059.4 | AAAAACCGCAGCCCAGGTTCTGATC | 353 |
| ID1 | 208937_s_at | -2607302720347.48 | GACATGAACGGCTGTTACTCACGCC | 354 |
| PPAP2C | 209529_at | -2576535604785.95 | TGTTCTTGGCGCTGTATGTGCAGGC | 355 |
| AQP3 | 39248_at | -2561344001860.94 | CTTCTACAGGCTTTTGGGAAGTAGG | 356 |
| PODXL | 201578_at | -2559443301040.98 | TGGAGGACACAGATGACTCTTTGGT | 357 |
| PRR15L | 219127_at | -2483388299723.69 | GAGTGGGTGGGGAATTTTCTCCTCT | 358 |
| EMP2 | 204975_at | -2470436470609.79 | CTGCACCTTCATCAGCGGCATGATG | 359 |
| MYO10 | 201976_s_at | -2463058577194.03 | TATAAACCACTCTTCAACAGCTGGC | 360 |
| SERPINB1 | 213572_s_at | -2374385129062.88 | AATACATCCGATGCGTAGATTCTTG | 361 |
| SDC4 | 202071_at | -2371552687950.61 | TGGCTTAGCCTGGGCAGGTCGTGTC | 362 |
| CRABP2 | 202575_at | -2354608471952.81 | GAGCAGGGTCTCTCTAAAGGGGACT | 363 |
| HTATIP2 | 209448_at | -2354028532889.45 | GTCTCTGAGTTACAAAAGTGCTAAT | 364 |
| DBNDD2 SYS1 SYS1-DBNDD2 | 218094_s_at | -2352744142308.53 | ACCAGTTTTTGGCTTACTCCTGAGA | 365 |
| ESRP1 | 219121_s_at | -2312028194710.22 | TTGTCTACACTCAGGCTGCAGTATT | 366 |
| HSD17B11 | 217989_at | -2304068718020.79 | TCCTGAGAGATACCTCACATTCCAA | 367 |
| GFPT1 | 202722_s_at | -2272343431090.56 | GGTTAGCCTTAGTTTCTCAGACTTG | 368 |
| S100A14 | 218677_at | -2240432231078.46 | TGTCCTCATCTCTGCAAAGTTCAGC | 369 |
| IGFBP7 | 201162_at | -2225724813680 | TTCCCAAGGACAGGCTTCAGCATCA | 370 |
| PTPRF | 200637_s_at | -2190473907894.45 | CTCCTACGCAGATGCTGTCACTGGC | 371 |
| HMGA1 | 206074_s_at | -2178312788057.87 | TGAGCAAGGGGGCCCGAATCGACCA | 372 |
| YWHAZ | 200641_s_at | -2145016988259.93 | AAGCCTGCTCTCTTGCAAAGACAGC | 373 |
| SCD | 200832_s_at | -2143962895648.8 | TAACTATAAGGTGCCTCAGTTTTCC | 374 |

(continued)

| Gene | Affymetrix ID | Covariance | Affymetrix Probe Sequence | SEQ ID NO: |
|---|---|---|---|---|
| SH3YL1 | 204019_s_at | -2139236372988.65 | CATATGGCATCTCTCAACTTTTCTT | 375 |
| UCP2 | 208998_at | -2139031352031.13 | GAAAGTTCAGCCAGAATCTTCGTCC | 376 |
| F3 | 204363_at | -2113802654784.93 | GGGCAGCTTCCTAATATGCTTTACA | 377 |
| AZGP1 | 209309_at | -2089576575474.55 | GCCTGTCTTGAGTAGACTTGGACCC | 378 |
| LIMCH1 | 212327_at | -2089195209441.08 | GATCCACCTCATATGTGAGTCCGTC | 379 |
| PLA2G2A | 203649_s_at | -2069037053701.26 | CGCTGCTGTGTCACTCATGACTGTT | 380 |
| ITGB5 | 201125_s_at | -2028321449243.62 | GCCTGTTGAAGGTACATCGTTTGCA | 381 |
| ABCC3 | 208161_s_at | -2007168680009.07 | TCTCCCGATTCCCAACTGAGTGTTA | 382 |
| DDR1 MIR4640 | 207169_x_at | -2000582844983.07 | AGGCAATTTTAATCCCCTGCACTAG | 383 |
| GATA3 | 209604_s_at | -1995114130212.84 | GGACAAACTGCCAGTTTTGTTTCCT | 384 |
| CYB561 | 209163_at | -1981172434786.63 | GTTCTTCAATCAGCTGGCACACACT | 385 |
| C10orf116 | 203571_s_at | -1962923571527.29 | ACCACCCAGGAAACCATCGACAAGA | 386 |
| PTPRF | 200635_s_at | -1924144465806.05 | AAGGACAGAACATTGCCTTCCTCGT | 387 |
| DKK1 | 204602_at | -1893211415469.31 | GGATATACAAGTTCTGTGGTTTCAG | 388 |
| SERPINB5 | 204855_at | -1863934443254.52 | GTGGTTGGCACTAGACTGGTGGCAG | 389 |
| ARHGAP29 | 203910_at | -1818117319379.63 | ATGTACTTGTTCTACCTGGATTGTC | 390 |
| GAS6 | 202177_at | -1817533234900.07 | CGCGGCTGCATGACACTGGAGGTCA | 391 |
| LAMB3 | 209270_at | -1817170377879.96 | GGTGCCCGGATCCAGAGTGTGAAGA | 392 |
| KLF5 | 209212_s_at | -1814910338390.4 | CTCCATCCTATGCTGCTACAATTGC | 393 |
| MAOA | 212741_at | -1811716715860.48 | TGAATGCCAGTCCAGATGTGCCTAG | 394 |
| NET1 | 201830_s_at | -1789348130490.25 | TTACATTCATTTAACCTGCCGATTA | 395 |
| CYBA | 203028_s_at | -1775049034494.02 | CACCCAGTGGTACTTTGGTGCCTAC | 396 |
| TGM2 | 201042_at | -1772139742186.19 | AGTGCTGGTCACTAACCAACAAGGT | 397 |
| ALDH2 | 201425_at | -1757839520621.92 | CTCTCTGAAACGCTTCCTATAACTC | 398 |
| HSPA1A HSPA1B | 200799_at | -1730673434053.48 | TTGTCAGTTCTCAATTTCCTGTGTT | 399 |
| JUP | 201015_s_at | -1729139912998.84 | ATTATCGCTTTATGTTTTTGGTTAT | 400 |
| HSPA1A HSPA1B | 200800_s_at | -1722098969341.57 | GGGGCTCAAGGGCAAGATCAGCGA G | 401 |

(continued)

| Gene | Affymetrix ID | Covariance | Affymetrix Probe Sequence | SEQ ID NO: |
|---|---|---|---|---|
| F11R | 221664_s_at | -1642391094616.93 | GAATAGGTATCTTGAGCTTGGTTCT | 402 |
| HBG1 HBG2 LOC100653006 LOC100653319 | 204419_x_at | -1595966820539.76 | ACACTCGCTTCTGGAACGTCTGAGG | 403 |
| KLF4 | 221841_s_at | -1553919884310.19 | AATCTATATTTGTCTTCCGATCAAC | 404 |
| CA12 | 214164_x_at | -1551710888005.42 | ACAAGGCCCAGGCTGGGGCCAGGGC | 405 |

*Kits*

Kits of the invention can be used for determining the responsiveness of a cancer patient (e.g., a patient having a solid tumor cancer, such as breast cancer, or a hematological cancer, such as lymphoma (e.g., CTCL) to liposomal formulation of cisplatin (e.g., LiPlaCis). Kits of the invention can include reagents and/or materials for, e.g., collecting and/or purifying nucleic acids from biological samples (such as those obtained from a patient to be treated with a target drug(s) of the invention), reagents for amplifying such nucleic acids to produce an amplified sample, and/or at least one device of the invention. Reagents for amplifying nucleic acids may include, e.g., PCR reagents, including but not limited to DNA polymerase, RNA polymerase, PCR buffer, magnesium chloride solutions, nucleic acid primers (e.g., primers designed to target particular biomarkers of responsiveness to a target drug(s) of interest), and/or any other PCR reagents as are well known in the art. In particular, kits useful in the method may include one or more of the following: a kit for RNA extraction from tumors (e.g., Trizol for mRNA, mirVana miRNA isolation kit from Ambion Inc), a kit for RNA labeling (e.g., MessageAmp from Ambion Inc., FlashTag from Genisphere Inc), a microarray for measuring biomarker expression (e.g., HG-U133A, HG-U133_Plus2 or miRNA-1.0 from Affymetrix Inc), a microarray hybridization station and scanner (e.g., GeneChip System 3000Dx from Affymetrix Inc), and/or software for analyzing the expression of biomarker genes or RNAs (e.g., miRNAs) as described in herein (e.g., implemented in R from R-Project or S-Plus from Insightful Corp.).

For example, a kit of the invention can include one or more probes capable of detecting one or more biomarkers of Tables 2-5 (e.g., the kit may include probes for the biomarkers of Tables 2-5). Such probes can, for example, include nucleic acids capable of hybridizing to the biomarker based on nucleic acid sequence complementarity. In particular, a probe has at least 85% sequence identity (e.g., 85%, 90%, 95%, 97%, 98%, 99%, or 100% sequence identity) to a nucleic acid sequence that is complementary or identical to at least 5 (e.g., at least 15) consecutive nucleotides of one or more biomarkers. The probes can be attached to a solid surface, such as a microarray. The kit may include NanoString capture probes, NanoString reporter probes, and/or one or more nCounter cartridges. The kit may include reagents for next generation sequencing, including but not limited to poly(T) oligonucleotides, dye terminators, sequencing adapters, adapter ligation reagents, reverse transcriptase, primers (e.g., random primers), DNA-cleaving enzymes, polymerases, and/or any combination thereof. The kit may also be one that includes a protein array and/or reagents for detection of the polypeptide product(s) of one or more biomarkers of Tables 2-5.

The following examples are intended to illustrate, rather than limit, the invention.

**EXAMPLES**

**Example 1. Identification of biomarkers of sensitivity and resistance to cisplatin using Affymetrix HG-U133A arrays.**

A key component of LiPlaCis is cisplatin, a common cancer drug that is encapsulated in a liposomal formulation. It is obvious that LiPlaCis will not work on a tumor if cisplatin does not work. Thus is it possible to predict part of the response to LiPlaCis as the response to cisplatin. The liposomal delivery to the tumor cell is a separate part of the requirement for LiPlaCis to work, and can be modeled separately, e.g. by measuring sPLA2 on the surface of tumor cells.

DNA chip measurements of the 60 cancer cell lines of the NCI60 data set were performed using Affymetrix HG-U133A arrays and logit normalized. For each array, the logit transformation was performed followed by a Z-transformation to mean zero and SD 1, and correlated to growth inhibition (log(GI50)). Growth inhibition data of LiPlaCis against the same cell lines were downloaded from the National Cancer Institute. Each gene's expression in each cell line was correlated to the growth of those cell lines (log(GI50)) in the presence of LiPlaCis. The Pearson correlation coefficient was then determined to identify genes positively and negatively correlated to sensitivity to LiPlaCis. Tables 2 and 3 show the top positively correlated genes (the biomarkers of sensitivity) and negatively correlated genes (the biomarkers of resistance), respectively, using the Affymetrix HG-U133A arrays.

**Example 2. Identification of biomarkers of sensitivity and resistance to LiPlaCis using Affymetrix HG-U133A arrays.**

DNA chip measurements of the 60 cancer cell lines of the NCI60 data set were also performed using HG-U133_Plus_2 arrays and logit normalized. For each array, the logit transformation was performed followed by a Z-transformation to mean zero and SD 1, and correlated to growth inhibition (log(GI50)). Growth inhibition data of LiPlaCis against the same cell lines were downloaded from the National Cancer Institute. Each gene's expression in each cell line was correlated to the growth of those cell lines (log(GI50)) in the presence of LiPlaCis. The covariance (Pearson correlation coefficient multiplied by standard deviation) was then determined to identify genes positively and negatively correlated to sensitivity to LiPlaCis. Tables 4 and 5 show the top positively correlated genes (the biomarkers of sensitivity) and negatively correlated genes (the biomarkers of resistance), respectively, using the Affymetrix HG-U133A arrays.

**Example 3. Predicting responsiveness to LiPlaCis in various cancer patient populations.**

An mRNA-based predictor of responsiveness to LiPlaCis developed according to the methods of the invention was applied to 3,522 patients having a variety of cancers. Each patient had a pre-treatment measurement of gene expression with an Affymetrix array. The predicted LiPlaCis sensitivity of each patient was calculated as the difference between the mean of the expression levels of the biomarkers of sensitivity (Table 2) and the mean of the expression levels of the biomarkers of resistance (Table 3) for the patient. When the patients were grouped by cancer types, and cancer types predicted to be more responsive to LiPlaCis were identified (Figure 1).

Of 27 different cancer types, solid tumor cancers were predicted to be more responsive to LiPlaCis treatment than hematological cancers. In particular, patients with hematological cancer types were predicted to be responsive to LiPlaCis treatment.

The median of the boxplots shown in Figure 1 is a cutoff that may be used to separate patients predicted to be responsive to LiPlaCis treatment from patients predicted to be non-responsive to LiPlaCis treatment for a given cancer type. Values above the median indicate patients predicted to be responsive to LiPlaCis, while values below the median indicate patients predicted to be non-responsive to LiPlaCis. For a test sample from an individual patient, it is useful to compare the test sample to the reference population for the same cancer type. If the test sample is above the median for the reference population of the same cancer type, then the patient is predicted to be responsive to LiPlaCis treatment. If the test sample is below the median for the reference population of the same cancer type, then the patient is predicted to be non-responsive to LiPlaCis treatment. This method for predicting patient responsiveness can also be used when the reference cancer population consists of only two patients: a patient responsive to LiPlaCis treatment and a patient non-responsive to LiPlaCis treatment.

**Example 4. Determining the expression of secreted phospholipase A2.**

In addition to determining the responsiveness to cisplatin or LiPlaCis using the genes in Tables 2-5, it is also possible to test for the presence of secreted phospholipase A2 (sPLA2-IIA) in the tumor tissue. sPLA2 is required for degradation of the liposomes that deliver the cisplatin to the tumor cell, and can be measured using standard immunocytochemistry techniques with a monoclonal antibody against sPLA2-IIA, e.g. Clone SCACC353 from Cayman Chemical. Any staining in this assay indicates the presence of sPLA2 and suggests susceptibility to LiPlaCis. Alternatively, the expression of sPLA2-IIA can be detected on the microarray as PLA2G2A (SEQ ID NO: 380). While in cancer cell lines growing in vitro there is a negative covariance between PLA2G2A expression and LiPlaCis response, in tissue there is a positive correlation between sPLA2A mRNA and immunohistochemistry (Mirtti et al APMIS 2009, 117: 151-161).

**Example 5. Predicting responsiveness of breast cancer patients to LiPlaCis.**

The diagnostic methods of the present invention can be used to predict the responsiveness of a breast cancer patient to treatment with LiPlaCis. In particular, the breast cancer patient may be one that has not previously received any cancer treatment or one that has received a cancer treatment other than LiPlaCis. Moreover, the patient may be one diagnosed with breast cancer or one with recurrence of prostate cancer.

A biological sample (e.g., a breast cancer tissue sample) may be obtained from the patient through methods well known in the art. The sample may be frozen and/or prepared, e.g., by formalin fixation and paraffin embedding. In particular, mRNA can be isolated from the sample and a gene expression profile can be determined, e.g., using a microarray platform, such as the Affymetrix HG-U133A or HG-U133_Plus_2 array, for one or more of the biomarkers shown in Tables 2-5. One or more of the biomarkers shown in Tables 2-5 can also be measured, e.g., by sequencing or PCR-based techniques, such as those described herein.

For example, the expression level of one or more biomarkers of sensitivity to LiPlaCis can be determined in the sample from the patient, such as one or more of COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), CBFB (SEQ ID NO: 15). In particular, the biomarker is COL5A2 (SEQ ID NO 73 or 211). The expression level of one or more biomarkers of resistance to LiPlaCis can also be determined in the sample from the patient, such as one or more of SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), LRP5 (SEQ ID NO: 112). In particular, the biomarker is SFN (SEQ ID NO: 96 or 324).

The breast cancer patient may be responsive to LiPlaCis if the expression level of one or more of the biomarkers of sensitivity is substantially similar to the expression level of the biomarkers of sensitivity in a cell or tissue known to be sensitive to LiPlaCis. The breast cancer patient may also be responsive to LiPlaCis if the expression level of one or more of the biomarkers of resistance is substantially dissimilar to the expression level of the biomarkers of resistance in a cell or tissue known to be resistant to LiPlaCis.

In addition to determining the responsiveness to cisplatin or LiPlaCis using the genes in Tables 2-5, it is also possible to test for the presence of secreted phospholipase A2 (sPLA2-IIA) in the tumor tissue. sPLA2 is required for degradation of the liposomes that deliver the cisplatin to the tumor cell, and can be measured using standard immunocytochemistry techniques with a monoclonal antibody against sPLA-IIA, e.g. Clone SCACC353 from Cayman Chemical. Any staining in this assay indicates the presence of sPLA2 and suggests susceptibility to LiPlaCis. Alternatively, the expression of sPLA2-IIA can be detected on the microarray as PLA2G2A (SEQ ID NO: 380). While in cancer cell lines growing in vitro there is a negative covariance between PLA2G2A expression and LiPlaCis response, in tissue there is a positive correlation between sPLA2A-IIA mRNA and immunohistochemistry (Mirtti et al APMIS 2009, 117: 151-161)

If the patient is predicted to be responsive, then the patient can be administered LiPlaCis, such as LiPlaCis administered intravenously at a dose of about 75 mg, or about 90 mg, or about 40 mg/mm$^2$ body surface area, or about 55 mg/mm$^2$ body surface area on day 1 and day 8 of a three week regimen. Conversely, if the patient is predicted to be non-responsive to LiPlaCis treatment, then the patient can be administered one or more therapies other than LiPlaCis.

**Example 6. Correlation between DRP score and clinical response (RECIST) in advanced breast cancer patients.**

The cisplatin response profile described in Example 1 and using the biomarkers of Tables 2-3 was validated in a Phase I/II clinical study. The purpose of the study was to correlate the DRP score to the response of the patients to LiPlaCis. The study population consisted of advanced breast cancer patients who provided informed consent to be included in a clinical trial of LiPlaCis and its companion diagnostic DRP (clinicaltrials.gov number NCT01861496). Ten hospitals in Denmark collected diagnostic biopsies from advanced breast cancer patients diagnosed between 1997 and 2016 with a mixture of receptor status. Twelve patients were above the cutoff 33 used for inclusion in the trial and were initiated on LiPlaCis treatment. Ten patients were evaluable for response. The overall outcome of the ten patients are described in Table 6.

**Table 6.** Overall outcome of first 10 patients in LiPlaCis

| All | DRP score | Weeks in study | Best response in LiPlaCis | Line treatments | Unique anticancer treatments | Platins (line) | Duration of latest treatment before LiPlaCis in weeks | Outcome | Gain in relation to latest previous treatment in weeks | Best response before LiPlaCis | Mean duration of treatments before LiPlaCis | Gain in relation to mean previous treatment in weeks | comments + best response |
|---|---|---|---|---|---|---|---|---|---|---|---|---|---|
| Patient #1 | 99 | 32 | PR | 6 | 9 | 0 | 17 | SD | 15 | SD+24weeks | 33,71 | -1,57 | BR previous: SD 46 weeks |
| Patient #2 | 35 | 13 | SD | 12 | 13 | Carboplatin (12) | 19 | SD | -6 | PR | 16,26 | -3,40 | Carboplatin, (in combination with trastuzumab) (SD) |
| Patient #3 | 66 | 5 | PD | 10 | 13 | Carboplatin (12) | 9 | SD | -4 | CR | 38,10 | -33,24 | Carboplatin, (i kombi med Gemcitabin+trastuzumab) (SD) |
| Patient #4 | 94,9 | 6 | PD | 8 | 16 | (4) Oxaliplatin inter Hepar | 8 | PD | -2 | SD+24weeks | 26,33 | -19,90 | Oxaliplatin, (in combination with capecitabine/5FU + trastuzumab). BR: SD 78 weeks |
| Patient #5 | 94,3 | 36 | SD+24weeks | 6 | 8 | 0 | 20 | SD | 16 | SD+24weeks | 27,93 | 8,21 | BR: SD 60 weeks |
| Patient #6 | 80,1 | 25 | PR | 8 | 10 | 0 | 5 | PD | 20 | SD+24weeks | 19,24 | 5,90 | BR previous: SD 52 weeks |
| Patient #7 | 39,4 | 9 | PD | 8 | 9 | 0 | 7 | PD | 2 | SD+24weeks | 29,43 | -20,43 | BR: SD 25 weeks |
| Patient #8 | 94,9 | 12 | SD | 5 | 8 | 0 | 12 | PD | 0 | SD | 11,07 | 0,93 | BR: SD 16 weeks |
| Patient #9 | 80,6 | 18 | SD | 4 | 4 | 0 | 17 | SD | 1 | SD | 8,32 | 9,25 | BR previous: SD 17 weeks |
| Patient #10 | 39,4 | 6 | PD | 9 | 11 | 0 | 12 | PD | -6 | SD+24weeks | 13,64 | -7,93 | BR: SD 36 weeks |
| **Mean** | **72,36** | **16,19** | | **7,60** | | | **12,60** | | **3,59** | **SUM** | 224,04 | -62,18 | |
| **Median** | **80,35** | **12,43** | | **8,00** | | | **12,00** | | **0,29** | **Mean** | 22,40 | -6,22 | |

Of these, three patients had received prior treatment with a platinum based compound, which could interfere with the ability of the DRP to predict response to subsequent platinum based treatment unless a new biopsy is obtained.

The overall outcome of the top third DRP, excluding patients who had previously been treated with platins is outlined in Table 7.

**Table 7.** Top third DRP excluding patients treated with platins before LiPlaCis treatment

| DRP>66 and no previous platin-treatment | DRP score | Weeks in study | Best response | Line treatments | Duration of latest treatment before LiPlaCis / Outcome | Gain in relation to latest previous treatment in weeks | Mean duration of treatments before LiPlaCis | Gain in relation to mean of all previous treatment in weeks |
|---|---|---|---|---|---|---|---|---|
| Patient #1 | 99 | 32 | PR | 8 | 17 | 15 | 33,71 | -1,57 |
| Patient #8 | 94,9 | 12 | SD | 7 | 12 | 0 | 11,07 | 0,93 |
| Patient #5 | 94,3 | 36 | SD+24weeks | 7 | 20 | 16 | 27,93 | 8,21 |
| Patient #9 | 80,6 | 18 | SD | 3 | 17 | 1 | 8,32 | 9,25 |
| Patient #6 | 80,1 | 25 | PR | 9 | 5 | 20 | 19,24 | 5,90 |
| **Mean** | **89,78** | **24,60** | | **6,80** | **14,20** | **10,40** | **20,06** | **4,54** |
| **Median** | **94,30** | **25,14** | | **7,00** | **17,00** | **15,14** | **19,24** | **5,90** |

The statistical analysis was pre-planned in a statistical analysis plan (v2.0, June 21, 2017) before initiation of analysis. The primary analysis was a one-sided Pearson correlation between the DRP score and the tumor response (RECIST criteria encoded as 4,3,2,1 for CR (complete remission), PR (partial response), SD (stable disease), PD (progressive disease), respectively). A secondary analysis was a cox proportional hazards analysis of time to progression or death using the median DRP score as a cutoff. A logrank test with a p-value of 0.05 or less was considered significant. Patients were stratified according to prior platinum treatment.

Patients treated with LiPlaCis on average had 8 prior treatments. Patients in the upper tertile defined by the DRP on average had a longer duration of treatment than on all prior treatments (median 25 weeks versus 20 weeks). Figure 4 shows the duration of treatment on LiPlaCis compared to the most recent prior treatment. Comparing the LiPlaCis treatment to the most recent prior treatment is a surrogate for "doctor's choice" often used in randomized trials. The most recent prior treatment is by doctor's choice, but patients were not treated while on study, thus the frequency of response evaluation and the depth of data monitoring might not be the same as when patients entered the study.

The DRP was very precise in predicting who will benefit from LiPlaCis in this study population. Patients in the upper tertile performed much better than patients in the middle tertile as defined by the DRP score (Figure 2 and 3). Patients in the upper tertile as defined by DRP score also, on average, benefited more from LiPlaCis treatment than from all other previous treatments. The improvement was most dramatic when comparing to the most recent treatment, where patients' risk per time unit of terminating drug was four times lower on LiPlaCis (HR=0.22, Figure 4) than on the previous treatment.

In other words, for all patients in the upper tertile defined by DRP, LiPlaCis was, on average, a clear improvement over the previous treatment.

Further trials should as soon as possible confirm this result so breast cancer patients in the future can gain the obvious and clear clinical benefit from treatment with LiPlaCis

**Example 8. Response of DRP positive patients to LiPlaCis treatment.**

The effectiveness of LiPlaCis administered as per the dosage regimen described herein was validated in a PhaseI/II clinical study. The study population consisted of advanced breast cancer patients who had been identified as DRP positive as per the methods described herein. The patients received LiPlaCis in the following dosage regimen: 2 doses of 75 mg each, administered on day 1 and day 8 of three week treatment cycle/s. Table 8 and Figure 5 elucidate the promising response of the DRP positive patients to this LiPlaCis treatment regimen. The duration of treatment in these patients is illustrated in Figure 6.

**Table 8.** Status and response of DRP positive patients to LiPlaCis treatment.

| Subject No. | Age | First dose | Cycles | Status | Best response | DRP score |
|---|---|---|---|---|---|---|
| Patient #1 | 51 | 30-05-2016 | 8 full cycles →4 doses (every 2nd week) | Off Study | PR | 99 |
| Patient #2 | 55 | 17-jan-17 | 4 (1 treatment cancelled) | Off Study | SD | 35 |
| Patient #3 | 71 | 31-jan-17 | 2 (1 treatment cancelled) | Off Study | PD | 66 |
| Patient #5 | 47 | 27-mar-17 | 10 | Off Study | PR | 94,3 |
| Patient #6 | 51 | 23-maj-17 | 8,5 | Off Study | PR | 80,1 |
| Patient #7 | 59 | 22-aug-17 | 3 | Off Study | SD | 39,4 |
| Patient #9 | 73 | 14-sep-17 | 6 | Ongoing | SD | 80,6 |
| Patient #8 | 52 | 18-sep-17 | 4 | Off Study | SD | 94,9 |
| Patient #10 | 61 | 04-okt-17 | 2 | Off Study | PD | 39,4 |
| Patient #11 | 60 | 04-okt-17 | 1 treatment | Off Study Dead/Cioms | NA - erstattes | 46,5 |
| Patient #12 | 60 | 05-okt-17 | 1 treatment | Off Study Dead/PD | NA - erstattes | - |
| Patient #4 | 51 | 14-11-2017 | 2 | Off Study | PD | 94,9 |

**Example 9. Analysis of adverse effects of LiPlaCis.**

Twelve patients treated with LiPlaCis in a Phase II clinical study, were analyzed for adverse effects of LiPlaCis, if any. The interim data shows that LiPlaCis is well tolerated, with mainly mild and only few moderate side effects; only four grade 3 events and two grade 4 events being recorded as related to study drug in the treated patients. While ototoxicity and nephrotoxicity are well known and frequent related adverse events to conventional cisplatin, no clinically relevant ototoxicity and nephrotoxicity was observed with LiPlaCis. Both ototoxicity and nephrotoxicity occurred at a much lower and milder grades than known with cisplatin. Fever, cytopenia, or clinically relevant platelet toxicity was also not observed. Hand-foot syndrome, a possible adverse effect due to liposomal drug delivery was expected, but not found in the study cohort. Conventional cisplatin treatment of metastatic breast cancer has a 10% response rate.

**Example 10. Preparation of LiPlaCis for administration.**

According to the methods described herein, LiPlaCis infusion liquid can be prepared by withdrawing the required amount of concentrate from vials of LiPlaCis Concentrate for Infusion, and diluting it in two infusion bags, each bag containing 50% of the dose. The amount of concentrate to be withdrawn from the vials of LiPlaCis Concentrate for Infusion can be calculated according to the dose that is to be administered, and the concentration of cisplatin in the LiPlaCis Concentrate for Infusion, as stated in the label. For example, if a patient is to receive a dose of 75 mg of LiPlaCis, which is to be prepared from vials of LiPlaCis Concentrate for Infusion, where the concentration of cisplatin is labeled as 1.1 mg/ml, the amount of LiPlaCis that is to be withdrawn from the vial can be calculated as follows:

$$V_{tot} = 75\ mg/\ 1.1\ mg/ml = 68.2\ ml$$

The required number of vials of LiPlaCis Concentrate for Infusion can be thawed, 68.2 ml can be withdrawn, and 34.2 ml can be added to each of two infusion bags (each bag containing 0.9% sodium chloride, 500 ml) via the medication valve. The infusion liquid can be mixed thoroughly, kept protected from light, and used within 8 hours.

**Example 11. Treating a breast cancer patient with LiPlaCis.**

A physician of skill in the art can treat a patient, such as a human patient with cancer (e.g., breast cancer) by administering LiPlaCis as per the dosage regimens described herein. For example, a patient can be administered two doses of cisplatin, each of about 75 mg of cisplatin, or 90 mg of cisplatin, or each dose comprising cisplatin amounting to about 40 $mg/mm^2$ body surface area, or about 55 $mg/mm^2$ body surface area, on day 1 and day 8 of a three week treatment cycle. The regimen can be repeated for 3 cycles or more. Alternatively, the patient can also be treated by administering escalated doses of cisplatin in subsequent treatment cycles. For example, a patient can be administered two doses of cisplatin, each of about 75 mg of cisplatin, or comprising cisplatin amounting to about 40 $mg/mm^2$ body surface area on day 1 and day 8 of the first three week treatment cycle, followed by two doses of cisplatin, each of about 90 mg of cisplatin, or comprising cisplatin amounting to about 55 $mg/mm^2$ body surface area on day 1 and day 8 of the next three week treatment cycle. Alternatively, a patient can also be administered two doses of cisplatin, each of about 75 mg of cisplatin, or comprising cisplatin amounting to about 40 $mg/mm^2$ body surface area on day 1 and day 8 of the first and second three week treatment cycles, followed by two doses of cisplatin, each of about 90 mg of cisplatin, or comprising cisplatin amounting to about 55 $mg/mm^2$ body surface area on day 1 and day 8 of the third three week treatment cycle. Alternatively, a patient can also be administered two doses of cisplatin, each of about 75 mg of cisplatin, or comprising cisplatin amounting to about 40 $mg/mm^2$ body surface area on day 1 and day 8 of the first three week treatment cycle, followed by two doses of cisplatin, each of about 90 mg of cisplatin, or comprising cisplatin amounting to about 55 $mg/mm^2$ body surface area on day 1 and day 8 of the second and third three week treatment cycles.

**Example 12. Evaluating safety and tolerability, and determining maximum tolerable dose (MTD) of LiPlaCis.**

A Phase I/II study was conducted to evaluate safety and tolerability and to determine the maximum tolerated dose (MTD) of LiPlaCis (Liposomal Cisplatin formulation) in patients with advanced or refractory tumors (see Figure 7). In cohort B and in dose step 5 (after 20 patients; see below), the patient population was limited to skin cancer (not screened for sensitivity) and metastatic breast cancer patients screened by the LiPlaCis DRP (described herein) to be sensitive to LiPlaCis. A Pharmacodynamic (PD) Proof of Concept study was performed in a cohort of 6 patients to investigate the targeted delivery of cisplatin (the active drug in LiPlaCis) in the tumor. Data from this study showed a 5-28-fold increase in DNA platinum adducts (GG-Pt) in tumor tissue over normal tissue of the same patient, compared to a 4-6-fold increase of DNA-platinum (GG-Pt) that is seen with conventional cisplatin, indicating targeted delivery of cisplatin to tumor with LiPlaCis.

*Primary objectives of the study:*

- To evaluate the safety and tolerability of LiPlaCis given on day 1 and day 8 (and possible day 15) every 3 weeks.
- To determine the MTD and the recommended dose (RD) of LiPlaCis given on day 1 and day 8 (and possible day 15) every 3 weeks.

*Secondary objectives of the study:*

- To evaluate pharmacokinetics (PK) of LiPlaCis given on day 1 and day 8 (and possible day 15) every 3 weeks.
- To evaluate the therapeutic efficacy of LiPlaCis given on day 1 and day 8 (and possible day 15) every 3 weeks.
- To evaluate the pharmacodynamics (PD) of LiPlaCis in selected patients.
- Progression-free survival (PFS) for patients from dose step 5.

*Disposition of subjects and exposure:*

Thirty patients were included in the phase I/II study. Four patients were included in dose step 1 (60 + 60 mg), one patient (Patient #14) was not properly screened and was replaced. Four patients were included in dose step 2 (90 + 90 mg), 3 patients were included in dose step 3 (120 + 120 mg) and two patients were included in the dose step 4 (90 + 90 + 45 mg). At dose step 4, both patients were withdrawn from the study, one due to infusion reaction and the other due to rapid progression of disease. Three patients were included in cohort A, and four patients in cohort B as one patient (Patient #30) was replaced. Seven patients were included in dose step 5. Table 9 outlines demographics, exposure, response and prior treatment in 25 patients.

**Table 9.** Demographics, exposure, response and prior treatment on 25 patients from Phase I/II study

| Subject No. | Gender | Age | Diagnosis | Dose, mg/ subject | Cycles adm. | Previous lines of treatment | Best response |
|---|---|---|---|---|---|---|---|
| Patient #13 | M | 64 | Hepatocellular | 60 + 60 mg | 2 Cycles | 1 | PD |
| Patient #14 | F | 55 | Colorectal Cancer - Adenocarcinoma | 60 + 60 mg | 1/2cycle | 5 Incl. Oxaliplatin | PD |
| Patient #15 | M | 66 | Colorectal Cancer - Adenocarcinoma | 60 + 60 mg | 2 Cycles | 4 Incl. Oxaliplatin | PD |
| Patient #16+ | F | 57 | NSCLC, Adenocarcinoma | 60 + 60 mg | 2 Cycles | 5 Incl. Carboplatin | PD |
| Patient #17*+ | F | 71 | Colon Cancer - Adenocarcinoma | 90 + 90 mg | 6 Cycles | 3 Incl. Oxaliplatin | SD-18 weeks |
| Patient #18' | M | 52 | Esophagus Cancer - Adenocarcinoma | 90 + 90 mg | 8 Cycles | 4 Incl. Carboplatin | PR23 weeks |
| Patient #19 | F | 60 | Colorectal Cancer - Adenocarcinoma | 90 + 90 mg | 2 Cycles | 4 Incl. Oxaliplatin+Cis | PD |
| Patient #20+ | F | 60 | Colorectal Cancer - Adenocarcinoma | 120 + 120 mg | 1 Cycle | 8 Incl. Oxaliplatin Carboplatin+ | PD |
| Patient #21*+ | M | 65 | Cancer cutis - Squamous cell carcinoma | 120 + 120 mg | 3 Cycles | 2 Incl. Carboplatin | PR CR after Operation |
| Patient #22+ | F | 50 | Colon Cancer - Adenocarcinoma | 120 + 120 mg | 1½ Cycles | 3 Incl. Oxaliplatin | PD |
| Patient #23 | M | 44 | NSCLC - Adenocarcinoma | 90 + 90 mg | 2 Cycles | 5 Incl. Carboplatin | PD |
| Patient #24 | M | 59 | NSCLC - Squamous cell carcinoma | 90+90+ 45mg | 2 Cycles | 2 Incl. Carboplatin | PD |
| Patient #25 | M | 60 | Pancreatic Cancer - Adenocarcinoma | 90+90+ 45mg | 1/3 of Cycle | 2 Incl. Oxaliplatin | PD |
| Patient #26* | M | 59 | Larynx cancer - Planocellulaer carcinoma | 60 + 60 mg | 6 Cycles | 6 Incl. Carboplatin+Cis | SD-23 weeks |
| Patient #27 | M | 48 | Gastric - Mixed Adenoneuroendocrine Carcinoma | 60 + 60 mg | 3 Cycles | 4 Incl. Carboplatin+Cis | SD-8 weeks |
| Patient #28* | F | 47 | Breast cancer - Adenocarcinoma | 60 + 60 mg | 4 Cycles | 8 Incl. Carboplatin | SD-14 weeks |
| Patient #29*+ | F | 38 | Breast cancer | 90 + 90 mg | 6 Cycles | 8 | SD-18 weeks |
| Patient #30+ | F | 62 | Pancreatic cancer - Adenocarcinoma | 90 + 90 mg | ½ cycle | 3 Incl. Oxaliplatin | PD |
| Patient #31*+ | M | 72 | Liver cancer - hepatocellular carcinoma | 90 + 90 mg | 6 Cycles | 3 | SD-18 weeks |
| Patient #32+ | M | 64 | Colon cancer - Adenocarcinoma | 90 + 90 mg | 1 Cycle | 3 | PD |

(continued)

| Subject No. | Gender | Age | Diagnosis | Dose, mg/ subject | Cycles adm. | Previous lines of treatment | Best response |
|---|---|---|---|---|---|---|---|
| Patient #33*+ | F | 50 | Breast cancer - Ductal carcinoma | 75 + 75 mg | 10 Cycles | 8 | PR-32 weeks |
| Patient #34+ | F | 55 | Breast cancer - Carcinoma in situ | 75 + 75 mg | 4 Cycles | 12 Incl. Carboplatin | SD-13 weeks |
| Patient #35+ | F | 72 | Breast cancer - Carcinoma | 75 + 75 mg | 2 Cycles | 12 Incl. Carboplatin | PD |
| Patient #36*+ | F | 46 | Breast cancer | 75 + 75 mg | 9 Cycles - ongoing | 7 | SD-27weeks |
| Patient #37*+ | F | 50 | Breast cancer - Carcinoma | 75 + 75 mg | 6 Cycles - ongoing | 9 | SD 19 weeks |
| *Narratives describing individual cases of patients responding to LiPlaCis therapy. + DRP evaluated patients | | | | | | | |

*Dose step 1 (60 + 60 mg):*

No dose-limiting toxicity (DLT) was reported for this dose level. Three severe adverse events (SAEs) were reported. Two hospitalizations due to drug related reversible fever and one hospitalization due to hypomagnesaemia to administer IV magnesium were reported. It was decided to escalate the dose to 90 + 90 mg.

*Dose step 2 (90 + 90 mg):*

No DLTs were reported for this dose level. Patient 01-006 had creatinine and Cr-EDTA values that corresponds to grade 2. Patient 01-005 and 01-007 experienced a rise in temperature corresponding to a grade 1 and grade 2, respectively. According to protocol, the next dose step should have been 135+135 mg, however it was decided that a dose increase from a total dose of 180 mg to 270 mg was a too large dose step to take and the dose should be increased to only 120 + 120 mg.

*Dose step 3 (120 + 120 mg):*

Two DLTs were reported at this dose step after inclusion of 3 patients. Kidney toxicity and 2 SAEs were reported for patient 01-008 and 01-010.

*Dose step 4 (90 + 90 + 45 mg):*

The next three patients that were included received 90 + 90 mg (01-011), 90 + 90 + 45 mg (01-012) and 90 + 90 + 45 mg (01-013). The day 15 treatment of 45 mg (Cohort 4) was added to investigate if a three-weekly schedule was feasible. At the same time, paracetamol was given prophylactic as pre-medication and the infusion time was increased to 3 hours for dosages above 90 mg to prevent infusion related reactions. Furthermore Cr-EDTA on day 8 prior to treatment was implemented.

**Measurement of PD markers**

Cohorts A (60 + 60 mg) and B (90 + 90 mg) were used to measure PD before the dose for the extension phase was decided. Total platinum, DNA-platinum and $sPLA_2$-IIA protein levels was measured in these. The dose for the extension phase was decided to be 75 + 75 mg due to fatigue at dose step 90 + 90 mg. The regimen of pre-medication was extended with prednisolone and ibuprofen. Post hydration was prolonged at the same time to protect the kidneys.

*Dose step 5 (75 + 75 mg) chosen to be the RD*

In this phase II part of the study only DRP screened advanced breast cancer patients and a few not screened skin cancer patients were enrolled. Recommended Dose (RD) was chosen at 75+75 mg, though there was no Dose Limiting Toxicity (DLT) at the 90+90 mg level.

*Adverse events in 25 patients*

In the ongoing study, all patients experienced one or more treatment emergent adverse events (TEAEs), and in all except 1 patient, one or more of the TEAEs were considered LiPlaCis related. A total of 485 AEs were reported for 25 patients, of whom 2 patients were ongoing, of these 62% were deemed possibly related to the study drug. Most of the LiPlaCis-related AEs were of mild to moderate severity, i.e., in 40% (10/25) of the patients. Severe TEAEs were reported for 12 patients (48%); in 8 patients (32%), one or more of the severe TEAEs were considered LiPlaCis-related, 3 patients in the 60 + 60 mg, 3 patients in the 90 + 90 mg, and 2 patients in the 120 +120 mg dose groups.

The most frequently reported LiPlaCis-related AEs were nausea (16/25; 64%) and fatigue (14/25; 56%), followed by hypomagnesaemia (12/25; 48%), vomiting (11/25; 44%), anorexia (8/25; 32%), fever (7/25; 28%), nephrotoxicity (6/25 24%), infusion-related reaction (IRR) (10/25; 40%), Chills (5/25; 20%), hypokalaemia (4/25; 17%). The other LiPlaCis related AEs were reported for 1, 2 or 3 patients each.

In total, 12 patients (48%) experienced one or more SAEs, and in 8 patients (32%) one or more of these SAEs were considered LiPlaCis-related. For 8 patients (32%), study treatment was discontinued because of an AE, 5 patients in the 90 + 90 mg dose group, and 3 patients in the 120 + 120 mg dose group. In all 8 patients, the AE leading to study treatment discontinuation was LiPlaCis-related. There were no deaths on the study.

*Adverse events in 12 patients (patient 14 to 25)*

The regimen of pre-medication was extended with prednisolone and ibuprofen to prevent IRR. AEs on infusion-related reaction was subsequently reduced from 40 % to 17% (2/12).

*SAE and study discontinuations*

Twenty SAE's were reported. In total twelve patients (48%) experienced one or more SAE and in 8 patients (22%) one or more of these SAEs were considered LiPlaCis-related. Fever (Grade 2) was the most frequently reported SAE, namely for 3/25 (12%) patients, these SAE's were not considered LiPlaCis-related, all three at 90 + 90 mg. The LiPlaCis-related SAEs seen were acute kidney injury in two patients, one Grade 3, and one Grade 1 (at 90+90 mg (CTC 2) and 120+120 mg (CTC 1)), respectively; infusion related fever in 2 patients (at 60+60 mg), hypomagnesaemia in 2 patients, one Grade 2 and one Grade 3 (both at 60+60 mg); Grade 3 thromboembolic event in one patient (90+90 mg), Grade 2 nausea in one patient (90+90 mg), and Grade 2 elevated kidney counts in one patient (120+120 mg), where elevated kidney counts (120+120 mg) and acute kidney injury (120+120 mg) led to DLT. No treatment related deaths were reported in the study.

*Drug response prediction (DRP)*

DRP is an assay that based on samples from a tumor can predict the likelihood for a tumor to respond to a specific drug. The DRP method builds on the comparison of sensitive and resistant cell lines including genomic information from the NCI60 cell lines, clinical tumor biology and clinical correlates in a systems biology network. mRNA measurements are used to make such drug prediction. Pre-clinical and clinical validation of response predictors have been developed for a number of drugs, with a unique signature of genes for each drug. This signature is matched to the corresponding genes in the universal microarray (which contains all genes) in order to make prediction for a specific drug for a specific patient. All breast cancer patients included in the phase II part of this study were predicted to be sensitive to LiPlaCis.

*DRP in 11 patients treated in this study:*

Data from this Phase I/II study shows that tumor response to LiPlaCis can be predicted by DRP independent of tumor type and including breast cancer. Of the 11 patients analysed (8 from the phase I part and 3 from the phase II part) with mixed solid tumors, 2 patients had a Partial Response (PR) (one of these was operated and in Complete Remission (CR) 1 year after) and 4 patients had Stable Disease (SD). The correlation between prediction and response to treatment was 0.5 with a one-sided p-value of 0.06. Due to the small number of patients and mixed tumor types, this is a successful validation of the DRP's ability to predict response. These early data suggest that patients predicted sensitive by DRP to LiPlaCis (top third) have a 67% probability of response, and a median of 18 weeks to progression.

*Conclusion:*

In this study (30 of approximately 40 patients were included), 2 DLTs were reported. This was a Grade 1 acute kidney injury in the first treatment cycle and a Grade 3 elevated kidney counts in the first treatment cycle both in the 120 mg dose group. It was decided to lower the dosage hereafter, and no further DLTs have been observed.

The toxicity observed in this study seems similar to what has been experienced with common cis-platinum containing regimen. Nephrotoxic effects have been observed with cisplatin therapies, although LiPlaCis appears to be well-tolerated. No ototoxicity or neurotoxicity was observed. These types of toxicities should be carefully looked for as these toxicities may depend on, e.g., the individual cumulated dose of LiPlaCis, numbers of prior treatment regimens and the type of anticancer drugs the patients have been exposed to. It should be mentioned that no Hand and Foot Syndrome, as well as no indication of bone marrow depletion and alopecia were observed.

Response and clinical benefit is notable as 3 PR are observed as well as 11 SD lasting from 8 to 32 weeks median time to progression 18 weeks. One of the PR patients was curative operated on and after one year still in CR. At present the study is including patients in the phase II part of the trial. The recommended dosage is two weekly doses of 75 mg in a 3 weekly cycle. Ten patients have been included on this dosage and further up to 20, mainly breast cancer patients are planned to be included.

**Example 13. Narratives describing individual cases of patients responding to LiPlaCis therapy**

Outlined below are narratives describing the individual patients treated with LiPlaCis.

**Patient #17:**

This patient is a 68-year-old woman diagnosed with colon cancer in April 2010. The patient underwent surgery in April 2010 and was subsequently treated with oxaliplatin + 5-FU / irinotecan + 5-FU / bevacizumab / Regorafinib (four lines of treatment were given). PR was observed in all cases as the best response. The patient met the entrance requirements for the LiPlaCis trial (Liver and lymph nodes, .54 mm, PS 0, normal Cr-EDTA).

In October 2013, the patient entered the LiPlaCis protocol in a dose-escalation part at 90+90 mg day 1 + 8 every 3 weeks and received 6 cycles (Cumulative dose: 1080 mg).

The patient exhibited a best response of SD of 18 weeks, as determined in November 2013 (verified Dec. 2013). The patient exhibited AE Grade 1: Fever, Vomiting, Nausea, Chills, and AE Grade 2: Hypomagnesemia, Fatigue, Bronchospasm. No grades 3 or 4 AE were observed.

The patient exited the LiPlaCis protocol in February 2014 after PD (new lesions) with status: PS 1, normal Cr-EDTA.

**Patient #18:**

This patient is a 47-year-old man diagnosed with esophagus cancer in September 2008. The patient had radiation therapy in February 2010, and underwent surgery in August 2012. From 2008, the patient was treated with Carboplatin + docetaxel + capecitabine / Cisplatin + 5-FU / Carboplatin + docetaxel + capecitabine / Irinotecan (four lines of treatment were given). PR was observed in all cases as the best response. The patient met the entrance requirements for the LiPlaCis trial (Lymph node 53 mm, PS 1, normal (lower end) Cr-EDTA).

In Novemeber 2013, the patient entered the LiPlaCis protocol in dose-escalation part at 90+90 mg day 1 + 8 every 3 weeks and received 8 cycles (Cumulative dose: 1170 mg).

The patient exhibited a best response of PR of 23 weeks, as determined in January 2014 (verified in February 2014). The patient exhibited AE Grade 1: Nausea, Vomiting, Diarrhea, Nutrition disorder, Chills, Hypomagnesemia, and AE Grade 2: Fatigue, Hypomagnesemia, Nausea. No grades 3 or 4 AE were observed.

The patient exited the LiPlaCis protocol in April 2014 after PD (new lesions) with status: PS 1, below normal Cr-EDTA (40 ml/min).

**Patient #21:**

This patient is a 65-year-old man diagnosed with cancer cutis, squamous cell carcinoma (well diff.) in May 2007. The patient underwent surgery in 2007, 2009 and 2010, had radiotherapy in 2011, and was treated with capecitabine + paclitaxel / vinorelbine + carboplatin (2 lines of treatment were given). PR was observed in all cases as the best response. The patient met the entrance requirements for the LiPlaCis trial (Tumor scalp wound 60 mm, PS 1, normal Cr-EDTA).

In January 2014, the patient entered the LiPlaCis protocol in dose-escalation part at 120+120 mg day 1 + 8 every 3 weeks and received 2½ cycles (Cumulative dose: 540 mg).

The patient exhibited a best response PR enabling CR after surgery and remained disease free after 12 months. Latest measurement were not evaluable by RECIST criteria. The patient exhibited AE Grade 1: Vomiting, Anorexia, Headache, Flu like symptoms, Hypomagnesemia, Nausea. AE Grade 2: Infusion Related Reaction, Fatigue, Dyspnea, Renal disorders. No grades 3 or 4 AE were observed.

The patient exited the LiPlaCis protocol in April 2014 for renal disorders (Cr-EDTA 42 ml/min).

**Patient #26:**

This patient is a 54-year-old male diagnosed with larynx cancer (Poorly diff.) in October 2009. The patient received radiation and underwent surgery in 2009, and was subsequently treated with Zalutumumab + Cisplatin / Taxol + Xeloda / Carboplatin + Vinorelbine / Bleomycin / Cetuximab + R05479599 / Bleomycin (six lines of treatment were given). SD was observed in all cases as best response. The patient met the entrance requirements for the LiPlaCis trial (Right side neck 145 mm, PS 1, normal Cr-EDTA).

In June 2015, the patient entered the LiPlaCis protocol in dose-escalation part at 60+60 mg day 1 + 8 every 3 weeks and received 6½ cycles (Cumulative dose: 780 mg).

The patient exhibited a best response of PR of 23 weeks, as determined in July 2015 (verified August 2015). Significant clinical response was observed on neck tumor and food intake. The patient exhibited AE Grade 1: Nausea, Flu like symptoms, Edema, Fatigue, Vomiting, Palmar Plantar Erythrodysethesia, Anemia, Hypokalemia, Weight loss, Headache, Diarrhea, Skin infection, AE Grade 2: Fatigue, Constipation, Weight loss, Anemia, Nausea, AE Grade 3: Hypomagnesemia, Hypermagnesemia, and SAE: Hypomagnesemia grade 3, Tracheal hemorrhage grade 3 (not related). The patient exited the LiPlaCis protocol in November 2015 after PD (new lesions) with status: PS 1, normal Cr-EDTA.

**Patient #28**

This patient is a 41-year-old woman diagnosed with breast cancer (Poorly diff.) in Mar 2009. The patient received radiation and underwent surgery to the left axil in 2009, and was subsequently treated with Taxotere + Herceptin / Vinorelbine + Herceptin / Xeloda + Lapatinib / Trastuzumab / Trastuzumab + Perstuzumab + Gemcitabin + Carboplatin / Epirubicin / Trastuzumab + Eribulin / R06895882 (eight lines of treatment were given). PR was observed in all cases as best response. The patient met the entrance requirements for the LiPlaCis trial (Lymph Nodes 52 mm, PS 0, normal Cr-EDTA).

In November 2015, the patient entered the LiPlaCis protocol in dose-escalation part at 60+60 mg day 1 + 8 every 3 weeks and received 4 cycles (Cumulative dose: 480 mg).

The patient exhibited a best response of SD of 14 weeks, as determined in July 2015 (verified August 2015). The patient exhibited AE Grade 1: Vomiting, Edema, Diarrhea, Nausea, Peripheral sensory neuropathy, Dyspnea, Pain groin, Cramps in hands, and AE Grade 2: Fever, Nausea, Anemia, Hypomagnesemia, Infection in port-$\alpha$-cath, Thromboembolic event, Weight loss, Infection, Creatinine increased, Edema both legs. No Grade 3 and Grade 4 AE were observed. The patient exhibited SAE: Fever (Not related) on 23 November 2015, Infection (Not related) as determined on 18 January 2016.

The patient exited the LiPlaCis protocol in February 2016 at Principal Investigator's decision (PS 1, normal Cr-EDTA).

**Patient #29:**

This patient is a 38-year-old woman diagnosed with breast cancer in Aug 2008. The patient underwent Mastectomy (left side) and was treated with Cyclophosphamid + Epirubicin + 5-FU and tamoxifen. I n 2009, the patient underwent prophylactic removal of right side breast and ovaries. The patient exhibited relapse in brain and liver in 2011 (ER neg, HER2 pos). The patient was treated with Herceptin / Herceptin + vinorelbine / docetaxel + Herceptin / capecitabine + lapatinib / Trastuzumab + Emtanzine / Herceptin + Lapatinib and whole-brain radiation (eight lines of treatment were given). CR was observed in one of the treatments as the best response. The patient met the entrance requirements for the LiPlaCis trial (PS 1, normal Cr-EDTA. Index tumors in liver, 37 mm).

In December 2015, the patient entered the LiPlaCis protocol at 90 +90 mg day 1 + 8 every 3 weeks and received 6 cycles (Cumulative dose: 1080 mg).

The patient exhibited a best response of SD of 22 weeks, as determined in February 2016 (verified March 2016). The patient exhibited AE Grade 1: Mucositis, Pain drainage tube, Weight loss, Hypokalemia, Edema ankles, Cushingoid, Hypomagnesemia, and AE Grade 2: Constipation, Urinary tract infection, Pain Back, Anemia, Stomach Pain, Fatigue, Biloma, Infection drainage cavity, Ulcus, Acute kidney injury, Ataxia. No Grade 3 and Grade 4 AE were observed. The patient exhibited SAE: Constipation (Not related) in January 2016, Infection of insertion of former drainage cavity (Not related) in March 2016.

The patient exited the LiPlaCis protocol in February 2016 (PS 2, Cr-EDTA 54 ml/min).

**Patient #31:**

This patient is a 71-year-old male diagnosed with liver cancer in August 2015. The patient did not undergo radiotherapy or surgery, and was treated with Doxorubicin/ Naxavar / Ly3039478 (three lines of treatment were given). SD was observed in all cases as best response. The patient met the entrance requirements for the LiPlaCis trial (Liver 166 mm, PS 1, normal Cr-EDTA).

In February 2016, the patient entered the LiPlaCis protocol in dose-escalation part at 90+90 mg day 1 + 8 every 3 weeks and received 6 cycles (Cumulative dose: 990 mg).

The patient exhibited best response of SD of 18 weeks, as determined in April 2016 (verified May 2016). The patient exhibited AE Grade 1: Infusion related reaction, Nausea, Vomiting, Anorexia, Fever, Creatinine increased, and AE Grade 2: Fatigue, Dry skin, Cronic kidney disease. No Grade 3 and Grade 4 AE were observed. The patient exited the LiPlaCis protocol in February 2016 due to increased kidney toxicity (PS 1, below normal Cr-EDTA 51ml/min).

**Patient #33:**

This patient is a 51-year-old woman diagnosed with breast cancer in October 2008. The patient underwent mastectomy (right side), and was treated with Adjuvant Epirubicin + Cyclophosphamid / Docetaxel, radiation and Tamoxifen. The patient exhibited relapse in bone and liver in December 2012, and was treated with docetaxel/ letrozole / vinorelbine- capecitabine/ eribulin / paclitaxel (eight lines of treatment were given). SD was observed as best response. The patient met the entrance requirements for the LiPlaCis trial (Multiple liver met 78 mm. PS 0, normal Cr-EDTA).

In May 2016, the patient entered the LiPlaCis protocol at 75+75 mg Phase II part day 1 + 8 every 3 weeks at Rigshospitalet and received 12 cycles (Cumulative dose: 1500 mg).

The patient exhibited a best response of PR of 32 weeks, as determined in July 2016 (verified August 2017). The patient exhibited AE Grade 1: Nausea, PSN in ankles. Edema, Fatigue, Neuropathy intermittent, Hypomagnesemia, Tinnitus, Vomiting, Anorexia, Constipation, Dyspepsia, Hyponatremia, Neuropathy in fingers, Pain right femur, AE Grade 2: Pain in epigastrium, Headache (infusion related), and AE Grade 3: Neutrophil Count Decreased. The patient exited the LiPlaCis protocol in January 2017 after PD (PS 1, normal Cr-EDTA).

**Patient #34:**

This patient is a 55-year-old woman diagnosed with breast cancer in August 2008. The patient underwent mastectomy left side, and was treated with Adjuvant Epirubicin + Herceptin + Tamoxifen + Docetaxel + radiation / Vinorelbine + Herceptin / Docetaxel + Herceptin / Lapatinib + Capecitabine / TDM-1 / Eribulin + Trastuzumab / Paclitaxel + Trastuzumab / Letrozol + Trastuzumab / Epirubicin / Exemestan / Capecitabine + Trastuzumab / Carboplatin + Trastuzumab (twelve lines of treatment were given). SD was observed as the best response. The patient met the entrance requirements for the LiPlaCis trial (Multiple liver met. 147 mm, PS 1, normal CrEDTA).

In January 2017, the patient entered the LiPlaCis protocol at 75+75 mg Phase II part day 1 + 8 every 3 weeks at Herlev and received 4 cycles (Cumulative dose: 525 mg).

The patient exhibited a best response of SD of 12 weeks, as determined in April 2017 ((not verified as patient went out of study due to new lesion). The patient exhibited AE Grade 1: Tremor, Stomach pain, Palpitation, Nausea, Hypomagnesemia, Edema extremities, Malaise, Vomiting, Dyspnea, Vertigo, Bloating, AE Grade 2: Anemia, Fatigue, Malaise, Nausea, AE Grade 3: Insomnia, High cholesterol, and SAE: Grade 3 Bilirubinemia. The patient exited the LiPlaCis protocol in Apr 2017 due to SAE and new lesions (PS 1, normal Cr-EDTA).

**Patient #36 (Ongoing):**

This patient is a 39-year-old woman diagnosed with breast cancer in August 2009. The patient underwent mastectomy (right side), and was treated with Neo adjuvant docetaxel / Adjuvant letrozole / Radiation / capecitabine + vinorelbine / tamoxifen/ epirubicin / fulvestrant / pactitaxel (seven lines of treatment were given). SD was observed as the best response. The patient met the entrance requirements for the LiPlaCis trial (Lung left side met. mm, PS 1, normal Cr-EDTA).

In March 2017, the patient entered the LiPlaCis protocol at 75+75 mg Phase II part, day 1 + 8 every 3 weeks at Vejle and have received 9 cycles (Cumulative dose: 1350 mg). The patient exhibited a best response of SD of 28 weeks, as determined in June 2017 (verified August 2017). A SD of more than 24 weeks changes response status to PR. The patient exhibited AE Grade 1: Constipation, Nausea, Closed auditory canal, Prickly sensation tongue. No Grade 2, 3 and 4 AE were observed. The patient is still in the LiPlaCis protocol (October 2017 values: PS 0, below normal Cr-EDTA).

**Patient #37 (Ongoing):**

This patient is a 40-year-old woman diagnosed with breast cancer in May 2006. The patient underwent mastectomy (right side), and was treated with Adjuvant epirubicin + cyclophosphamide + 5-FU / tamoxifen / Radiation / Docetaxel / Letrozol / Fulvestrant / Docetaxel / Capecitabine / Eribulin / Paclitaxel (nine lines of treatment were given). SD was observed as the best response. The patient met the entrance requirements for the LiPlaCis trial (Liver met. PS 0, normal Cr-EDTA, ALT/AST/Alkaline Phosphatase above 5xULN).

In March 2017, the patient entered the LiPlaCis protocol at 75+75 mg Phase II part, day 1 + 8 every 3 weeks at Vejle and have received 7 cycles (Cumulative dose: 1050 mg). The patient best response of SD of 20 weeks, as determined in June 2017 (verified August 2017). The patient exhibited AE Grade 1: Dyspnea, Fatigue, Anorexia. The patient is still in the LiPlaCis protocol (Oct 2017 values: PS 0, normal Cr-EDTA, normal ALT/AST/Alkaline Phosphatase).

**OTHER EMBODIMENTS**

All publications, patents, and patent applications mentioned in the above specification are hereby incorporated by reference. Various modifications and variations of the described device and methods of use of the invention will be apparent to those skilled in the art without departing from the scope and spirit of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention. For example, it is anticipated that measuring the level of proteins, metabolites, identifying genetic mutations and DNA copy number variations, all will be useful in determining patient responsiveness.

**CLAUSES**

1. A method of treating a subject with cancer comprising administering to the subject at least first and second doses of a composition comprising a secretory phospholipase A2 ($sPLA_2$) hydrolysable, cisplatin-containing liposome on day 1 and day 8, respectively, of at least one three week treatment cycle, wherein each of the doses of the composition comprise cisplatin in an amount of about 75 mg to about 90 mg or cisplatin in an amount of about 40 $mg/m^2$ body surface area to about 55 $mg/m^2$ body surface area of the subject.

2. The method of clause 1, wherein the first and second doses of the composition comprise about 75 mg of cisplatin.

3. The method of clause 1, wherein the first and second doses of the composition comprise about 90 mg of cisplatin.

4. The method of clause 1, wherein the first and second doses of the composition comprise about 40 $mg/m^2$ body surface area of the subject.

5. The method of clause 1, wherein the first and second doses of the composition comprise about 55 $mg/m^2$ body surface area of the subject.

6. The method of any one of clauses 1-5, wherein an amount of about 150 mg to about 180 mg cisplatin is administered to the subject in each three week treatment cycle.

7. The method of clause 6, wherein an amount of about 150 mg cisplatin is administered to the subject in each three week treatment cycle.

8. The method of clause 6, wherein an amount of about 180 mg cisplatin is administered to the subject in each three week treatment cycle.

9. The method of any one of clauses 1-8, further comprising administering one or more additional therapies to the subject prior to, concurrently with, or after administration of the composition, wherein optionally the one or more additional therapies comprise surgery, radiation, or a therapeutic agent, and wherein optionally the therapeutic agent is selected from the group consisting of docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, and rituximab.

10. The method of any one of clauses 1-9, wherein the composition is administered to the subject intravenously, intramuscularly, transdermally, intradermally, intra-arterially, intracranially, subcutaneously, intraorbitally, intraventricularly, intraspinally, intraperitoneally, or intranasally.

11. The method of clause 10, wherein the composition is administered to the subject by intravenous infusion.

12. The method of clause 11, wherein the composition is administered to the subject over a period of 2-3 hours.

13. The method of clause 12, wherein the composition is administered to the subject as a 2 hour infusion.

14. The method of clause 12, wherein the composition is administered to the subject as a 3 hour infusion.

15. The method of any one of clauses 1-14, wherein the three week treatment cycle is repeated two to twenty times.

16. The method of any one of clauses 1-15, wherein the subject has been determined to be responsive to the composition prior to administration of the composition.

17. The method of any one of clauses 1-15, wherein the method further comprises determining the responsiveness of the subject to the composition, wherein the method comprises:

(a) contacting a sample comprising one or more nucleic acid molecules from the subject with a device comprising:

i) one or more single-stranded nucleic acid molecules capable of specifically hybridizing with nucleotides of one or more biomarkers of sensitivity selected from those listed in Tables 2 and/or 4, or a complement thereof; and/or
ii) one or more single-stranded nucleic acid molecules capable of specifically hybridizing with nucleotides of one or more biomarkers of resistance selected from those listed in Tables 3 and/or 5, or a complement thereof; and

(b) detecting a level of the one or more biomarkers of sensitivity or the complement thereof and/or the one or more biomarkers of resistance or the complement thereof in the sample by detecting hybridization between the one or more single-stranded nucleic acid molecules of the device and the one or more nucleic acid molecules of the sample.

18. The method of clause 17, wherein the one or more biomarkers of sensitivity is not C1QR1 (SEQ ID NO: 13), SLA (SEQ ID NO: 48), PTPN7 (SEQ ID NO: 77), CENTB1 (SEQ ID NO: 37), IFI16 (SEQ ID NO: 17 or 261), ARHGEF6 (SEQ ID NO: 36 or 294), CD3D (SEQ ID NO: 81), ARHGAP15 (SEQ ID NO: 30), HCLS1 (SEQ ID NO: 16 or 259), CD53 (SEQ ID NO: 282), PTPRCAP (SEQ ID NO: 8), or PTPRC (SEQ ID NO: 10, 18, 25, or 243).

19. The method of clause 17 or 18, wherein the subject is determined to be responsive to the composition comprising $sPLA_2$ hydrolysable, cisplatin-containing liposome if:

i) the level of the biomarkers of sensitivity or the complement thereof is substantially similar to the level of the biomarkers of sensitivity or the complement thereof in a cell or tissue known to be sensitive to the composition;

and/or
ii) the level of the biomarkers of resistance or the complement thereof is substantially dissimilar to the level of the biomarkers of resistance or the complement thereof in a cell or tissue known to be resistant to the composition.

20. The method of any one of clauses 17-19, comprising detecting a level of PLA2G2A (SEQ ID NO: 380), or a complement thereof, in the sample from the subject.

21. The method of clause 20, wherein the method comprises determining the level of PLA2G2A, or a complement thereof, by performing microarray analysis or qRT-PCR.

22. The method of any one of clauses 17 to 21, further comprising detecting $sPLA_2$ protein in a tumor sample from the subject.

23. The method of clause 22, comprising contacting the tumor sample with an anti-$sPLA_2$ antibody and detecting binding between the $sPLA_2$ protein and the anti-$sPLA_2$ antibody.

24. The method of clause 23, wherein said method further comprises administering one or more cancer therapies other than the composition comprising $sPLA_2$ hydrolysable, cisplatin-containing liposome to the subject.

25. The method of any one of clauses 18-24, wherein the cell or tissue known to be sensitive to the composition comprising $sPLA_2$ hydrolysable, cisplatin-containing liposome and/or the cell or tissue known to be resistant to the composition is of the same type as a cell or tissue in the sample from the patient or from which the one or more nucleic acid molecules of the sample are derived, wherein optionally the cell or tissue known to be sensitive to the composition and/or the cell or tissue known to be resistant to the composition is of the same type of cancer as a cell or tissue in the sample from the subject or from which the one or more nucleic acid molecules of the sample are derived.

26. The method of any one of clauses 18-25, wherein the sample from the subject is a tumor sample.

27. The method of any one of clauses 1-26, wherein the subject is resistant to one or more cancer therapies other than the composition, wherein optionally the one or more cancer therapies comprise surgery, radiation, or a therapeutic agent.

28. The method of clause 27, wherein the subject exhibits cancer relapse after treatment with the one or more cancer therapies.

29. The method of any one of clauses 1-28, wherein the cancer is selected from a solid tumor cancer and a hematological cancer.

30. The method of any one of clauses 1-29, wherein the cancer is selected from the group consisting of breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, renal cell carcinoma (RCC), esophageal cancer, melanoma, glioma, pancreatic cancer, gastrointestinal stromal tumors (GIST), sarcoma, non-small cell lung carcinoma (NSCLC), prostate cancer, ovarian cancer, colon cancer, bladder cancer, and squamous cell carcinoma of the head and neck (SCCHN).

31. The method of clause 30, wherein the breast cancer is an estrogen receptor-positive (ERpos) breast cancer and/or a metastatic form of breast cancer.

32. The method of any one of clauses 1-31, wherein the subject has not been administered a treatment for cancer.

33. The method of any one of clauses 1-31, wherein the subject exhibits cancer relapse after a first cancer treatment and prior to treatment with the composition.

34. The method of any one of clauses 1-15, wherein the responsiveness of the subject to the composition is not determined prior to administration of the compound to the subject.

35. The method of any one of clauses 17-33, wherein the device comprises at least two, at least three, at least four, at least five, at least six, at least seven, at least eight, at least nine, at least ten, or more single-stranded nucleic acid molecules of i) and/or ii).

36. The method of any one of clauses 17-33, wherein the one or more single-stranded nucleic acid molecules of the device have a length in the range of 10 to 100 nucleotides in length, wherein optionally, the one or more of the single-stranded nucleic acid molecules have a length in the range of 20 to 60 nucleotides.

37. The method of any one of clauses 17-36, wherein the one or more single-stranded nucleic acid molecules are labeled or immobilized on a solid substrate.

38. The method of any one of clauses 17-37, comprising converting the level of the one or more biomarkers of sensitivity or the complement thereof and/or the one or more biomarkers of resistance or the complement thereof into a mean score, wherein the mean score indicates the responsiveness of the subject to the composition.

39. The method of clause 38, wherein the method further comprises subtracting the mean score for the one or more of the biomarkers of resistance from the mean score for the one or more of the biomarkers of sensitivity to obtain a difference score, wherein the difference score indicates the responsiveness of the subject to the composition.

40. The method of clause 38 or 39, wherein the mean score and/or the difference score above a cutoff value indicates that the subject is responsive to the composition.

41. The method of clause 40, wherein the cutoff value is about 0.1, about 0.15, about 0.2, about 0.25, about 0.3, about 0.35, about 0.4, about 0.45, about 0.5, or greater.

42. The method of any one of clauses 18-41, wherein the device is a microarray or is for performing a quantitative reverse transcriptase polymerase chain reaction (qRT-PCR) reaction.

43. The method of any one of clauses 18-42, wherein the level of the one or more biomarkers of sensitivity or the complement thereof and/or the one or more biomarkers of resistance or the complement thereof are detected by performing microarray analysis or qRT-PCR.

44. The method of any one of clauses 18-43, wherein the nucleic acid molecules of the sample comprise mRNA or a cDNA thereof.

45. The method of any one of clauses 18-44, wherein the biomarker of sensitivity is selected from one or more of COL5A2 (SEQ ID NO: 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), EBI2 (SEQ ID NO: 9), ANP32E (SEQ ID NO: 11), SFPQ (SEQ ID NO: 12, 38 or 272), FNBP1 (SEQ ID NO: 14 or 28), CBFB (SEQ ID NO: 15), SFRS7 (SEQ ID NO: 19 or 54), and CAP350 (SEQ ID NO: 20 or 61).

46. The method of clause 45, wherein the biomarkers of sensitivity comprise COL5A2 (SEQ ID NO 73 or 211) and ITGA4 (SEQ ID NO: 1).

47. The method of clause 45, wherein the biomarkers of sensitivity comprise COL5A2 (SEQ ID NO 73 or 211), ITGA4 (SEQ ID NO: 1), and MSN (SEQ ID NO: 2).

48. The method of clause 45, wherein the biomarkers of sensitivity comprise COL5A2 (SEQ ID NO 73 or 211), ITGA4 (SEQ ID NO: 1), MSN (SEQ ID NO: 2), and FAM46A (SEQ ID NO: 3 or 280).

49. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), and DOCK2 (SEQ ID NO: 5 or 223).

50. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), and EVL (SEQ ID NO: 6).

51. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), and SACS (SEQ ID NO: 7).

52. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), and PTPRCAP (SEQ ID NO: 8).

53. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), and EBI2 (SEQ ID NO: 9).

54. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), and PTPRC (SEQ ID NO: 10, 18, 25, or 243).

55. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), and ANP32E (SEQ ID NO: 11).

56. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), and SFPQ (SEQ ID NO: 12, 38 or 272).

57. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), and C1QR1 (SEQ ID NO: 13).

58. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), and FNBP1 (SEQ ID NO: 14 or 28).

59. The method of clause 45, wherein the biomarkers of sensitivity comprise COL5A2 (SEQ ID NO 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), PTPRCAP (SEQ ID NO: 8), EBI2 (SEQ ID NO: 9), PTPRC (SEQ ID NO: 10, 18, 25, or 243), ANP32E (SEQ ID NO: 11), SFPQ (SEQ ID NO: 12, 38 or 272), C1QR1 (SEQ ID NO: 13), FNBP1 (SEQ ID NO: 14 or 28), and CBFB (SEQ ID NO: 15).

60. The method clause 45, wherein the biomarkers of sensitivity comprise COL5A2 (SEQ ID NO: 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), EBI2 (SEQ ID NO: 9), ANP32E (SEQ ID NO: 11), SFPQ (SEQ ID NO: 12, 38 or 272), FNBP1 (SEQ ID NO: 14 or 28), CBFB (SEQ ID NO: 15), and SFRS7 (SEQ ID NO: 19 or 54).

61. The method of claim 45, wherein the biomarkers of sensitivity comprise COL5A2 (SEQ ID NO: 73 or 211), ITGA4 (SEQ ID NO: 1), MSN (SEQ ID NO: 2), FAM46A (SEQ ID NO: 3 or 280), ITGB2 (SEQ ID NO: 4), DOCK2 (SEQ ID NO: 5 or 223), EVL (SEQ ID NO: 6), SACS (SEQ ID NO: 7), EBI2 (SEQ ID NO: 9), ANP32E (SEQ ID NO: 11), SFPQ (SEQ ID NO: 12, 38 or 272), FNBP1 (SEQ ID NO: 14 or 28), CBFB (SEQ ID NO: 15), SFRS7 (SEQ ID NO: 19 or 54), and CAP350 (SEQ ID NO: 20 or 61).

62. The method of any one of clauses 17-61, wherein the biomarker of resistance is selected from one or more of SFN (SEQ ID NO: 96 or 324), LISCH7 (SEQ ID NO: 97), EPB41L4B (SEQ ID NO: 98), MST1R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), and LRP5 (SEQ ID NO: 112).

63. The method of clause 62, wherein the biomarkers of resistance comprise SFN (SEQ ID NO: 96 or 324) and LISCH7 (SEQ ID NO: 97).

64. The method of clause 62, wherein the biomarkers of resistance comprise SFN (SEQ ID NO: 96 or 324), LISCH7 (SEQ ID NO: 97), and EPB41L4B (SEQ ID NO: 98).

65. The method of clause 62, wherein the biomarkers of resistance comprise SFN (SEQ ID NO: 96 or 324), LISCH7 (SEQ ID NO: 97), EPB41L4B (SEQ ID NO: 98), and MST1R (SEQ ID NO: 99).

66. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), and ITGB4 (SEQ ID NO: 100).

67. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), and DBNDD2 (SEQ ID NO: 102 or 365).

68. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), and TACSTD1 (SEQ ID NO: 104).

69. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), and MISP (SEQ ID NO: 105).

70. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), and KRT8 (SEQ ID NO: 106).

71. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), and JUP (SEQ ID NO: 107 or 400).

72. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), and KRT18 (SEQ ID NO: 108 or 306).

73. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), and FA2H (SEQ ID NO: 109).

74. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), and MGAT4B (SEQ ID NO: 110).

75. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), and DSG2 (SEQ ID NO:111 or 312).

76. The method of clause 62, wherein the biomarkers of resistance comprise SFN (SEQ ID NO: 96 or 324), LISCH7 (SEQ ID NO: 97), EPB41L4B (SEQ ID NO: 98), MST1 R (SEQ ID NO: 99), ITGB4 (SEQ ID NO: 100), DBNDD2 (SEQ ID NO: 102 or 365), TACSTD1 (SEQ ID NO: 104), MISP (SEQ ID NO: 105), KRT8 (SEQ ID NO: 106), JUP (SEQ ID NO: 107 or 400), KRT18 (SEQ ID NO: 108 or 306), FA2H (SEQ ID NO: 109), MGAT4B (SEQ ID NO: 110), DSG2 (SEQ ID NO:111 or 312), and LRP5 (SEQ ID NO: 112).

77. The method of any one of clauses 17-76, wherein the biomarker of sensitivity is selected from one or more of CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), TMEM47 (SEQ ID NO: 215), CSRP2 (SEQ ID NO: 216), DPYSL3 (SEQ ID NO: 217), HTRA1 (SEQ ID NO: 218), SLC39A6 (SEQ ID NO: 219), and LAT2 (SEQ ID NO: 220).

78. The method of clause 77, wherein the biomarkers of sensitivity comprise CALD1 (SEQ ID NO: 206) and COL6A2 (SEQ ID NO: 207).

79. The method of clause 77, wherein the biomarkers of sensitivity comprise CALD1 (SEQ ID NO: 206), COL6A2 (SEQ ID NO: 207), and FERMT2 (SEQ ID NO: 208).

80. The method of clause 77, wherein the biomarkers of sensitivity comprise CALD1 (SEQ ID NO: 206), COL6A2 (SEQ ID NO: 207), FERMT2 (SEQ ID NO: 208), and BNIP3 (SEQ ID NO: 209 or 263).

81. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), and RAB31 (SEQ ID NO: 210).

82. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), and COL5A2 (SEQ ID NO: 73 or 211).

83. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), and MPO (SEQ ID NO: 212).

84. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), and SRPX (SEQ ID NO: 213).

85. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), and ARHGDIB (SEQ ID NO: 214).

86. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), and TMEM47 (SEQ ID NO: 215).

87. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), TMEM47 (SEQ ID NO: 215), and CSRP2 (SEQ ID NO: 216).

88. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), TMEM47 (SEQ ID NO: 215), CSRP2 (SEQ ID NO: 216), and DPYSL3 (SEQ ID NO: 217).

89. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), TMEM47 (SEQ ID NO: 215), CSRP2 (SEQ ID NO: 216), DPYSL3 (SEQ ID NO: 217), and HTRA1 (SEQ ID NO: 218).

90. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), TMEM47 (SEQ ID NO: 215), CSRP2 (SEQ ID NO: 216), DPYSL3 (SEQ ID NO: 217), HTRA1 (SEQ ID NO: 218), and SLC39A6 (SEQ ID NO: 219).

91. The method of clause 77, wherein the biomarkers of sensitivity comprise CALD1 (SEQ ID NO: 206), COL6A2 (SEQ ID NO: 207), FERMT2 (SEQ ID NO: 208), BNIP3 (SEQ ID NO: 209 or 263), RAB31 (SEQ ID NO: 210), COL5A2 (SEQ ID NO: 73 or 211), MPO (SEQ ID NO: 212), SRPX (SEQ ID NO: 213), ARHGDIB (SEQ ID NO: 214), TMEM47 (SEQ ID NO: 215), CSRP2 (SEQ ID NO: 216), DPYSL3 (SEQ ID NO: 217), HTRA1 (SEQ ID NO: 218), SLC39A6 (SEQ ID NO: 219), and LAT2 (SEQ ID NO: 220).

92. The method of any one of clauses 17-91, wherein the biomarker of resistance is selected from one or more of KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), SOX9 (SEQ ID NO: 121, 315, or 319), LYZ (SEQ ID NO: 316), IER3 (SEQ ID NO: 127 or 317), PERP (SEQ ID NO: 318), ATP1B1 (SEQ ID NO: 320), and IFI27 (SEQ ID NO: 321).

93. The method of clause 92, wherein the biomarkers of resistance comprise KRT18 (SEQ ID NO: 108 or 306) and LGALS3 (SEQ ID NO: 307).

94. The method of clause 92, wherein the biomarkers of resistance comprise KRT18 (SEQ ID NO: 108 or 306), LGALS3 (SEQ ID NO: 307), and DSP (SEQ ID NO: 308).

95. The method of clause 92, wherein the biomarkers of resistance comprise KRT18 (SEQ ID NO: 108 or 306), LGALS3 (SEQ ID NO: 307), DSP (SEQ ID NO: 308), and IGFBP4 (SEQ ID NO: 309).

96. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), and SPINT2 (SEQ ID NO: 310).

97. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), and CDH1 (SEQ ID NO: 311).

98. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), and DSG2 (SEQ ID NO: 111 or 312).

99. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), and RAB25 (SEQ ID NO: 313).

100. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), and PTPRF (SEQ ID NO: 314, 371, or 387).

101. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), and SOX9 (SEQ ID NO: 121, 315, or 319).

102. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), SOX9 (SEQ ID NO: 121, 315, or 319), and LYZ (SEQ ID NO: 316).

103. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), SOX9 (SEQ ID NO: 121, 315, or 319), LYZ (SEQ ID NO: 316), and IER3 (SEQ ID NO: 127 or 317).

104. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), SOX9 (SEQ ID NO: 121, 315, or 319), LYZ (SEQ ID NO: 316), IER3 (SEQ ID NO: 127 or 317), and PERP (SEQ ID NO: 318).

105. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), SOX9 (SEQ ID NO: 121, 315, or 319), LYZ (SEQ ID NO: 316), IER3 (SEQ ID NO: 127 or 317), PERP (SEQ ID NO: 318), and ATP1B1 (SEQ ID NO: 320).

106. The method of clause 92, wherein the biomarkers of resistance comprise KRT18 (SEQ ID NO: 108 or 306), LGALS3 (SEQ ID NO: 307), DSP (SEQ ID NO: 308), IGFBP4 (SEQ ID NO: 309), SPINT2 (SEQ ID NO: 310), CDH1 (SEQ ID NO: 311), DSG2 (SEQ ID NO: 111 or 312), RAB25 (SEQ ID NO: 313), PTPRF (SEQ ID NO: 314, 371, or 387), SOX9 (SEQ ID NO: 121, 315, or 319), LYZ (SEQ ID NO: 316), IER3 (SEQ ID NO: 127 or 317), PERP (SEQ ID NO: 318), ATP1B1 (SEQ ID NO: 320), and IFI27 (SEQ ID NO: 321).

107. A composition comprising $sPLA_2$ hydrolysable, cisplatin-containing liposome for use in treating cancer, wherein the composition is formulated for administration of at least two doses of cisplatin, wherein each of the doses comprise cisplatin in an amount of about 75 mg to about 90 mg or cisplatin in an amount of about 40 $mg/m^2$ body surface area to about 55 $mg/m^2$ body surface area, wherein the formulation is characterized as being for adminstration on day 1 and day 8, respectively, of at least one three week treatment cycle.

108. Use of a composition comprising $sPLA_2$ hydrolysable, cisplatin-containing liposome in the manufacture of a medicament for treating cancer in a subject in need thereof, wherein the composition is formulated for administration of at least two doses of cisplatin, wherein each of the doses comprise cisplatin in an amount of about 75 mg to about 90 mg or cisplatin in an amount of about 40 $mg/m^2$ body surface area to about 55 $mg/m^2$ body surface area, wherein the formulation is characterized as being for administration on day 1 and day 8, respectively, of at least one three week treatment cycle.

109. A kit comprising:

i) a composition comprising $sPLA_2$ hydrolysable, cisplatin-containing liposome for use in treating cancer, wherein the composition is formulated for administration of at least two doses of cisplatin, wherein each of the doses comprise cisplatin in an amount of about 75 mg to about 90 mg or cisplatin in an amount of about 40 $mg/m^2$ body surface area to about 55 $mg/m^2$ body surface area, wherein the formulation is characterized to be administered on day 1 and day 8, respectively, of at least one three week treatment cycle; and, optionally,
ii) instructions for administering the composition to a subject in need thereof.

## Claims

1. A composition comprising a secretory phospholipase A2 ($sPLA_2$) hydrolysable, cisplatin-containing liposome, for use in treating cancer in a subject, wherein the composition is formulated for administration of at least two doses of cisplatin, wherein each of the doses comprises cisplatin in an amount of about 75 mg to about 90 mg, or cisplatin in an amount of about 40 $mg/m^2$ body surface area to about 55 $mg/m^2$ body surface area of the subject, and wherein the composition is formulated for administration on day 1 and day 8, respectively, of at least one three week treatment cycle.

2. The composition for use according to claim 1, wherein each of the doses comprises cisplatin in an amount of:

   (a) about 75 mg; or
   (b) about 90 mg.

3. The composition for use according to claim 1, wherein each of the doses comprises cisplatin in an amount of:

   (a) about 40 $mg/m^2$ body surface area of the subject; or
   (b) about 55 $mg/m^2$ body surface area of the subject.

4. The composition for use according to any one of claims 1-3, wherein the composition is formulated for administration of about 150 mg to about 180 mg cisplatin in each three week treatment cycle.

5. The composition for use according to claim 4, wherein the composition is formulated for administration of:

   (a) about 150 mg cisplatin in each three week treatment cycle; or
   (b) about 180 mg cisplatin in each three week treatment cycle.

6. The composition for use according to any one of claims 1-5, wherein the composition is formulated for administration prior to, concurrently with, or after administration of one or more additional therapies, wherein optionally the one or more additional therapies comprise surgery, radiation, or a therapeutic agent, and wherein optionally the therapeutic agent is selected from the group consisting of docetaxel, cabazitaxel, mitoxantrone, estramustine, prednisone, carboplatin, bevacizumab, paclitaxel, gemcitabine, doxorubicin, topotecan, etoposide, tamoxifen, letrozole, sorafenib, fluorouracil, capecitabine, oxaliplatin, interferon-alpha, 5-fluorouracil (5-FU), a histone deacetylase (HDAC) inhibitor, ipilimumab, bortezomib, carfilzomib, thalidomide, lenalidomide, pomalidomide, dexamethasone, cyclophosphamide, vincristine, melphalan, tegafur, irinotecan, cetuximab, leucovorin, SN-38, everolimus, temsirolimus, bleomycin, lomustine, depsipeptide, erlotinib, cisplatin, busulfan, epirubicin, arsenic trioxide, bendamustine, fulvestrant, teniposide, adriamycin, decitabine, estramustine, azaguanine, aclarubicin, mitomycin, paclitaxel, taxotere, APO010, ara-c, methylprednisolone, methotrexate, methyl-gag, belinostat, idarubicin, IL4-PR38, valproic acid, all-trans retinoic acid (ATRA), cytoxan, suberoylanilide hydroxamic acid, leukeran, fludarabine, vinblastine, dacarbazine, hydroxyurea, tegafur, daunorubicin, mechlorethamine, streptozocin, carmustine, mercaptopurine, dactinomycin, tretinoin, ifosfamide, floxuridine, thioguanine, PSC 833, herceptin, celecoxib, iressa, anastrozole, and rituximab.

7. The composition for use according to any one of claims 1-6, wherein the composition is formulated for intravenous, intramuscular, transdermal, intradermal, intra-arterial, intracranial, subcutaneous, intraorbital, intraventricular, intraspinal, intraperitoneal, or intranasal administration, wherein optionally the composition is formulated for administration as an intravenous infusion.

8. The composition for use according to claim 7, wherein the composition is formulated for intravenous infusion over a period of 2-3 hours.

9. The composition for use according to claim 8, wherein the composition is formulated for administration:

   (a) as a 2 hour infusion; or

(b) as a 3 hour infusion.

**10.** The composition for use according to any one of claims 1-9, wherein the three week treatment cycle is repeated two to twenty times.

**11.** The composition for use according to any one of claims 1-10, wherein:

A) the subject has been determined to be responsive to the composition by:

(a) contacting a sample comprising one or more nucleic acid molecules from the subject with a device comprising:

i) one or more single-stranded nucleic acid molecules capable of specifically hybridizing with nucleotides of one or more biomarkers of sensitivity selected from those listed in Tables 2 and/or 4, or a complement thereof, wherein the one or more biomarkers of sensitivity is not C1QR1 (SEQ ID NO: 13), SLA (SEQ ID NO: 48), PTPN7 (SEQ ID NO: 77), CENTB1 (SEQ ID NO: 37), IFI16 (SEQ ID NO: 17 or 261), ARHGEF6 (SEQ ID NO: 36 or 294), CD3D (SEQ ID NO: 81), ARHGAP15 (SEQ ID NO: 30), HCLS1 (SEQ ID NO: 16 or 259), CD53 (SEQ ID NO: 282), PTPRCAP (SEQ ID NO: 8), or PTPRC (SEQ ID NO: 10, 18, 25, or 243); and/or
ii) one or more single-stranded nucleic acid molecules capable of specifically hybridizing with nucleotides of one or more biomarkers of resistance selected from those listed in Tables 3 and/or 5, or a complement thereof;

(b) detecting a level of the one or more biomarkers of sensitivity or the complement thereof and/or the one or more biomarkers of resistance or the complement thereof in the sample by detecting hybridization between the one or more single-stranded nucleic acid molecules of the device and the one or more nucleic acid molecules of the sample; and
(c) determining the subject is to be responsive to the composition if:

i) the level of the biomarkers of sensitivity or the complement thereof is substantially similar to the level of the biomarkers of sensitivity or the complement thereof in a cell or tissue known to be sensitive to the composition; and/or
ii) the level of the biomarkers of resistance or the complement thereof is substantially dissimilar to the level of the biomarkers of resistance or the complement thereof in a cell or tissue known to be resistant to the composition;

B) the subject has been determined to be responsive to the composition prior to administration of the composition by detecting a level of PLA2G2A (SEQ ID NO: 380), or a complement thereof, in the sample from the subject and/or
C) the subject has been determined to be responsive to the composition by detecting $sPLA_2$ protein in a tumour sample from the subject.

**12.** The composition for use according to any one of claims 1-11, wherein:

(a) the subject is resistant to one or more cancer therapies other than the composition, wherein optionally the one or more cancer therapies comprise surgery, radiation, or a therapeutic agent; and/or
(b) the subject exhibits cancer relapse after treatment with the one or more cancer therapies.

**13.** The composition for use according to any one of claims 1-12, wherein the cancer is selected from:

(a) a solid tumour cancer and a hematological cancer; and/or
(b) a group consisting of breast cancer, acute myelogenous leukemia (AML), acute lympho-blastic leukemia (ALL), chronic lymphocytic leukemia (CLL), myelodysplastic syndrome (MDS), chronic myelogenous leukemia - chronic phase (CMLCP), diffuse large B-cell lymphoma (DLBCL), cutaneous T-cell lymphoma (CTCL), peripheral T-cell lymphoma (PTCL), Hodgkin's lymphoma, hepatocellular carcinoma (HCC), cervical cancer, renal cell carcinoma (RCC), esophageal cancer, melanoma, glioma, pancreatic cancer, gastrointestinal stromal tumors (GIST), sarcoma, non-small cell lung carcinoma (NSCLC), prostate cancer, ovarian cancer, colon cancer, bladder cancer, and squamous cell carcinoma of the head and neck (SCCHN), wherein optionally the breast cancer is an estrogen receptor-positive (ERpos) breast cancer and/or a metastatic form of breast cancer.

**14.** The composition for use according to any one of claims 1-13, wherein the subject:

(a) has not been administered a treatment for cancer; or
(b) exhibits cancer relapse after a first cancer treatment and prior to treatment with the composition.

**15.** A kit comprising:

i) a composition comprising $sPLA_2$ hydrolysable, cisplatin-containing liposome for use in treating cancer, wherein the composition is formulated for administration of at least two doses of cisplatin, wherein each of the doses comprise cisplatin in an amount of about 75 mg to about 90 mg or cisplatin in an amount of about 40 $mg/m^2$ body surface area to about 55 $mg/m^2$ body surface area, wherein the formulation is characterized to be administered on day 1 and day 8, respectively, of at least one three week treatment cycle; and, optionally,
ii) instructions for administering the composition to a subject in need thereof.

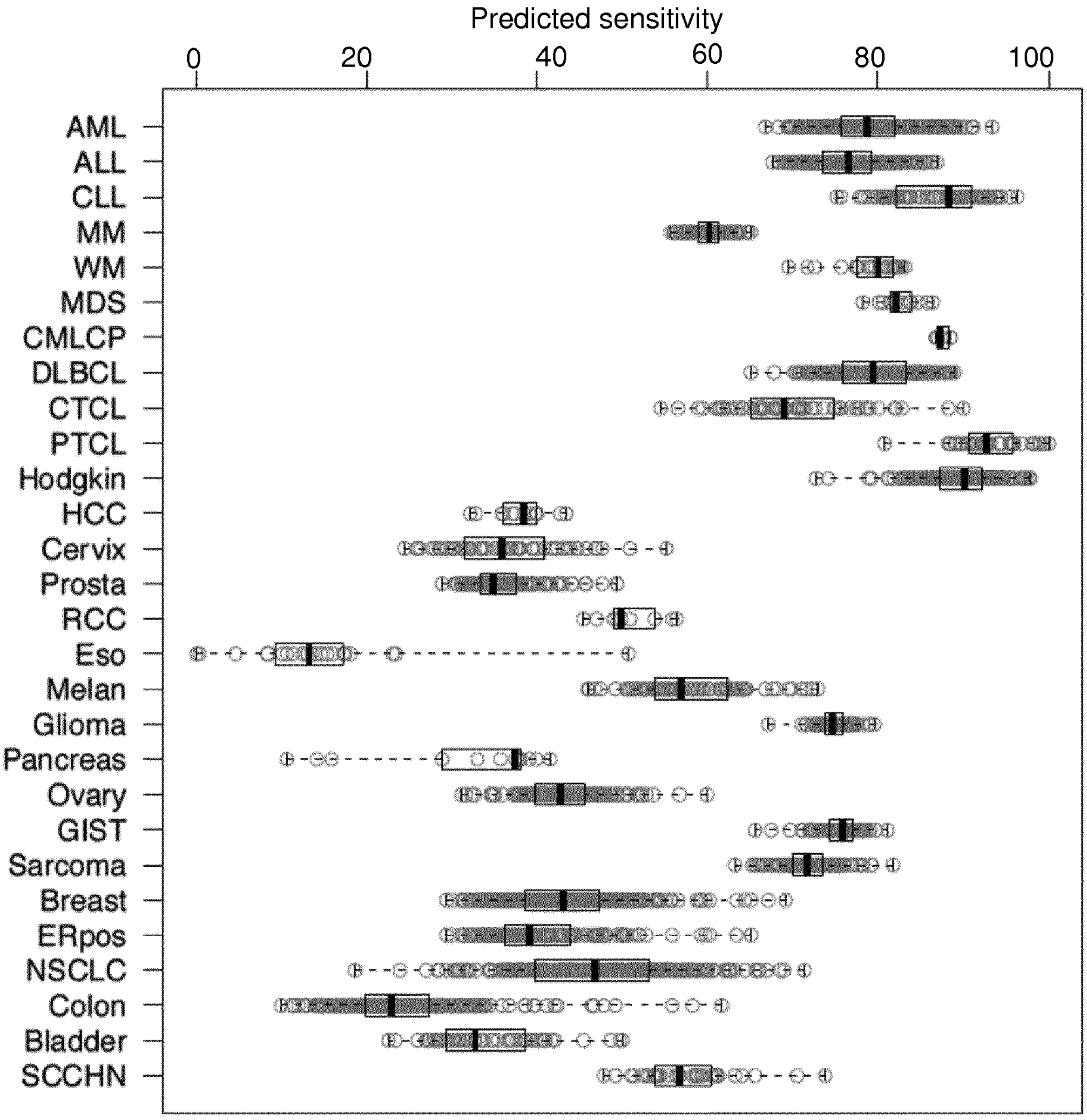
Predicted sensitivity
0
20
40
60
80
100
AML
ALL
CLL
MM
WM
MDS
CMLCP
DLBCL
CTCL
PTCL
Hodgkin
HCC
Cervix
Prosta
RCC
Eso
Melan
Glioma
Pancreas
Ovary
GIST
Sarcoma
Breast
ERpos
NSCLC
Colon
Bladder
SCCHN

Figure 1

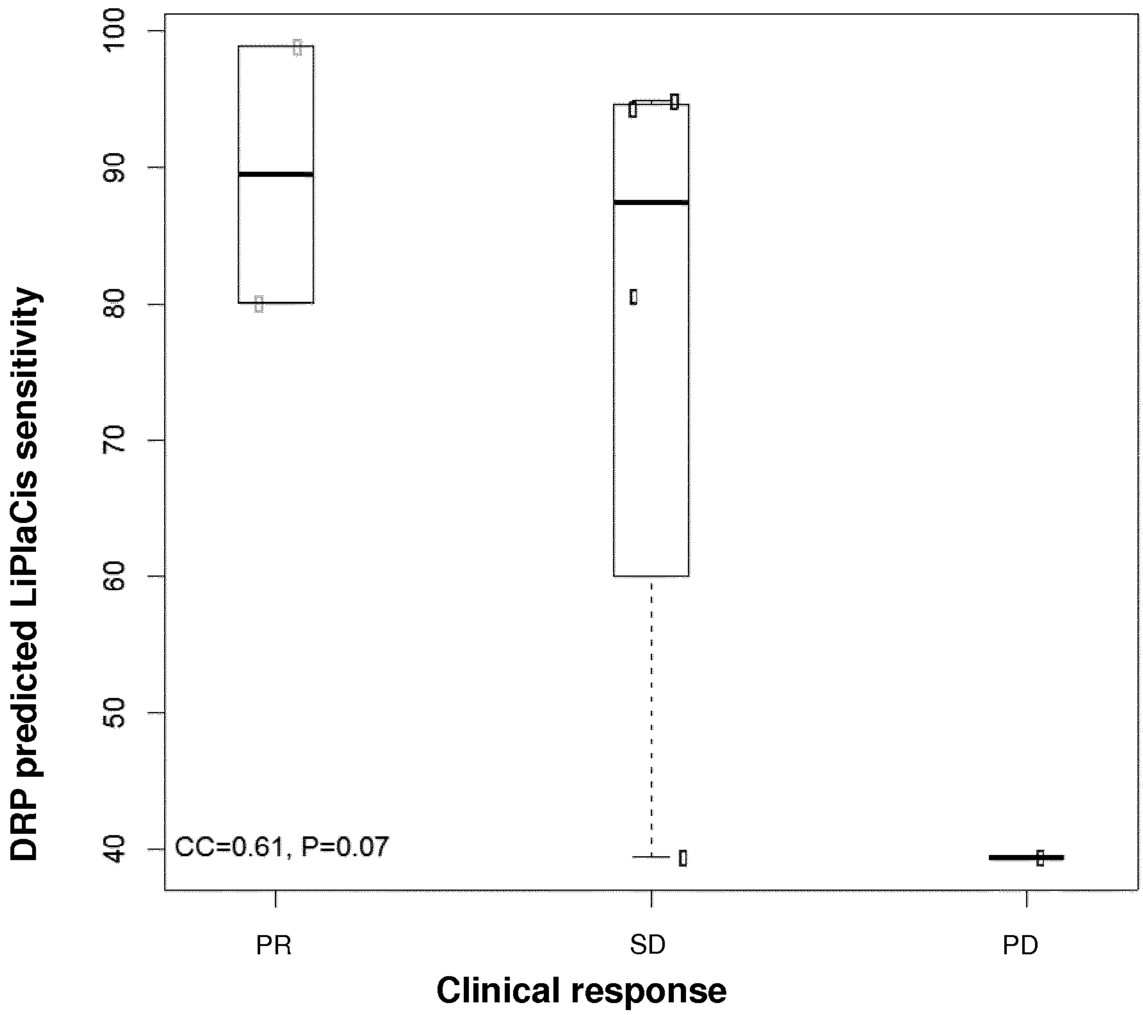
DRP predicted LiPlaCis sensitivity
100
90
80
70
60
50
40
CC=0.61, P=0.07
PR
SD
PD
Clinical response

Figure 2

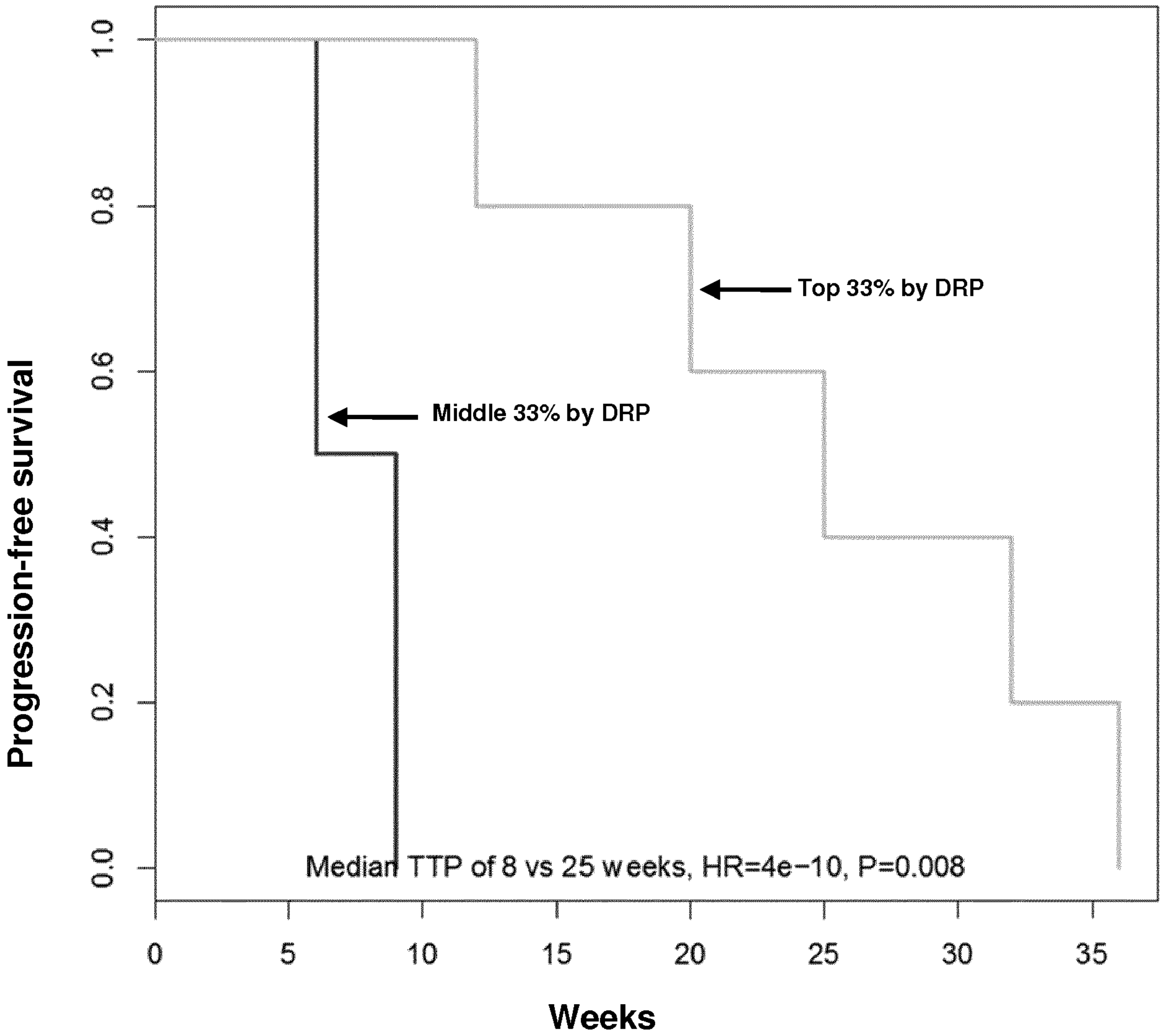
1.0
0.8
0.6
0.4
0.2
0.0
Progression-free survival
Top 33% by DRP
Middle 33% by DRP
Median TTP of 8 vs 25 weeks, HR=4e−10, P=0.008
0
5
10
15
20
25
30
35
Weeks

Figure 3

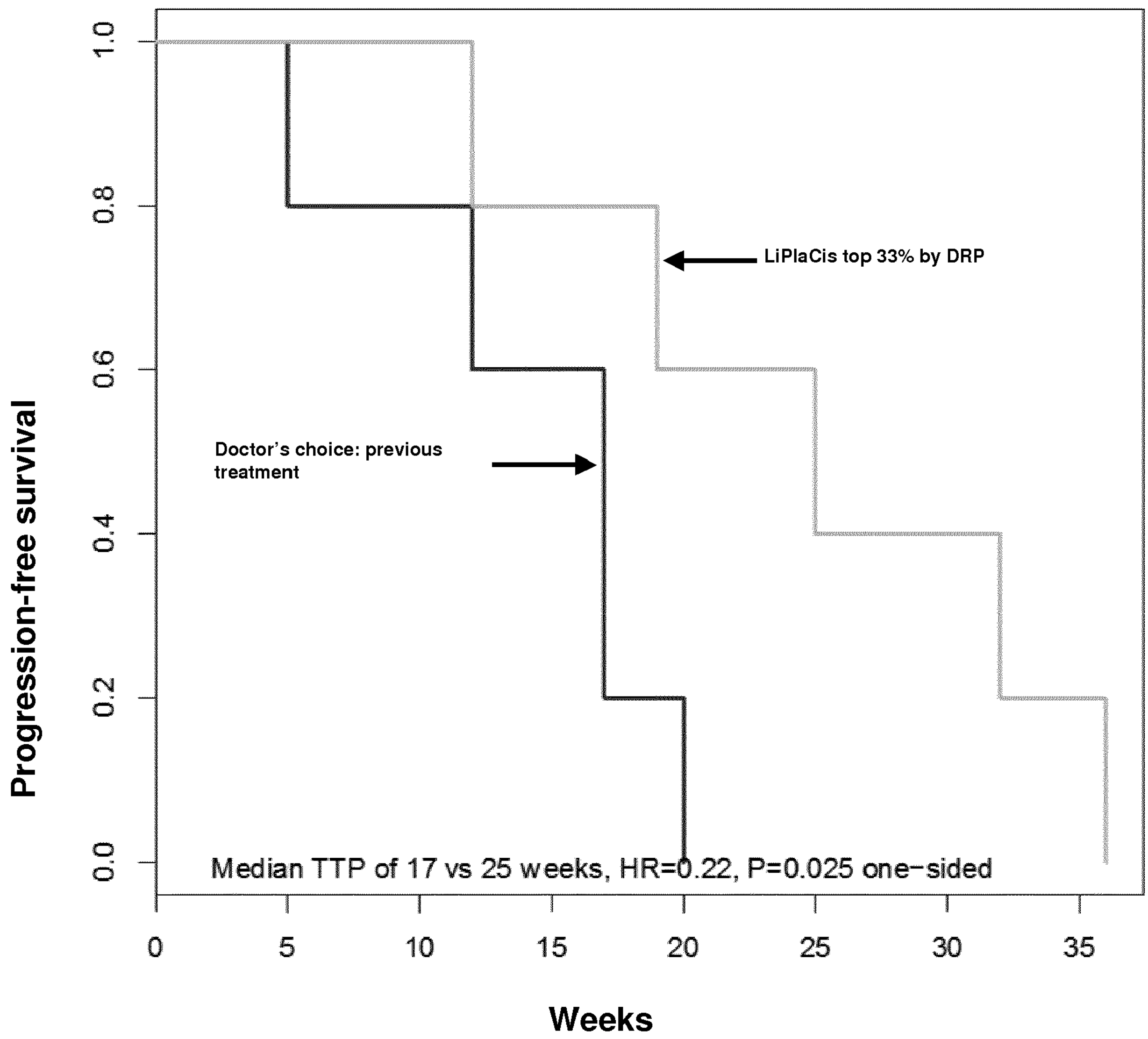
1.0
0.8
0.6
0.4
0.2
0.0
Progression-free survival
LiPlaCis top 33% by DRP
Doctor's choice: previous
treatment
Median TTP of 17 vs 25 weeks, HR=0.22, P=0.025 one−sided
0
5
10
15
20
25
30
35
Weeks

Figure 4

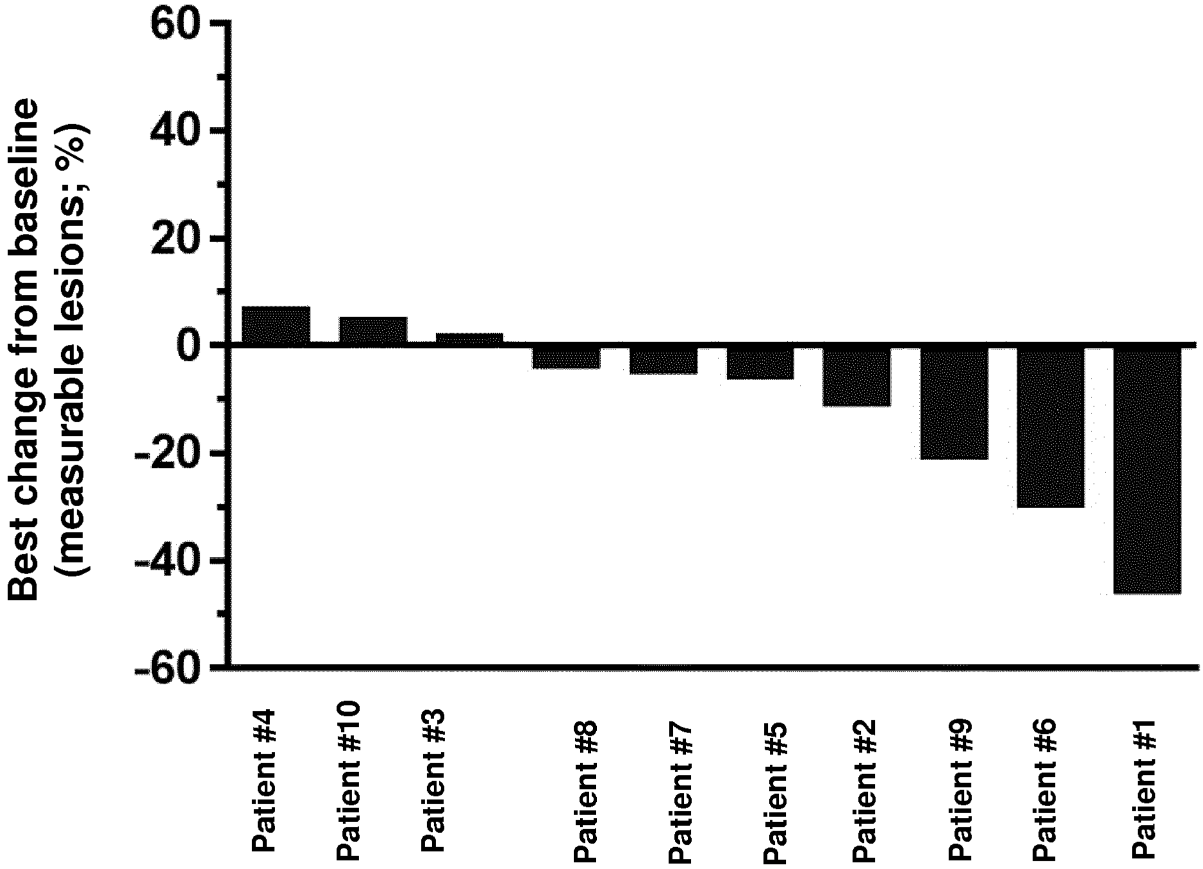
60
40
20
0
-20
-40
-60
Best change from baseline
(measurable lesions; %)
Patient #4
Patient #10
Patient #3
Patient #8
Patient #7
Patient #5
Patient #2
Patient #9
Patient #6
Patient #1

Figure 5

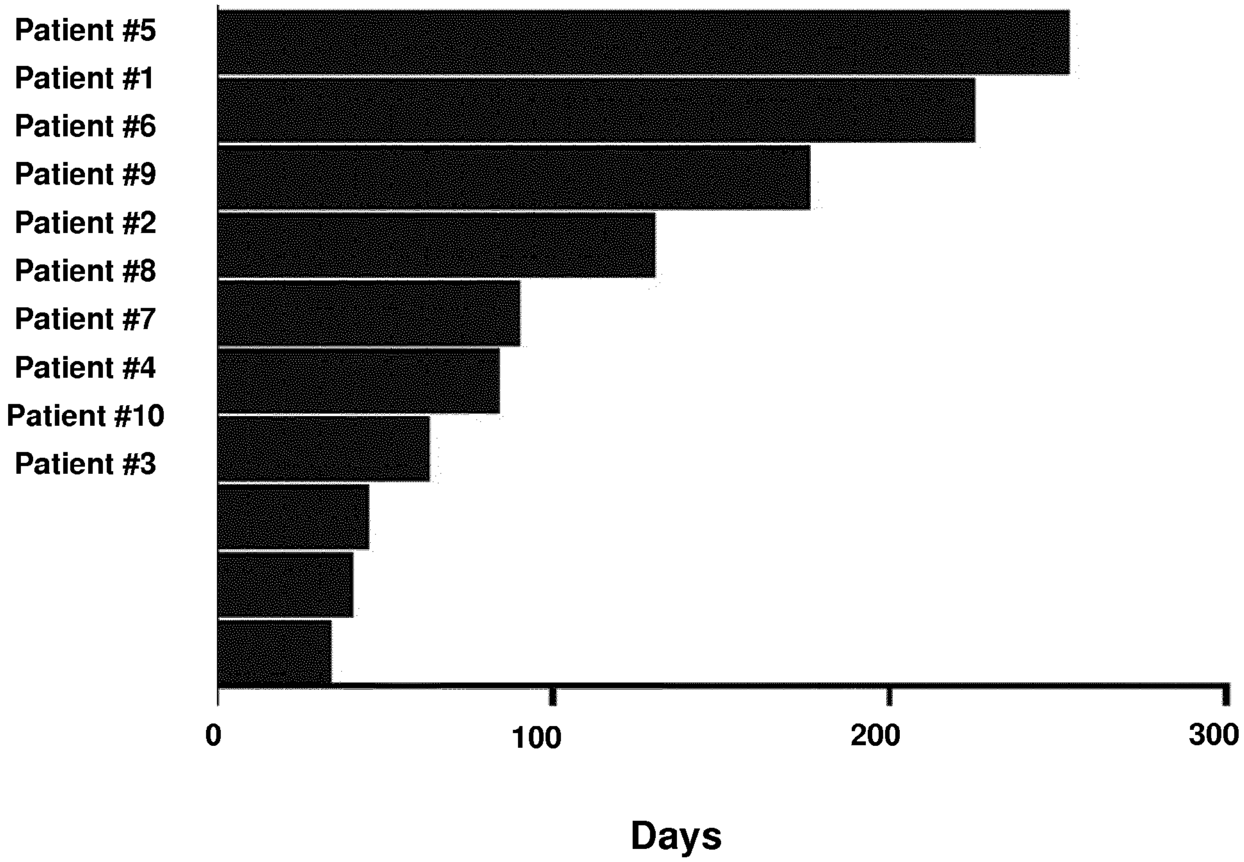
Patient #5
Patient #1
Patient #6
Patient #9
Patient #2
Patient #8
Patient #7
Patient #4
Patient #10
Patient #3
0
100
200
300
Days

Figure 6

Figure 7

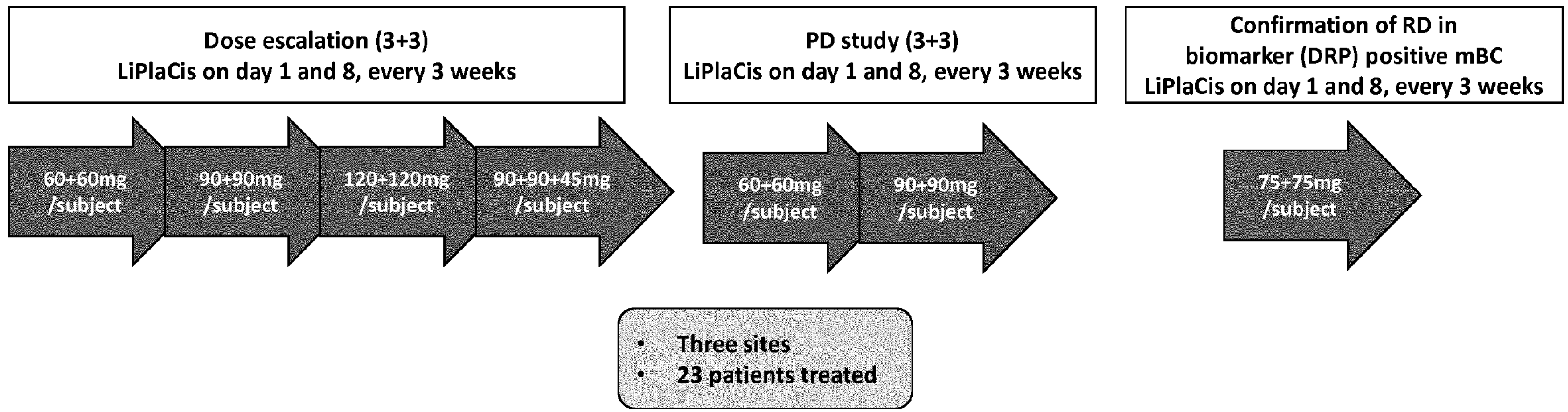
Dose escalation (3+3)
LiPlaCis on day 1 and 8, every 3 weeks
60+60mg /subject
90+90mg /subject
120+120mg /subject
90+90+45mg /subject
PD study (3+3)
LiPlaCis on day 1 and 8, every 3 weeks
60+60mg /subject
90+90mg /subject
Confirmation of RD in
biomarker (DRP) positive mBC
LiPlaCis on day 1 and 8, every 3 weeks
75+75mg /subject
• Three sites
• 23 patients treated

Europäisches Patentamt
European Patent Office
Office européen des brevets

# EUROPEAN SEARCH REPORT

Application Number
EP 19 15 4186

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2012/177726 A1 (PETERSEN MORTEN JUST [DK] ET AL) 12 July 2012 (2012-07-12)<br>* paragraph [0089] - paragraph [0093] *<br>* example 1 *<br>* claims *<br>----- | 1-15 | INV.<br>A61K9/00<br>A61K9/127<br>A61K31/282 |
| X | WO 2009/141450 A2 (LIPLASOME PHARMA AS [DK]; VIKBJERG ANDERS FALK [DK] ET AL.) 26 November 2009 (2009-11-26)<br>* claims *<br>* figures *<br>----- | 1-15 | |
| X | WO 2011/047689 A2 (BIO BEDST APS [DK]; MADSEN MOGENS WINKEL [DK] ET AL.) 28 April 2011 (2011-04-28)<br>* claims *<br>----- | 1-15 | |
| X | POURHASSAN HOUMAN ET AL: "Revisiting the use of sPLA2-sensitive liposomes in cancer therapy", JOURNAL OF CONTROLLED RELEASE, ELSEVIER, AMSTERDAM, NL, vol. 261, 27 June 2017 (2017-06-27), pages 163-173, XP085150027, ISSN: 0168-3659, DOI: 10.1016/J.JCONREL.2017.06.024<br>* page 169, paragraph 2 - page 171, paragraph 1 *<br>----- | 1-15 | TECHNICAL FIELDS SEARCHED (IPC)<br>A61K |

The present search report has been drawn up for all claims

1

| Place of search | Date of completion of the search | Examiner |
|---|---|---|
| Munich | 29 May 2019 | S. von Eggelkraut-G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 15 4186

This annex lists the patent family members relating to the patent documents cited in the above-mentioned European search report. The members are as contained in the European Patent Office EDP file on
The European Patent Office is in no way liable for these particulars which are merely given for the purpose of information.

29-05-2019

| Patent document cited in search report | Publication date | Patent family member(s) | Publication date |
|---|---|---|---|
| US 2012177726 A1 | 12-07-2012 | AU 2010294971 A1 | 22-03-2012 |
| | | CA 2772965 A1 | 24-03-2011 |
| | | CN 102630175 A | 08-08-2012 |
| | | CY 1119982 T1 | 12-12-2018 |
| | | DK 177532 B1 | 08-09-2013 |
| | | DK 2477699 T3 | 12-02-2018 |
| | | EP 2477699 A1 | 25-07-2012 |
| | | ES 2658971 T3 | 13-03-2018 |
| | | HR P20180225 T1 | 09-03-2018 |
| | | HU E038017 T2 | 28-09-2018 |
| | | JP 2013505204 A | 14-02-2013 |
| | | JP 2015127349 A | 09-07-2015 |
| | | JP 2017052790 A | 16-03-2017 |
| | | LT 2477699 T | 26-02-2018 |
| | | NO 2477699 T3 | 07-04-2018 |
| | | PL 2477699 T3 | 30-05-2018 |
| | | PT 2477699 T | 15-02-2018 |
| | | SI 2477699 T1 | 30-03-2018 |
| | | US 2012177726 A1 | 12-07-2012 |
| | | WO 2011032563 A1 | 24-03-2011 |
| WO 2009141450 A2 | 26-11-2009 | AU 2009248673 A1 | 26-11-2009 |
| | | CA 2725529 A1 | 26-11-2009 |
| | | CN 102065840 A | 18-05-2011 |
| | | CY 1115325 T1 | 04-01-2017 |
| | | DK 2299977 T3 | 05-11-2012 |
| | | EP 2123258 A1 | 25-11-2009 |
| | | EP 2299977 A2 | 30-03-2011 |
| | | ES 2393170 T3 | 19-12-2012 |
| | | HR P20120776 T1 | 31-10-2012 |
| | | JP 5767580 B2 | 19-08-2015 |
| | | JP 2011521913 A | 28-07-2011 |
| | | PT 2299977 E | 07-09-2012 |
| | | SI 2299977 T1 | 30-11-2012 |
| | | US 2012009243 A1 | 12-01-2012 |
| | | WO 2009141450 A2 | 26-11-2009 |
| WO 2011047689 A2 | 28-04-2011 | AU 2010310240 A1 | 22-03-2012 |
| | | CA 2772973 A1 | 28-04-2011 |
| | | CN 102639114 A | 15-08-2012 |
| | | CY 1115139 T1 | 14-12-2016 |
| | | DK 177529 B1 | 08-09-2013 |
| | | DK 2490671 T3 | 16-06-2014 |
| | | EP 2490671 A2 | 29-08-2012 |
| | | ES 2465565 T3 | 06-06-2014 |
| | | HR P20140413 T1 | 04-07-2014 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 19 15 4186

This annex lists the patent family members relating to the patent documents cited in the above-mentioned European search report. The members are as contained in the European Patent Office EDP file on
The European Patent Office is in no way liable for these particulars which are merely given for the purpose of information.

29-05-2019

| Patent document cited in search report | Publication date | Patent family member(s) | Publication date |
|---|---|---|---|
| | | JP 5778160 B2 | 16-09-2015 |
| | | JP 2013508315 A | 07-03-2013 |
| | | PT 2490671 E | 30-05-2014 |
| | | RS 53321 B | 31-10-2014 |
| | | SI 2490671 T1 | 31-07-2014 |
| | | SM T201400065 B | 07-07-2014 |
| | | US 2012219618 A1 | 30-08-2012 |
| | | WO 2011047689 A2 | 28-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20120177726 A
- US 20110201515 A
- US 20110229888 A
- US 20130017971 A
- US 5822715 A
- US 20030073083 A
- US 20050227266 A
- US 20050208512 A
- US 20050123945 A
- US 20030129629 A
- US 20020006613 A


### Non-patent literature cited in the description


- **JONGE et al.** *Eur J Cancer.,* 2010, vol. 46 (16), 3016-21
- Handbook of Pharmaceutical Excipients. Pharmaceutical Press, 2009
- **MORTAZAVI et al.** *Nat. Methods,* 2008, vol. 5, 621-628
- **CHEN et al.** *Mol. Cancer Ther.,* 2012, vol. 11, 34-33
- **WANG et al.** *J. Nat. Cancer Inst.,* 2013, vol. 105, 1284-1291
- **KNUDSEN et al.** *PLoS One,* 2014, vol. 9, e87415
- **VAPNIK V N.** Statistical Learning Theory. John Wiley & Sons, 1998
- The Elements of Statistical Learning. **HASTIE et al.** Data Mining, Inference, and Prediction. Springer, 1996
- An Introduction to Categorical Data Analysis. John Wiley & Sons
- Neural Network Modeling of Physiological Processes. **V. TRESP et al.** Computational Learning Theory and Natural Learning Systems 2. MIT Press, 1994
- **MIRTTI et al.** *APMIS,* 2009, vol. 117, 151-161